# EUROPEAN PATENT APPLICATION

(11) **EP 3 412 314 A1**
(43) Date of publication of application: **12.12.2018**
(21) Application number: 18182890.6
(22) Date of filing: 25.05.2012
(51) Int. Cl.: A61K 47/61, A61K 38/38, A61K 38/55

(54) **THERAPEUTIC PROTEINS CONJUGATED TO POLYSIALIC ACID AND METHODS OF PREPARING SAME**

(30) Priority: 27.05.2011 US 201161490869 P
(62) Divisional of application: 12725992.7
(71) Applicant: Baxalta GmbH, 6300 Zug (CH); Baxalta Incorporated, Bannockburn, IL 60015 (US)
(72) Inventor: Siekmann, Juergen, 1210 Vienna (AT); Weber, Alfred, 1210 Vienna (AT); Rottensteiner, Hanspeter, 1210 Vienna (AT); Turecek, Peter, 3400 Klosterneuburg (AT)
(74) Representative: Hoffmann Eitle

(57) **Abstract**

The present disclosure relates to materials and methods of conjugating a water soluble polymer to a therapeutic protein.

## Description

### FIELD OF THE INVENTION

The present disclosure relates to materials and methods for conjugating a water soluble polymer to a therapeutic protein.

### BACKGROUND OF THE INVENTION

The preparation of conjugates by forming a covalent linkage between a water soluble polymer and a therapeutic protein can be carried out by a variety of chemical methods. PEGylation of polypeptide drugs protects them in circulation and improves their pharmacodynamic and pharmacokinetic profiles (Harris and Chess, Nat Rev Drug Discov. 2003; 2:214-21). The PEGylation process attaches repeating units of ethylene glycol (polyethylene glycol (PEG)) to a polypeptide drug. PEG molecules have a large hydrodynamic volume (5-10 times the size of globular proteins), are highly water soluble and hydrated, non-toxic, non-immunogenic and rapidly cleared from the body. PEGylation of molecules can lead to increased resistance of drugs to enzymatic degradation, increased half-life *in vivo,* reduced dosing frequency, decreased immunogenicity, increased physical and thermal stability, increased solubility, increased liquid stability, and reduced aggregation. The first PEGylated drugs were approved by the FDA in the early 1990s. Since then, the FDA has approved several PEGylated drugs for oral, injectable, and topical administration.

Polysialic acid (PSA), also referred to as colominic acid (CA), is a naturally occurring polysaccharide. It is a homopolymer of N-acetylneuraminic acid with α(2→8) ketosidic linkage and contains vicinal diol groups at its non-reducing end. It is negatively charged and a natural constituent of the human body. It can easily be produced from bacteria in large quantities and with pre-determined physical characteristics (US Patent No. 5,846,951). Because the bacterially-produced PSA is chemically and immunologically identical to PSA produced in the human body, bacterial PSA is non-immunogenic, even when coupled to proteins. Unlike some polymers, PSA acid is biodegradable. Covalent coupling of colominic acid to catalase and asparaginase has been shown to increase enzyme stability in the presence of proteolytic enzymes or blood plasma. Comparative studies *in vivo* with polysialylated and unmodified asparaginase revealed that polysialylation increased the half-life of the enzyme (Fernandes and Gregoriadis, Int J Pharm. 2001;217:215-24).

Coupling of PEG-derivatives to peptides or proteins is reviewed by Roberts et al. (Adv Drug Deliv Rev 2002;54:459-76). One approach for coupling water soluble polymers to therapeutic proteins is the conjugation of the polymers via the carbohydrate moieties of the protein. Vicinal hydroxyl (OH) groups of carbohydrates in proteins can be easily oxidized with sodium periodate (NaIO4) to form active aldehyde groups (Rothfus and Smith, J Biol Chem 1963; 238:1402-10; van Lenten and Ashwell, J Biol Chem 1971;246:1889-94). Subsequently the polymer can be coupled to the aldehyde groups of the carbohydrate by use of reagents containing, for example, an active hydrazide group (Wilchek M and Bayer EA, Methods Enzymol 1987;138:429-42). A more recent technology is the use of reagents containing aminooxy groups which react with aldehydes to form oxime linkages (WO 96/40662, WO2008/025856).

Additional examples describing conjugation of a water soluble polymer to a therapeutic protein are described in WO 06/071801 which teaches the oxidation of carbohydrate moieties in Von Willebrand factor and subsequent coupling to PEG using hydrazide chemistry; US Publication No. 2009/0076237 which teaches the oxidation of rFVIII and subsequent coupling to PEG and other water soluble polymers (e.g. PSA, HES, dextran) using hydrazide chemistry; WO 2008/025856 which teaches oxidation of different coagulation factors, e.g. rFIX, FVIII and FVIIa and subsequent coupling to e.g., PEG, using aminooxy chemistry by forming an oxime linkage; and US Patent No. 5,621,039 which teaches the oxidation of FIX and subsequent coupling to PEG using hydrazide chemistry.

Recently, an improved method was described comprising mild periodate oxidation of sialic acids to generate aldehydes followed by reaction with an aminooxy group containing reagent in the presence of catalytic amounts of aniline (Dirksen A., and Dawson PE, Bioconjugate Chem. 2008;19,2543-8; and Zeng Y et al., Nature Methods 2009;6:207-9). The aniline catalysis dramatically accelerates the oxime ligation, allowing the use of very low concentrations of the reagent. The use of nucleophilic catalysts are also described in Dirksen, A., et al., J Am Chem Soc., 128:15602-3 (2006); Dirksen, A., et al., Angew chem. Int Ed., 45:7581-4 (2006); Kohler, J.J., ChemBioChem., 10:2147-50 (2009); Giuseppone, N., et al., J Am Chem Soc., 127:5528-39 (2005); and Thygesen, M.B., et al., J Org Chem., 75:1752-5 (2010).

Notwithstanding the aforementioned techniques and reagents, there remains a need in the art for materials and methods for conjugating water soluble polymers to therapeutic proteins with minimum process steps and with high efficiency, while increasing half-life and retaining biological activity.

### SUMMARY OF THE INVENTION

The present disclosure provides materials and methods for conjugating polymers to proteins that improves the protein's pharmacodynamic and/or pharmacokinetic properties while maximizing the yields of conjugation reactions.

In one embodiment of the present disclosure, a method of preparing a therapeutic protein conjugate is provided comprising the step of contacting a therapeutic protein, or biologically-active fragment thereof, with a thiol reductant and a water soluble polymer, or functional derivative thereof, under conditions that (a) produce a reduced cysteine sulfhydryl group on the therapeutic protein, and (b) allow conjugation of the water-soluble polymer to the reduced cysteine sulfhydryl group; said therapeutic protein having an amino acid sequence with no more than one accessible cysteine sulhydryl group.

In another embodiment, the aforementioned method is provided wherein the therapeutic protein is selected from the group consisting of a protein of the serpin superfamily selected from the group consisting of: A1PI (alpha-1 proteinase inhibitor), or A1AT (alpha-1-antitrypsin), ATR (alpha-1-antitrypsin-related protein), AACT or ACT (alpha-1-antichymotrypsin), PI4 (proteinase inhibitor 4), PCI or PROCI (protein C inhibitor), CBG, (corticosteroid-binding globulin), TBG (thyroxine-binding globulin), AGT (angiotensinogen), centerin, PZI (protein Z-dependent protease inhibitor), PI2 (proteinase inhibitor 2), PAI2 or PLANH2 (plasminogen activator inhibitor-2), SCCA1 (squamous cell carcinoma antigen 1), SCCA2 (squamous cell carcinoma antigen 2), PI5 (proteinase inhibitor 5), PI6 (proteinase inhibitor 6), megsin, PI8 (proteinase inhibitor 8), PI9 (proteinase inhibitor 9), PI10 (proteinase inhibitor 10), epipin, yukopin, PI13 (proteinase inhibitor 13), PI8L1 (proteinase inhibitor 8-like 1), AT3 or ATIII (antithrombin-III), HC-II or HCF2 (heparin cofactor II), PAI1 or PLANH1 (plasminogen activator inhibitor-1), PN1 (proteinase nexin I), PEDF, (pigment epithelium-derived factor), PLI (plasmin inhibitor), C1IN or C1 INH (plasma proteinase C1 inhibitor), CBP1 (collagen-binding protein 1), CBP2 (collagen-binding protein 2), PI12 (proteinase inhibitor 12), and PI14 (proteinase inhibitor 14); a protein selected from the group consisting of: antithrombin III, alpha-1-antichymotrypsin, human serum albumin, alcoholdehydrogenase, biliverdin reductase, buturylcholinesterase, complement C5a, cortisol-binding protein, creatine kinase, ferritin, heparin cofactor, interleukin 2, protein C inhibitor, tissue factor; vitronectin; ovalbumin, plasminogen-activator inhibitor, neuroserpin, C1-Inhibitor, nexin, alpha-2-antiplasmin, heparin cofactor II, alpha1-antichymotrypsin, alpha1-microglobulin; and a blood coagulation factor protein selected from the group consisting of: Factor IX (FIX), Factor VIII (FVIII), Factor VIIa (FVIIa), Von Willebrand Factor (VWF), Factor FV (FV), Factor X (FX), Factor XI (FXI), Factor XII (FXII), Factor XIII (FXIII) thrombin (FII), protein C, protein S, tPA, PAI-1, tissue factor (TF) and ADAMTS 13 protease. In a related embodiment, the therapeutic protein is A1PI. In another related embodiment, the therapeutic protein is human serum albumin.

In another embodiment, the aforementioned method is provided wherein the therapeutic protein is a glycoprotein. In a related embodiment, the therapeutic protein is glycosylated in vivo. In another related embodiment, the therapeutic protein is glycosylated in vitro.

In another embodiment, the aforementioned method is provided comprising a quantity of therapeutic protein between 0.100 and 10.0 gram weight. In various embodiments, the quantity of therapeutic protein is between 0.01 and 10.0 gram weight, between 0.02 and 9.0 gram weight, between 0.03 and 8.0 gram weight, between 0.04 and 7.0 gram weight, between 0.05 and 6.0 gram weight, between 0.06 and 5.0 gram weight, between 0.07 and 4.0 gram weight, between 0.08 and 3.0 gram weight, between 0.09 and 2.0 gram weight, and between 0.10 and 1.0 gram weight. Thus, in one embodiment, the methods of the rpesent disclosure are applicable to large-scale production of therapeutic protein conjugates.

In another embodiment, the aforementioned method is provided wherein the water-soluble polymer is selected from the group consisting of linear, branched or multi-arm water soluble polymer. In another embodiment, the aforementioned method is provided wherein the water-soluble polymer has a molecular weight between 3,000 and 150,000 Daltons (Da). In various embodiments, the water-soluble polymer has a molecular weight between 5,000 and 125,000, between 6,000 and 120,000, between 7,000 and 115,000, between 8,000 and 110,000, between 9,000 and 100,000, between 10,000 and 80,000, between 15,000 and 75,000, between 20,000 and 60,000, between 30,000 and 50,000, and between 35,000 and 45,000 Da. In one embodiment, the water-soluble polymer is linear and has a molecular weight between 10,000 and 50,000 Da. In still another embodiment, the water-soluble polymer is linear and has a molecular weight of 20,000.

In another embodiment, the aforementioned method is provided wherein the water-soluble polymer is selected from the group consisting of polyethylene glycol (PEG), branched PEG, PolyPEG® (Warwick Effect Polymers; Coventry, UK), polysialic acid (PSA), starch, hydroxylethyl starch (HES), hydroxyalkyl starch (HAS), carbohydrate, polysaccharides, pullulane, chitosan, hyaluronic acid, chondroitin sulfate, dermatan sulfate, dextran, carboxymethyl-dextran, polyalkylene oxide (PAO), polyalkylene glycol (PAG), polypropylene glycol (PPG), polyoxazoline, polyacryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC), and functional derivatives thereof.

In still another embodiment, the aforementioned method is provided wherein the water soluble polymer is derivatized to contain a sulfhydryl-specific group selected from the group consisting of: maleimide (MAL), vinylsulfones, orthopyridyl-disulfides (OPSS) and iodacetamides. In one embodiment, the water soluble polymer is PEG and the sulfhydryl-specific group is MAL. In still another embodiment, the water soluble polymer is PSA and the sulfhydryl-specific group is MAL.

In yet another embodiment, the aforementioned method is wherein the thiol reductant is selected from the group consisting of: Tris[2-carboxyethyl] phosphine hydrochloride (TCEP), dithiothreitol (DTT), dithioerythritol (DTE), sodium borohydride (NaBH₄), sodium cyanoborohydride (NaCNBH₃), β-mercaptoethanol (BME), cysteine hydrochloride and cysteine. In one embodiment, the thiol reductant is TCEP.

In another embodiment, the aforementioned method is provided wherein the thiol reductant concentration is between 1 and 100-fold molar excess relative to the therapeutic protein concentration. In still another embodiment, the thio reductant concentration is between 1 and 10-fold molar excess relative to the therapeutic protein concentration. In various embodiments, the thio reductant concentration is between 1 and 9, 1 and 8, 1 and 7, 1 and 6, 1 and 5, 2 and 4, and 3 and 4-fold molar excess relative to the therapeutic protein concentration.

In another embodiment, the aforementioned method is provided wherein the amino acid sequence of the therapeutic protein contains no more than one cysteine residue. In another embodiment, the aforementioned method is provided wherein the accessible cysteine sulfhydryl group is present in a native amino acid sequence of the therapeutic protein. In still another embodiment, the aforementioned method is provided wherein the amino acid sequence of therapeutic protein is modified to include the accessible cysteine sulfhydryl group. In yet another embodiment, the aforementioned method is provided wherein the conditions that produce a reduced cysteine sulfhydryl group on the therapeutic protein do not reduce a disulfide bond between other cysteine amino acids in the protein. In another embodiment, the aforementioned method is wherein therapeutic protein comprises only one cysteine residue which comprises an accessible sulfhydryl group that is completely or partially oxidized, said only one cysteine residue is not involved in a disulfide bond with another cysteine residue in the therapeutic protein's amino acid sequence.

In another embodiment of the present disclosure, the aforementioned method is provided further comprising the step of purifying the therapeutic protein conjugate. In various embodiments, the therapeutic protein conjugate is purified using a technique selected from the group consisting of ion-exchange chromatography, hydrophobic interaction chromatography, size exclusion chromatography and affinity chromatography or combinations thereof.

Instill another embodiment, the aforementioned method is provided wherein the therapeutic protein, water-soluble polymer and thiol reductant are incubated together in a single vessel, wherein the reduction of the oxidized SH group and the conjugation reaction is carried out simultaneously. In another embodiment, the thiol reductant is removed following incubation with the therapeutic protein and prior to incubating the therapeutic protein with the water-soluble polymer, wherein the reduction of the oxidized SH group and the conjugation reaction is carried out sequentially.

In yet another embodiment of the present disclosure, the aforementioned method is provided wherein the therapeutic protein conjugate retains at least 20% biological activity relative to native therapeutic protein. In another embodiment the therapeutic protein conjugate retains at least 60% biological activity relative to native therapeutic protein. In one embodiment, the therapeutic protein conjugate retains between 10 to 100% biological activity relative to native therapeutic protein. In various embodiments, the therapeutic protein conjugate retains at least 10%, 20%, 30%, 40%, 50%, 60%, 70%, 80%, or 90% biological activity relative to native therapeutic protein.

In yet another embodiment of the present disclosure, the aforementioned method is provided wherein at least 70% of the therapeutic protein conjugate comprises a single water-soluble polymer. In another embodiment 10-100% of the therapeutic protein conjugate comprises a single water-soluble polymer. In various embodiments, 10%, 20%, 30%, 40%, 50%, 60%, 70%, 80%, or 90% of the therapeutic protein conjugate comprises a single water-soluble polymer.

In still another embodiment of the present disclosure, the aforementioned method is provided wherein the therapeutic protein conjugate has an increased half-life relative to native therapeutic protein. In another embodiment, the therapeutic protein conjugate has at least a 1.5-fold increase in half-life relative to native therapeutic protein. In one embodiment, the therapeutic protein conjugate has at least a 1 to 10-fold increase in half-life relative to native therapeutic protein. In various embodiments, the therapeutic protein conjugate has at least a 1, 1.5, 2, 2.5, 3, 3.5, 4, 4.5, 5, 5.5, 6, 6.5, 7, 7.5, 8, 8.5, 9 or 9.5-fold increase in half-life relative to native therapeutic protein.

In still another embodiment of the present disclosure, a method of preparing an A1PI conjugate is provided comprising the steps of contacting the A1PI with TCEP under conditions that allow the reduction of a sulfhydryl group on the A1PI, and contacting a linear PEG derivatized to contain a MAL group with the A1PI under conditions that allow conjugation of the water-soluble polymer to the reduced sulfhydryl group; said A1PI comprising only one cysteine residue which comprises an accessible sulfhydryl group that is completely or partially oxidized, said only one cysteine residue is not involved in a di-sulfide bond with another cysteine residue in the A1PI's amino acid sequence; said TCEP concentration is between 3 and 4-fold molar excess relative to the A1PI concentration; wherein at least 70% of the A1PI conjugate comprises a single water-soluble polymer; said A1PI conjugate having an increased half-life relative to native A1PI; and said A1PI conjugate retaining at least 60% biological activity relative to native A1PI.

### FIGURES

Figure 1 shows stabilization of an oxime linkage by reduction with NaCNBH3 to form an alkoxyamine linkage.
Figure 2 shows the synthesis of 3-oxa-pentane-1,5-dioxyamine containing two active aminooxy groups in a two-step organic reaction employing a modified Gabriel-Synthesis of primary amines.
Figure 3 shows a pharmacokinetic profile obtained with PEGylated A1PI.

### DETAILED DESCRIPTION OF THE INVENTION

The pharmacological and immunological properties of therapeutic proteins can be improved by chemical modification and conjugation with polymeric compounds such as those described herein. The present disclosure provides material and methods for preparing therapeutic conjugates that are biologically active and have an extended half-life relative to a non-conjugated therapeutic protein. The properties of the resulting conjugates generally strongly depend on the structure and the size of the polymer. Thus, polymers with a defined and narrow size distribution are usually preferred in the art. Synthetic polymers like PEG can be manufactured easily with a narrow size distribution, while PSA can be purified in such a manner that results in a final PSA preparation with a narrow size distribution. In addition PEGylation reagents with defined polymer chains and narrow size distribution are on the market and commercially available for a reasonable price.

### METHODS OF PREPARING THERAPEUTIC PROTEIN CONJUGATES

As described herein, the instant disclosure provides a method of preparing a therapeutic protein conjugate. The various components of the methods provided by the instant disclosure, e.g., therapeutic proteins, water-soluble polymers, reducing agents, and the like, as well as the various conditions provided by the methods, e.g., reaction time and concentrations of the various components, are described below.

In one embodiment of the instant disclosure, a therapeutic protein is contacted with a thiol reductant to produce a reduced cysteine sulfhydryl group on the therapeutic protein. In another embodiment, the therapeutic protein with a reduced cysteine sulfhydryl group is contacted with a water-soluble polymer to produce a therapeutic protein conjugate. In various embodiments, the reaction steps to conjugate a water-soluble polymer to a therapeutic protein are carried out separately and "sequentially." By way of example, starting materials and reagents such as therapeutic protein, thiol reductant/reducing agent, and water soluble polymer, etc., are separated between individual reaction steps (i.e., the therapeutic protein is first reduced, followed by removal of the reducing agent, and then contacted with a water-soluble polymer). In another embodiment, the starting materials and reagents necessary to complete a conjugation reaction according to the present disclosure is carried out in a single vessel ("simultaneous").

In various embodiments of the present disclosure, a sulfhydryl - (SH) specific reagent (e.g., a water-soluble polymer with a SH-specific/compatible end group or linker) is conjugated to a SH group present on the therapeutic protein. In various embodiments, the SH group is present on a cysteine residue of the therapeutic protein. The instant disclosure provides methods whereby the therapeutic protein comprises multiple (e.g., more than one) cysteine residues, but only one of such cysteine residues is accessible, and therefore available, for conjugation to a water-soluble polymer. For example, a therapeutic protein may have multiple cysteine residues in its naturally-occurring amino acid sequence. Such a therapeutic protein, however, has no more than one accessible cysteine SH group as described below. According to various embodiments of the instant disclosure, such a therapeutic protein is site-specifically conjugated to a water-soluble polymer under conditions that allow conjugation of the water-soluble polymer to the accessible sulfhydryl group without disrupting disulfide bridges present in the therapeutic protein.

According to various embodiments of the instant disclosure and as described further below, the amino acid sequence of a therapeutic protein may naturally contain a single (i.e., one), accessible SH group on a cysteine residue. Alternatively, the amino acid sequence of a therapeutic protein may be modified using standard molecular biological techniques to contain a single, accessible SH group on a cysteine residue. Such a modification may be necessary when, for example, (i) the natural (i.e., wild-type) amino acid sequence of the therapeutic protein does not include a cysteine residue; (ii) the amino acid sequence of the therapeutic protein includes multiple cysteine residues, but all of which are involved in disulfide bridges or are otherwise not accessible (e.g., buried in the folded protein); (iii) the amino acid sequence of the therapeutic protein includes multiple cysteine residues with more than one of such cysteine residues being accessible. In the aforementioned scenarios (ii) and (iii), the instant disclosure contemplates the use of standard molecular biological techniques to engineer a modified amino acid sequence that will result in a therapeutic protein with a single accessible SH group. Alternatively, the instant disclosure contemplates the use of standard chemical techniques to modify the therapeutic protein that will result in a therapeutic protein with a single accessible SH group.

By way of example, the instant disclosure provides a method for PEGylation of a therapeutic protein (e.g., A1PI), with a SH-specific reagent (e.g. MAL-PEG), which is performed in the presence of a mild reductive agent (e.g. TCEP). This method can be performed as a simultaneous approach or, in the alternative, using a sequential approach (first reduction, then conjugation). SH-specific reagents include, but are not limited to, maleimide (MAL), vinylsulfones, orthopyridyl-disulfides (OPSS) and iodacetamides.

In various embodiments of the invention, the aforementioned method is provided wherein any water-soluble polymer is conjugated to a therapeutic protein.

In various embodiments of the invention, the aforementioned method is provided wherein the therapeutic protein contains one accessible free SH group, which is not involved in disulfide bridges. In various embodiments, the therapeutic proteins and peptides having one free accessible SH groups are prepared by methods of recombinant DNA technology (i.e., the protein's amino acid sequence is modified such that only one accessible SH group is present on the protein). In various embodiments of the instant disclosure, serpins such as A1PI (alpha-1 proteinase inhibitor), or A1AT (alpha-1-antitrypsin), ATR (alpha-1-antitrypsin-related protein), AACT or ACT (alpha-1-antichymotrypsin), PI4 (proteinase inhibitor 4), PCI or PROCI (protein C inhibitor), CBG, (corticosteroid-binding globulin), TBG (thyroxine-binding globulin), AGT (angiotensinogen), centerin, PZI (protein Z-dependent protease inhibitor), PI2 (proteinase inhibitor 2), PAI2 or PLANH2 (plasminogen activator inhibitor-2), SCCA1 (squamous cell carcinoma antigen 1), SCCA2 (squamous cell carcinoma antigen 2), PI5 (proteinase inhibitor 5), PI6 (proteinase inhibitor 6), megsin, PI8 (proteinase inhibitor 8), PI9 (proteinase inhibitor 9), PI10 (proteinase inhibitor 10), epipin, yukopin, PI13 (proteinase inhibitor 13), PI8L1 (proteinase inhibitor 8-like 1), AT3 or ATIII (antithrombin-III), HC-II or HCF2 (heparin cofactor II), PAI1 or PLANH1 (plasminogen activator inhibitor-1), PN1 (proteinase nexin I), PEDF, (pigment epithelium-derived factor), PLI (plasmin inhibitor), C1IN or C1 INH (plasma proteinase C1 inhibitor), CBP1 (collagen-binding protein 1), CBP2 (collagen-binding protein 2), PI12 (proteinase inhibitor 12), and PI14 (proteinase inhibitor 14) and blood coagulation proteins such as Factor IX (FIX), Factor VIII (FVIII), Factor VIIa (FVIIa), Von Willebrand Factor (VWF), Factor FV (FV), Factor X (FX), Factor XI (FXI), Factor XII (FXII), thrombin (FII), protein C, protein S, tPA, PAI-1, tissue factor (TF) and ADAMTS 13 protease are contemplated for use in the described methods.

### THERAPEUTIC PROTEINS

As described herein, the term therapeutic protein refers to any therapeutic protein molecule which exhibits biological activity that is associated with the therapeutic protein. In one embodiment of the present disclosure, the therapeutic protein molecule is a full-length protein. In various embodiments of the present disclosure, the therapeutic protein may be produced and purified from its natural source. Alternatively, according to the present disclosure, the term "recombinant therapeutic protein" includes any therapeutic protein obtained via recombinant DNA technology. In certain embodiments, the term encompasses proteins as described herein.

As used herein, "endogenous therapeutic protein" includes a therapeutic protein which originates from the mammal intended to receive treatment. The term also includes therapeutic protein transcribed from a transgene or any other foreign DNA present in said mammal. As used herein, "exogenous therapeutic protein " includes a blood coagulation protein which does not originate from the mammal intended to receive treatment.

As used herein, "plasma-derived therapeutic protein " or "plasmatic" includes all forms of the protein, for example a blood coagulation protein, found in blood obtained from a mammal having the property participating in the coagulation pathway.

As disclosed herein, the addition of a water soluble polymer is one approach to improve the properties of therapeutic proteins. In certain embodiments of the present disclosure, the polypeptides are exemplified by the following therapeutic proteins: enzymes, antigens, antibodies, receptors, blood coagulation proteins, growth factors, hormones, and ligands.

In certain embodiments, the therapeutic protein is a member of the serpin family of proteins (e.g., A1PI (alpha-1 proteinase inhibitor), or A1AT (alpha-1-antitrypsin), ATR (alpha-1-antitrypsin-related protein), AACT or ACT (alpha-1-antichymotrypsin), PI4 (proteinase inhibitor 4), PCI or PROCI (protein C inhibitor), CBG, (corticosteroid-binding globulin), TBG (thyroxine-binding globulin), AGT (angiotensinogen), centerin, PZI (protein Z-dependent protease inhibitor), PI2 (proteinase inhibitor 2), PAI2 or PLANH2 (plasminogen activator inhibitor-2), SCCA1 (squamous cell carcinoma antigen 1), SCCA2 (squamous cell carcinoma antigen 2), PI5 (proteinase inhibitor 5), PI6 (proteinase inhibitor 6), megsin, PI8 (proteinase inhibitor 8), PI9 (proteinase inhibitor 9), PI10 (proteinase inhibitor 10), epipin, yukopin, PI13 (proteinase inhibitor 13), PI8L1 (proteinase inhibitor 8-like 1), AT3 or ATIII (antithrombin-III), HC-II or HCF2 (heparin cofactor II), PAI1 or PLANH1 (plasminogen activator inhibitor-1), PN1 (proteinase nexin I), PEDF, (pigment epithelium-derived factor), PLI (plasmin inhibitor), C1IN or C1 INH (plasma proteinase C1 inhibitor), CBP1 (collagen-binding protein 1), CBP2 (collagen-binding protein 2), PI12 (proteinase inhibitor 12), and PI14 (proteinase inhibitor 14)). The serpins (serine proteinase inhibitors) are a superfamily of proteins (300-500 amino acids in size) that fold into a conserved structure and employ an unique suicide substrate-like inhibitory mechanism (Silverman, G.A., et al., J. Biol. Chem., 276(36):33293-33296 (2001); incorporated by reference in its entirety).

In certain embodiments, the therapeutic protein is a member of the coagulation factor family of proteins (e.g., Factor IX (FIX), Factor VIII (FVIII), Factor VIIa (FVIIa), Von Willebrand Factor (VWF), Factor FV (FV), Factor X (FX), Factor XI (FXI), Factor XII (FXII), thrombin (FII), protein C, protein S, tPA, PAI-1, tissue factor (TF) and ADAMTS 13 protease).

In various embodiments, the therapeutic proteins have one or more than one cysteine residue. In one embodiment, where a therapeutic protein has only one cysteine residue, the cysteine residue comprises an accessible sulfhydryl group that is completely or partially oxidized. Such a sulhydryl group on the cysteine, while not involved in a di-sulfide bond with another cysteine residue in the therapeutic protein's amino acid sequence, may be bound to a "free" cysteine residue or any other sulfur-containing compound (e.g., glutathione) following purification. As disclosed herein, reduction of such a cysteine on the therapeutic protein increases the efficiency of coupling, for example, a water-soluble polymer to the sulhydryl group on the cysteine.

In another embodiment, the therapeutic protein contains more than one cysteine, yet has only one cysteine residue that comprises an accessible sulfhydryl group that is completely or partially oxidized (i.e., only one cysteine residue that is not involved in a di-sulfide bond with another cysteine residue in the therapeutic protein's amino acid sequence or is not otherwise accessible due to, for example, intra- or inter - protein-protein interactions such as burial as a result of protein folding or formation of dimers, and the like).

In various embodiments, cysteine residues may be added or removed from a therapeutic protein's amino acid sequence, thereby allowing conjugation of a water-soluble according to the present disclosure. U.S Patent No. 5,766,897, which is incorporated by reference in its entirety, describes the production "cysteine-PEGylated proteins" in general. Polypeptide variants, analogs and derivatives are discussed below.

The therapeutic proteins provided herein should not be considered to be exclusive. Rather, as is apparent from the present disclosure provided herein, the methods of the present disclosure are applicable to any protein wherein attachment of a water soluble polymer is desired according to the present disclosure. For example, therapeutic proteins are described in US 2007/0026485, incorporated herein by reference in its entirety.

### A1PI

In one embodiment, A1PI is conjugated to a water soluble polymer according to the methods provided in the instant disclosure. A1PI (α1-proteinase inhibitor or A1PI or alpha 1-antitrypsin or α1-antitrypsin or A1AT) is a 52 kD glycoprotein of 394 amino acids present in human plasma (Carrell, R.W., et al., Nature 298(5872):329-34 (1982); UniProtKB/Swiss-Prot Accession No. P01009). Structure and variation of human alpha 1-antitrypsin. One carbohydrate chain (N-glycan) is added to each of three β-asparagine residues by post-translational modification (Brantly, Am. J. Respir. Cell. Mol. Boil., 27:652-654 (2002)). A1PI is encoded by a 12.2 kb gene on the human chromosome 14q31-32,3 which consists of three non-coding introns and four coding exons.

The two amino acids Met358-Ser359 are the active center of the protein. A1PI is largely synthesized in the hepatocytes, but the protein biosynthesis of A1PI followed by release into the bloodstream also takes place in mononuclear phagocytes, in intestinal cells and epithelial cells of the lung (NN., Am. J. Respir. Crit. Care Med., 168:818-900 (2003), Travis, J., and Salvesen, G.S., Annu. Rev. Biochem., 52:655-709 (1983)).

A1PI can be detected throughout the tissue of the body, but has particular physiological significance in the lung. The considerable number of permanent cellular defense events which is due to the large contact surface of the lung with the air breathed in causes increased release of highly active proteases in the surrounding alveolar tissue, if the balance between protease and inhibitor is shifted as a result of genetically caused under-expression of A1PI or toxic substances such as cigarette smoke, NE can destroy the cells of the alveoli. This may result in the formation of life-threatening lung emphysema, a chronic obstructive pulmonary disease/COPD (Klebe, g., Spektrum, 2nd ed., 351-366 (2009)).

WO 2005/027821 and U.S. Patent Nos. 5,981,715, 6,284,874, and 5,616,693, each incorporated by reference in its entirety, discloses a process for purifying A1PI. U.S. Patent No. 5,981,715 also discloses A1PI replacement or A1PI augmentation therapies. A1PI deficiency is an autosomal, recessive hereditary disorder displayed by a large number of allelic variants and has been characterized into an allelic arrangement designated as the protease inhibitor (Pi) system. These alleles have been grouped on the basis of the alpha-1-PI levels that occur in the serum of different individuals. Normal individuals have normal serum levels of alpha-1-PI (normal individuals have been designated as having a PiMM phenotype). Deficient individuals have serum alpha-1-PI levels of less than 35% of the average normal level (these individuals have been designated as having a PiZZ phenotype). Null individuals have undetectable A1PI protein in their serum (these individuals have been designated as having a Pi(null)(null) phenotype).

A1PI deficiency is characterized by low serum (less than 35% of average normal levels) and lung levels of A1PI. These deficient individuals have a high risk of developing panacinar emphysema. This emphysema predominates in individuals who exhibit PiZZ, PiZ(null) and Pi(null)(null) phenotypes. Symptoms of the condition usually manifests in afflicted individuals in the third to fourth decades of life.

The emphysema associated with A1PI deficiency develops as a result of insufficient A1PI concentrations in the lower respiratory tract to inhibit neutrophil elastase, leading to destruction of the connective tissue framework of the lung parenchyma. Individuals with A1PI deficiency have little protection against the neutrophil elastase released by the neutrophils in their lower respiratory tract. This imbalance of protease:protease inhibitor in A1PI deficient individuals results in chronic damage to, and ultimately destruction of the lung parenchyma and alveolar walls.

Individuals with severe A1PI deficiency typically exhibit endogenous serum A1PI levels of less than 50 mg/dl, as determined by commercial standards. Individuals with these low serum A1PI levels have greater than an 80% risk of developing emphysema over a lifetime. It is estimated that at least 40,000 patients in the United States, or 2% of all those with emphysema, have this disease resulting from a defect in the gene coding for A1PI. A deficiency in A1PI represents one of the most common lethal hereditary disorders of Caucasians in the United States and Europe.

Therapy for patients with A1PI deficiency is directed towards replacement or augmentation of A1PI levels in the serum. If serum levels of A1PI are increased, this is expected to lead to higher concentrations in the lungs and thus correct the neutrophil elastase: A1PI imbalance in the lungs and prevent or slow destruction of lung tissue. Studies of normal and A1PI deficient populations have suggested that the minimum protective serum A1PI levels are 80 mg/dl or 11 µM (about 57 mg/dl; using pure standards). Consequently, most augmentation therapy in A1PI deficient patients is aimed toward providing the minimum protective serum level of A1PI, since serum A1PI is the source of alveolar A1PI.

A1PI preparations have been available for therapeutic use since the mid 1980's. The major use has been augmentation (replacement) therapy for congenital A1PI deficiency. The half-life of human A1PI in vivo is 4.38 days with a standard deviation of 1.27 days. The currently recommended dosage of 60 mg A1PI/kg body weight weekly will restore low serum levels of A1PI to levels above the protective threshold level of 11 µM or 80 mg/dl.

U.S. Patent No. 4,496,689, incorporated by reference in its entirety, discloses water-soluble polymers covalently attached to A1PI. Additional publications disclose the conjugation of water-soluble polymers to the single cysteine residue of A1PI (Cantin, A.M., et al., Am. J. Respir. Cell. Mol. Biol., 27:659-665 (2002); Tyagi, S.C., J. Biol. Chem., 266:5279-5285 (1991)).

### FVII

In one embodiment, FVII is conjugated to a water soluble polymer according to the methods provided in the instant disclosure. FVII (also known as stable factor or proconvertin) is a vitamin K-dependent serine protease glycoprotein with a pivotal role in hemostasis and coagulation (Eigenbrot, Curr Protein Pept Sci. 2002;3:287-99).

FVII is synthesized in the liver and secreted as a single-chain glycoprotein of 48 kD. FVII shares with all vitamin K-dependent serine protease glycoproteins a similar protein domain structure consisting of an amino-terminal gamma-carboxyglutamic acid (Gla) domain with 9-12 residues responsible for the interaction of the protein with lipid membranes, a carboxy-terminal serine protease domain (catalytic domain), and two epidermal growth factor-like domains containing a calcium ion binding site that mediates interaction with tissue factor. Gamma-glutamyl carboxylase catalyzes carboxylation of Gla residues in the amino-terminal portion of the molecule. The carboxylase is dependent on a reduced form of vitamin K for its action, which is oxidized to the epoxide form. Vitamin K epoxide reductase is required to convert the epoxide form of vitamin K back to the reduced form.

The major proportion of FVII circulates in plasma in zymogen form, and activation of this form results in cleavage of the peptide bond between arginine 152 and isoleucine 153. The resulting activated FVIIa consists of a NH2-derived light chain (20 kD) and a COOH terminal-derived heavy chain (30 kD) linked via a single disulfide bond (Cys 135 to Cys 262). The light chain contains the membrane-binding Gla domain, while the heavy chain contains the catalytic domain.

The plasma concentration of FVII determined by genetic and environmental factors is about 0.5 mg/mL (Pinotti et al., Blood. 2000;95:3423-8). Different FVII genotypes can result in several-fold differences in mean FVII levels. Plasma FVII levels are elevated during pregnancy in healthy females and also increase with age and are higher in females and in persons with hypertriglyceridemia. FVII has the shortest half-life of all procoagulant factors (3-6 h). The mean plasma concentration of FVIIa is 3.6 ng/mL in healthy individuals and the circulating half-life of FVIIa is relatively long (2.5 h) compared with other coagulation factors.

Hereditary FVII deficiency is a rare autosomal recessive bleeding disorder with a prevalence estimated to be 1 case per 500,000 persons in the general population (Acharya et al., J Thromb Haemost. 2004;2248-56). Acquired FVII deficiency from inhibitors is also very rare. Cases have also been reported with the deficiency occurring in association with drugs such as cephalosporins, penicillins, and oral anticoagulants. Furthermore, acquired FVII deficiency has been reported to occur spontaneously or with other conditions, such as myeloma, sepsis, aplastic anemia, with interleukin-2 and antithymocyte globulin therapy.

Reference FVII polynucleotide and polypeptide sequences include, e.g., GenBank Accession Nos. J02933 for the genomic sequence, M13232 for the cDNA (Hagen et al. PNAS 1986; 83: 2412-6), and P08709 for the polypeptide sequence (references incorporated herein in their entireties). A variety of polymorphisms of FVII have been described, for example see Sabater-Lleal et al. (Hum Genet. 2006; 118:741-51) (reference incorporated herein in its entirety).

### Factor IX

FIX is a vitamin K-dependent plasma protein that participates in the intrinsic pathway of blood coagulation by converting FX to its active form in the presence of calcium ions, phospholipids and FVIIIa. The predominant catalytic capability of FIX is as a serine protease with specificity for a particular arginine-isoleucine bond within FX. Activation of FIX occurs by FXIa which causes excision of the activation peptide from FIX to produce an activated FIX molecule comprising two chains held by one or more disulfide bonds. Defects in FIX are the cause of recessive X-linked hemophilia B.

Hemophilia A and B are inherited diseases characterized by deficiencies in FVIII and FIX polypeptides, respectively. The underlying cause of the deficiencies is frequently the result of mutations in FVIII and FIX genes, both of which are located on the X chromosome. Traditional therapy for hemophilias often involves intravenous administration of pooled plasma or semi-purified coagulation proteins from normal individuals. These preparations can be contaminated by pathogenic agents or viruses, such as infectious prions, HIV, parvovirus, hepatitis A, and hepatitis C. Hence, there is an urgent need for therapeutic agents that do not require the use of human serum.

The level of the decrease in FIX activity is directly proportional to the severity of hemophilia B. The current treatment of hemophilia B consists of the replacement of the missing protein by plasma-derived or recombinant FIX (so-called FIX substitution or replacement treatment or therapy).

Polynucleotide and polypeptide sequences of FIX can be found for example in the UniProtKB/Swiss-Prot Accession No. P00740, US Pat. No. 6,531,298.

### Factor VIII

Coagulation factor VIII (FVIII) circulates in plasma at a very low concentration and is bound non-covalently to Von Willebrand factor (VWF). During hemostasis, FVIII is separated from VWF and acts as a cofactor for activated factor IX (FIXa)-mediated FX activation by enhancing the rate of activation in the presence of calcium and phospholipids or cellular membranes.

FVIII is synthesized as a single-chain precursor of approximately 270-330 kD with the domain structure A1-A2-B-A3-C1-C2. When purified from plasma (e.g., "plasma-derived" or "plasmatic"), FVIII is composed of a heavy chain (A1-A2-B) and a light chain (A3-C1-C2). The molecular mass of the light chain is 80 kD whereas, due to proteolysis within the B domain, the heavy chain is in the range of 90-220 kD.

FVIII is also synthesized as a recombinant protein for therapeutic use in bleeding disorders. Various in vitro assays have been devised to determine the potential efficacy of recombinant FVIII (rFVIII) as a therapeutic medicine. These assays mimic the in vivo effects of endogenous FVIII. In vitro thrombin treatment of FVIII results in a rapid increase and subsequent decrease in its procoagulant activity, as measured by in vitro assays. This activation and inactivation coincides with specific limited proteolysis both in the heavy and the light chains, which alter the availability of different binding epitopes in FVIII, e.g. allowing FVIII to dissociate from VWF and bind to a phospholipid surface or altering the binding ability to certain monoclonal antibodies.

The lack or dysfunction of FVIII is associated with the most frequent bleeding disorder, hemophilia A. The treatment of choice for the management of hemophilia A is replacement therapy with plasma derived or rFVIII concentrates. Patients with severe haemophilia A with FVIII levels below 1%, are generally on prophylactic therapy with the aim of keeping FVIII above 1% between doses. Taking into account the average half-lives of the various FVIII products in the circulation, this result can usually be achieved by giving FVIII two to three times a week.

Reference polynucleotide and polypeptide sequences include, e.g., UniProtKB/Swiss-Prot P00451 (FA8_HUMAN); Gitschier J et al., Characterization of the human Factor VIII gene, Nature, 312(5992): 326-30 (1984); Vehar GH et al., Structure of human Factor VIII, Nature, 312(5992):337-42 (1984); Thompson AR. Structure and Function of the Factor VIII gene and protein, Semin Thromb Hemost, 2003:29;11-29 (2002).

### Von Willebrand Factor

Von Willebrand factor (VWF) is a glycoprotein circulating in plasma as a series of multimers ranging in size from about 500 to 20,000 kD. Multimeric forms of VWF are composed of 250 kD polypeptide subunits linked together by disulfide bonds. VWF mediates initial platelet adhesion to the sub-endothelium of the damaged vessel wall. Only the larger multimers exhibit hemostatic activity. It is assumed that endothelial cells secrete large polymeric forms of VWF and those forms of VWF which have a low molecular weight (low molecular weight VWF) arise from proteolytic cleavage. The multimers having large molecular masses are stored in the Weibel-Pallade bodies of endothelial cells and liberated upon stimulation.

VWF is synthesized by endothelial cells and megakaryocytes as prepro-VWF that consists to a large extent of repeated domains. Upon cleavage of the signal peptide, pro-VWF dimerizes through disulfide linkages at its C-terminal region. The dimers serve as protomers for multimerization, which is governed by disulfide linkages between the free end termini. The assembly to multimers is followed by the proteolytic removal of the propeptide sequence (Leyte et al., Biochem. J. 274 (1991), 257-261).

The primary translation product predicted from the cloned cDNA of VWF is a 2813-residue precursor polypeptide (prepro-VWF). The prepro-VWF consists of a 22 amino acid signal peptide and a 741 amino acid propeptide, with the mature VWF comprising 2050 amino acids (Ruggeri Z.A., and Ware, J., FASEB J., 308-316 (1993).

Defects in VWF are causal to Von Willebrand disease (VWD), which is characterized by a more or less pronounced bleeding phenotype. VWD type 3 is the most severe form in which VWF is completely missing, and VWD type 1 relates to a quantitative loss of VWF and its phenotype can be very mild. VWD type 2 relates to qualitative defects of VWF and can be as severe as VWD type 3. VWD type 2 has many sub forms, some being associated with the loss or the decrease of high molecular weight multimers. Von Willebrand disease type 2a (VWD-2A) is characterized by a loss of both intermediate and large multimers. VWD-2B is characterized by a loss of highest-molecular-weight multimers. Other diseases and disorders related to VWF are known in the art.

The polynucleotide and amino acid sequences of prepro-VWF are available at GenBank Accession Nos. NM_000552 and NP_000543, respectively.

Other blood coagulation proteins according to the present invention are described in the art, e.g. Mann KG, Thromb Haemost, 1999;82:165-74.

### A. Polypeptides

In one aspect, the starting material of the present disclosure is a protein or polypeptide. Therapeutic protein molecules contemplated include full-length proteins, precursors of full length proteins, biologically active subunits or fragments of full length proteins, as well as biologically active derivatives and variants of any of these forms of therapeutic proteins. Thus, therapeutic protein include those that (1) have an amino acid sequence that has greater than about 60%, about 65%, about 70%, about 75%, about 80%, about 85%, about 90%, about 91%, about 92%, about 93%, about 94%, about 95%, about 96%, about 97%, about 98% or about 99% or greater amino acid sequence identity, over a region of at least about 25, about 50, about 100, about 200, about 300, about 400, or more amino acids, to a polypeptide encoded by a referenced nucleic acid or an amino acid sequence described herein; and/or (2) specifically bind to antibodies, e.g., polyclonal or monoclonal antibodies, generated against an immunogen comprising a referenced amino acid sequence as described herein, an immunogenic fragment thereof, and/or a conservatively modified variant thereof.

As used herein "biologically active derivative," "biologically active fragment," "biologically active analog" or "biologically active variant" includes any derivative or fragment or analog or variant of a molecule having substantially the same functional and/or biological properties of said molecule, such as binding properties, and/or the same structural basis, such as a peptidic backbone or a basic polymeric unit.

An "analog," such as a "variant" or a "derivative," is a compound substantially similar in structure and having the same biological activity, albeit in certain instances to a differing degree, to a naturally-occurring molecule.

A "derivative," for example, is a type of analog and refers to a polypeptide sharing the same or substantially similar structure as a reference polypeptide that has been modified, e.g., chemically.

A polypeptide variant, for example, is a type of analog and refers to a polypeptide sharing substantially similar structure and having the same biological activity as a reference polypeptide (i.e., "native polypeptide" or "native therapeutic protein"). Variants differ in the composition of their amino acid sequences compared to the naturally-occurring polypeptide from which the variant is derived, based on one or more mutations involving (i) deletion of one or more amino acid residues at one or more termini of the polypeptide and/or one or more internal regions of the naturally-occurring polypeptide sequence (e.g., fragments), (ii) insertion or addition of one or more amino acids at one or more termini (typically an "addition" or "fusion") of the polypeptide and/or one or more internal regions (typically an "insertion") of the naturally-occurring polypeptide sequence or (iii) substitution of one or more amino acids for other amino acids in the naturally-occurring polypeptide sequence.

Variant polypeptides include insertion variants, wherein one or more amino acid residues are added to a therapeutic protein amino acid sequence of the present disclosure. Insertions may be located at either or both termini of the protein, and/or may be positioned within internal regions of the therapeutic protein amino acid sequence. Insertion variants, with additional residues at either or both termini, include for example, fusion proteins and proteins including amino acid tags or other amino acid labels. In one aspect, the therapeutic protein molecule optionally contains an N-terminal Met, especially when the molecule is expressed recombinantly in a bacterial cell such as E. coli.

In deletion variants, one or more amino acid residues in a therapeutic protein polypeptide as described herein are removed. Deletions can be effected at one or both termini of the therapeutic protein polypeptide, and/or with removal of one or more residues within the therapeutic protein amino acid sequence. Deletion variants, therefore, include fragments of a therapeutic protein polypeptide sequence.

In substitution variants, one or more amino acid residues of a therapeutic protein polypeptide are removed and replaced with alternative residues. In one aspect, the substitutions are conservative in nature and conservative substitutions of this type are well known in the art. Alternatively, the present disclosure embraces substitutions that are also non-conservative. Exemplary conservative substitutions are described in Lehninger, [Biochemistry, 2nd Edition; Worth Publishers, Inc., New York (1975), pp.71-77] and are set out immediately below.

**CONSERVATIVE SUBSTITUTIONS**

| **SIDE CHAIN CHARACTERISTIC** | **AMINO ACID** |
|---|---|
| Non-polar (hydrophobic): | |
| A. Aliphatic | A L I V P |
| B. Aromatic | FW |
| C. Sulfur-containing | M |
| D. Borderline | G |

| Uncharged-polar: | |
|---|---|
| A. Hydroxyl | STY |
| B. Amides | NQ |
| C. Sulfhydryl | C |
| D. Borderline | G |
| Positively charged (basic) | KRH |
| Negatively charged (acidic) | DE |

Alternatively, exemplary conservative substitutions are set out immediately below.

**CONSERVATIVE SUBSTITUTIONS II**

| **ORIGINAL RESIDUE** | **EXEMPLARY SUBSTITUTION** |
|---|---|
| Ala (A) | Val, Leu, Ile |
| Arg (R) | Lys, Gln, Asn |
| Asn (N) | Gln, His, Lys, Arg |
| Asp (D) | Glu |
| Cys (C) | Ser |
| Gln (Q) | Asn |
| Glu (E) | Asp |
| His (H) | Asn, Gln, Lys, Arg |
| Ile (I) | Leu, Val, Met, Ala, Phe, |
| Leu (L) | Ile, Val, Met, Ala, Phe |
| Lys (K) | Arg, Gln, Asn |
| Met (M) | Leu, Phe, Ile |
| Phe (F) | Leu, Val, Ile, Ala |
| Pro (P) | Gly |
| Ser (S) | Thr |
| Thr (T) | Ser |
| Trp (W) | Tyr |
| Tyr (Y) | Trp, Phe, Thr, Ser |
| Val (V) | Ile, Leu, Met, Phe, Ala |

As described herein, in various embodiments, the therapeutic protein is modified to introduce or delete cysteines, glycosylation sites, or other amino acids with compatible side chains for directed water-soluble polymer attachment. Such modification may be accomplished using standard molecular biological techniques known in the art and can be accomplished recombinantly (e.g., engineering an amino acid sequence to delete or insert one or more cysteines) such that the purified, modified protein comprises the desired sequence. Alternatively, such modification may be accomplished in vitro following production and purification of the protein.

### B. Polynucleotides

Nucleic acids encoding a therapeutic protein of the present disclosure include, for example and without limitation, genes, pre-mRNAs, mRNAs, cDNAs, polymorphic variants, alleles, synthetic and naturally-occurring mutants.

Polynucleotides encoding a therapeutic protein of the present disclosure also include, without limitation, those that (1) specifically hybridize under stringent hybridization conditions to a nucleic acid encoding a referenced amino acid sequence as described herein, and conservatively modified variants thereof; (2) have a nucleic acid sequence that has greater than about 95%, about 96%, about 97%, about 98%, about 99%, or higher nucleotide sequence identity, over a region of at least about 25, about 50, about 100, about 150, about 200, about 250, about 500, about 1000, or more nucleotides (up to the full length sequence of 1218 nucleotides of the mature protein), to a reference nucleic acid sequence as described herein. Exemplary "stringent hybridization" conditions include hybridization at 42oC in 50% formamide, 5X SSC, 20 mM Na•PO4, pH 6.8; and washing in IX SSC at 55oC for 30 minutes. It is understood that variation in these exemplary conditions can be made based on the length and GC nucleotide content of the sequences to be hybridized. Formulas standard in the art are appropriate for determining appropriate hybridization conditions. See Sambrook et al., Molecular Cloning: A Laboratory Manual (Second ed., Cold Spring Harbor Laboratory Press, 1989) §§ 9.47-9.51.

### C. Production of therapeutic proteins

A "naturally-occurring" polynucleotide or polypeptide sequence is typically from a mammal including, but not limited to, primate, e.g., human; rodent, e.g., rat, mouse, hamster; cow, pig, horse, sheep, or any mammal. The nucleic acids and proteins of the present disclosure can be recombinant molecules (e.g., heterologous and encoding the wild type sequence or a variant thereof, or non-naturally occurring). In various embodiments, a naturally-occurring therapeutic protein is purified from blood or blood plasma samples obtained from a human.

Production of a therapeutic protein includes any method known in the art for (i) the production of recombinant DNA by genetic engineering, (ii) introducing recombinant DNA into prokaryotic or eukaryotic cells by, for example and without limitation, transfection, electroporation or microinjection, (iii) cultivating said transformed cells, (iv) expressing therapeutic protein, e.g. constitutively or upon induction, and (v) isolating said blood coagulation protein, e.g. from the culture medium or by harvesting the transformed cells, in order to obtain purified therapeutic protein.

In other aspects, the therapeutic protein is produced by expression in a suitable prokaryotic or eukaryotic host system characterized by producing a pharmacologically acceptable blood coagulation protein molecule. Examples of eukaryotic cells are mammalian cells, such as CHO, COS, HEK 293, BHK, SK-Hep, and HepG2.

A wide variety of vectors are used for the preparation of the therapeutic protein and are selected from eukaryotic and prokaryotic expression vectors. Examples of vectors for prokaryotic expression include plasmids such as, and without limitation, pRSET, pET, and pBAD, wherein the promoters used in prokaryotic expression vectors include one or more of, and without limitation, lac, trc, trp, recA, or araBAD. Examples of vectors for eukaryotic expression include: (i) for expression in yeast, vectors such as, and without limitation, pAO, pPIC, pYES, or pMET, using promoters such as, and without limitation, AOX1, GAP, GAL1, or AUG1; (ii) for expression in insect cells, vectors such as and without limitation, pMT, pAc5, pIB, pMIB, or pBAC, using promoters such as and without limitation PH, p10, MT, Ac5, OpIE2, gp64, or polh, and (iii) for expression in mammalian cells, vectors such as and without limitation pSVL, pCMV, pRc/RSV, pcDNA3, or pBPV, and vectors derived from, in one aspect, viral systems such as and without limitation vaccinia virus, adeno-associated viruses, herpes viruses, or retroviruses, using promoters such as and without limitation CMV, SV40, EF-1, UbC, RSV, ADV, BPV, and β-actin.

### D. Administration

In one embodiment a conjugated therapeutic protein of the present disclosure may be administered by injection, such as intravenous, intramuscular, or intraperitoneal injection.

To administer compositions comprising a conjugated therapeutic protein of the present disclosure to human or test animals, in one aspect, the compositions comprise one or more pharmaceutically acceptable carriers. The terms "pharmaceutically" or "pharmacologically acceptable" refer to molecular entities and compositions that are stable, inhibit protein degradation such as aggregation and cleavage products, and in addition do not produce allergic, or other adverse reactions when administered using routes well-known in the art, as described below. "Pharmaceutically acceptable carriers" include any and all clinically useful solvents, dispersion media, coatings, antibacterial and antifungal agents, isotonic and absorption delaying agents and the like, including those agents disclosed above.

As used herein, "effective amount" includes a dose suitable for treating a disease or disorder or ameliorating a symptom of a disease or disorder. In one embodiment, "effective amount" includes a dose suitable for treating a mammal having an autosomal recessive disorder leading to A1PI deficiency as described herein. In one embodiment, "effective amount" includes a dose suitable for treating a mammal having a bleeding disorder as described herein. As used herein, "effective amount" also includes a dose suitable for treating a mammal having a bleeding disorder as described herein.

The compositions may be administered orally, topically, transdermally, parenterally, by inhalation spray, vaginally, rectally, or by intracranial injection. The term parenteral as used herein includes subcutaneous injections, intravenous, intramuscular, intracisternal injection, or infusion techniques. Administration by intravenous, intradermal, intramuscular, intramammary, intraperitoneal, intrathecal, retrobulbar, intrapulmonary injection and or surgical implantation at a particular site is contemplated as well. Generally, compositions are essentially free of pyrogens, as well as other impurities that could be harmful to the recipient.

Single or multiple administrations of the compositions can be carried out with the dose levels and pattern being selected by the treating physician. For the prevention or treatment of disease, the appropriate dosage will depend on the type of disease to be treated, as described above, the severity and course of the disease, whether drug is administered for preventive or therapeutic purposes, previous therapy, the patient's clinical history and response to the drug, and the discretion of the attending physician.

The present disclosure also relates to a pharmaceutical composition comprising an effective amount of a conjugated therapeutic protein as defined herein. The pharmaceutical composition may further comprise a pharmaceutically acceptable carrier, diluent, salt, buffer, or excipient. The pharmaceutical composition can be used for treating the above-defined bleeding disorders. The pharmaceutical composition of the present disclosure may be a solution or a lyophilized product. Solutions of the pharmaceutical composition may be subjected to any suitable lyophilization process.

As an additional aspect, the present disclosure includes kits which comprise a composition of the present disclosure packaged in a manner which facilitates its use for administration to subjects. In one embodiment, such a kit includes a compound or composition described herein (e.g., a composition comprising a conjugated therapeutic protein), packaged in a container such as a sealed bottle or vessel, with a label affixed to the container or included in the package that describes use of the compound or composition in practicing the method. In one embodiment, the kit contains a first container having a composition comprising a conjugated therapeutic protein and a second container having a physiologically acceptable reconstitution solution for the composition in the first container. In one aspect, the compound or composition is packaged in a unit dosage form. The kit may further include a device suitable for administering the composition according to a specific route of administration. Preferably, the kit contains a label that describes use of the therapeutic protein or peptide composition.

### WATER SOLUBLE POLYMERS

In one embodiment of the instant disclosure, a therapeutic protein conjugate molecule is bound to a water-soluble polymer including, but not limited to, polyethylene glycol (PEG), branched PEG, PolyPEG® (Warwick Effect Polymers; Coventry, UK), polysialic acid (PSA), starch, hydroxylethyl starch (HES), hydroxyalkyl starch (HAS), carbohydrate, polysaccharides, pullulane, chitosan, hyaluronic acid, chondroitin sulfate, dermatan sulfate, starch, dextran, carboxymethyl-dextran, polyalkylene oxide (PAO), polyalkylene glycol (PAG), polypropylene glycol (PPG) polyoxazoline, poly acryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC), and functional derivatives thereof.

According to various embodiments of the instant disclosure, water soluble polymers may be modified or derivatized by attaching a functional linker or a specific and desired end group chemistry to enable such a water-soluble polymer derivative to attach in a site specific manner to a therapeutic protein (with at least one accessible site compatible with such end group chemistry). For example, water-soluble polymer functional derivatives such as N-hydroxysuccinimide ester-PEG (NHS-PEG), PEG carbonate, PEG aldehydes, aminooxy-PEG, PEG hydrazide (PEG-Hz), PEG hydrazine, PEG maleimide (MAL-PEG), PEG thiol (PEG-SH), Amino PEG (PEG -NH2), Carboxyl PEG (PEG-COOH), Hydroxyl PEG (PEG-OH), PEG epoxide, oxidized PSA, aminooxy-PSA, PSA hydrazide, PEG vinylsulfone, PEG orthpyridyl-disulfide (OPSS), PEG ioacetamide, PEG benzotriazole, PSA-SH, MAL-PSA, PSA hydrazide, PSA hydrazine and PSA-NH2 are contemplated by the present disclosure.

In one embodiment of the present disclosure, the water soluble polymer has a molecular weight range of 350 to 150,000, 500 to 100,000, 1000 to 80,000, 1500 to 60,000, 2,000 to 45,000 Da, 3,000 to 35,000 Da, and 5,000 to 25,000 Da. In various embodiments, the water-soluble polymer is a PEG or PSA with a molecular weight of 10,000, 20,000 , 30,000 , 40,000 , 50,000 , 60,000 , 70,000 , or 80,000 Da. In one embodiment, the water-soluble polymer is a PEG or PSA with a molecular weight of 20, 000 Da.

In one embodiment, the therapeutic protein derivative retains the full functional activity of native therapeutic protein products, and provides an extended half-life in vivo, as compared to native therapeutic protein products. In another embodiment of the present disclosure, the half-life of the construct is decreased or increased 0.5, 0.6, 0.7, 0.8, 0.9, 1.0, 1.1, 1.2, 1.3, 1.4, 1.5, 2, 3, 4, 5, 6, 7, 8, 9, or 10-fold relative to the in vivo half-life of native therapeutic protein.

In another embodiment, the therapeutic protein derivative retains at least 10, 11, 12, 13, 14, 15, 16, 17, 18, 19, 20, 21, 22, 23, 24, 25, 26, 27, 28, 29, 30, 31, 32, 34, 35, 36, 37, 38, 39, 40, 41, 42, 43, 44. 45, 46, 47, 48, 49, 50, 51, 52, 53, 54, 55, 56,57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, or 150 percent (%) biological activity relative to native blood coagulation protein.

In one embodiment, the biological activity of, for example a conjugated A1PI protein and native A1PI protein are determined by a neutrophil elastase inhibitory capacity assay (Travis J, Johnson D (1981): Method Enzymol 80, 754-764).

In one embodiment, the biological activity of the derivative and native blood coagulation protein (e.g., FVII) are determined by the ratios of chromogenic activity to blood coagulation factor antigen value (blood coagulation factor:Chr: blood coagulation factor:Ag).

### A. Sialic acid and PSA

PSAs consist of polymers (generally homopolymers) of N-acetylneuraminic acid. The secondary amino group normally bears an acetyl group, but it may instead bear a glycolyl group. Possible substituents on the hydroxyl groups include acetyl, lactyl, ethyl, sulfate, and phosphate groups.

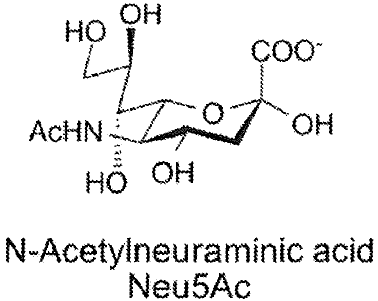

### Structure of sialic acid (N-acetylneuraminic acid)

PSAs and modified PSAs (mPSAs) generally comprise linear polymers consisting essentially of N-acetylneuraminic acid moieties linked by 2,8- or 2,9- glycosidic linkages or combinations of these (e.g. alternating 2,8- and 2,9- linkages). In particularly preferred PSAs and mPSAs, the glycosidic linkages are α-2,8. Such PSAs and mPSAs are conveniently derived from colominic acids, and are referred to herein as "CAs" and "mCAs". Typical PSAs and mPSAs comprise at least 2, preferably at least 5, more preferably at least 10 and most preferably at least 20 N-acetylneuraminic acid moieties. Thus, they may comprise from 2 to 300 N-acetylneuraminic acid moieties, preferably from 5 to 200 N-acetylneuraminic acid moieties, or most preferably from 10 to 100 N-acetylneuraminic acid moieties. PSAs and CAs preferably are essentially free of sugar moieties other than N-acetylneuraminic acid. Thus PSAs and CAs preferably comprise at least 90%, more preferably at least 95% and most preferably at least 98% N-acetylneuraminic acid moieties.

Where PSAs and CAs comprise moieties other than N-acetylneuraminic acid (as, for example in mPSAs and mCAs) these are preferably located at one or both of the ends of the polymer chain. Such "other" moieties may, for example, be moieties derived from terminal N-acetylneuraminic acid moieties by oxidation or reduction.

For example, WO 2001/087922 describes such mPSAs and mCAs in which the non-reducing terminal N-acetylneuraminic acid unit is converted to an aldehyde group by reaction with sodium periodate. Additionally, WO 2005/016974 describes such mPSAs and mCAs in which the reducing terminal N-acetylneuraminic acid unit is subjected to reduction to reductively open the ring at the reducing terminal N-acetylneuraminic acid unit, whereby a vicinal diol group is formed, followed by oxidation to convert the vicinal diol group to an aldehyde group.

Different PSA derivatives can be prepared from oxidized PSA containing a single aldehyde group at the non reducing end. The preparation of aminooxy PSA is described below in Example 5, the preparation of PSA maleimide is described below in Example 14. PSA-NH2 containing a terminal amino group can be prepared by reductive amination with NH4Cl and PSA-SH containing a terminal sulfhydryl group by reaction of PSA-NH2 with 2-iminothiolane (Traut's reagent), both procedures are described in US 7,645,860 B2. PSA hydrazine can be prepared by reaction of oxidized PSA with hydrazine according to US 7,875,708 B2. PSA hydrazide can be prepared by reaction of oxidized PSA with adipic acid dihydrazide (WO 2011/012850 A2).

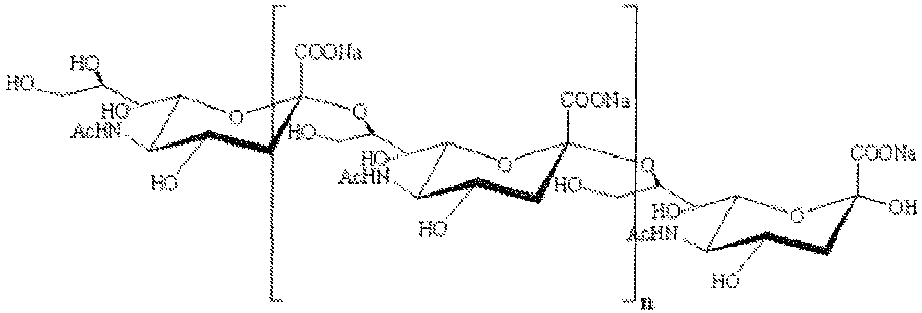

### Structure of colominic acid (homopolymer of N-acetylneuraminic acid)

Colominic acids (a sub-class of PSAs) are homopolymers of N-acetylneuraminic acid (NANA) with α (2→8) ketosidic linkage, and are produced, inter alia, by particular strains of Escherichia coli possessing K1 antigen. Colominic acids have many physiological functions. They are important as a raw material for drugs and cosmetics.

Comparative studies in vivo with polysialylated and unmodified asparaginase revealed that polysialylation increased the half-life of the enzyme (Fernandes and Gregoriadis, Biochimica Biophysica Acta 1341: 26-34, 1997).

As used herein, "sialic acid moieties" includes sialic acid monomers or polymers ("polysaccharides") which are soluble in an aqueous solution or suspension and have little or no negative impact, such as side effects, to mammals upon administration of the PSA-blood coagulation protein conjugate in a pharmaceutically effective amount. The polymers are characterized, in one aspect, as having 1, 2, 3, 4, 5, 10, 20, 30, 40, 50, 60, 70, 80, 90, 100, 200, 300, 400, or 500 sialic acid units. In certain aspects, different sialic acid units are combined in a chain.

In one embodiment of the present disclosure, the sialic acid portion of the polysaccharide compound is highly hydrophilic, and in another embodiment the entire compound is highly hydrophilic. Hydrophilicity is conferred primarily by the pendant carboxyl groups of the sialic acid units, as well as the hydroxyl groups. The saccharide unit may contain other functional groups, such as, amine, hydroxyl or sulphate groups, or combinations thereof. These groups may be present on naturally-occurring saccharide compounds, or introduced into derivative polysaccharide compounds.

The naturally occurring polymer PSA is available as a polydisperse preparation showing a broad size distribution (e.g. Sigma C-5762) and high polydispersity (PD). Because the polysaccharides are usually produced in bacteria carrying the inherent risk of copurifying endotoxins, the purification of long sialic acid polymer chains may raise the probability of increased endotoxin content. Short PSA molecules with 1-4 sialic acid units can also be synthetically prepared (Kang SH et al., Chem Commun. 2000;227-8; Ress DK and Linhardt RJ, Current Organic Synthesis. 2004;1:31-46), thus minimizing the risk of high endotoxin levels. However PSA preparations with a narrow size distribution and low polydispersity, which are also endotoxin-free, can now be manufactured. Polysaccharide compounds of particular use for the present disclosure are, in one aspect, those produced by bacteria. Some of these naturally-occurring polysaccharides are known as glycolipids. In one embodiment, the polysaccharide compounds are substantially free of terminal galactose units.

### B. Polyethylene glycol (PEG) and PEGylation

In certain aspects, therapeutic proteins are conjugated to a water soluble polymer by any of a variety of chemical methods (Roberts JM et al., Advan Drug Delivery Rev 2002;54:459-76). For example, in one embodiment a therapeutic protein is modified by the conjugation of PEG to free amino groups of the protein using N-hydroxysuccinimide (NHS) esters. In another embodiment the water soluble polymer, for example PEG, is coupled to free SH groups using maleimide chemistry or the coupling of PEG hydrazides or PEG amines to carbohydrate moieties of the therapeutic protein after prior oxidation.

The conjugation is in one aspect performed by direct coupling (or coupling via linker systems) of the water soluble polymer to a therapeutic protein under formation of stable bonds. In addition degradable, releasable or hydrolysable linker systems are used in certain aspects the present disclosure (Tsubery et al., J Biol Chem 2004;279:38118-24 / Greenwald et al., J Med Chem 1999;42:3657-67 / Zhao et al., Bioconj Chem 2006;17:341-51 / WO2006/138572A2 / US7259224B2 / US7060259B2).

In various embodiments of the present disclosure, a therapeutic protein is modified via lysine residues by use of polyethylene glycol derivatives containing an active N-hydroxysuccinimide ester (NHS) such as succinimidyl succinate, succinimidyl glutarate or succinimidyl propionate. These derivatives react with the lysine residues of the therapeutic protein under mild conditions by forming a stable amide bond. In addition lysine residues can be modified by reductive amination with PEG aldehydes in the presence of NaCNBH3 to form a secondary amine bond. Carbohydrate residues (predominantly N-glycans) can be modified with aminooxy PEG or PEG hydrazide after prior oxidation. Free SH groups in proteins can react with PEG maleimide, PEG vinylsulfones, PEG orthopyridyl-disulfides and PEG iodacetamides. An overview of PEG chemistry is given by Roberts et al. (Adv Drug Deliv Rev 2002;54:459-76).

In various embodiments of the present disclosure, the chain length of the PEG derivative is 5,000 Da. Other PEG derivatives with chain lengths of 500 to 2,000 Da, 2,000 to 5,000 Da, greater than 5,000 up to 10,000 Da or greater than 10,000 up to 20,000 Da, or greater than 20,000 up to 150,000 Da are used in various embodiments, including linear and branched structures. In one embodiment of the present disclosure, the chain length of the PEG derivative is 20,000 Da.

Alternative methods for the PEGylation of amino groups are, without limitation, the chemical conjugation with PEG carbonates by forming urethane bonds, or the reaction with aldehydes or ketones by reductive amination forming secondary amide bonds.

In various embodiments of the present disclosure a therapeutic protein molecule is chemically modified using PEG derivatives that are commercially available. These PEG derivatives in alternative aspects have a linear or branched structures. Examples of PEG-derivatives containing NHS groups are listed below.

The following PEG derivatives are non-limiting examples of those commercially available from Nektar Therapeutics (Huntsville, Ala.; see www.nektar.com/PEG reagent catalog; Nektar Advanced PEGylation, price list 2005-2006):
mPEG-Succinimidyl propionate (mPEG-SPA) 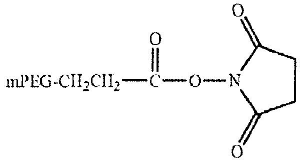
mPEG-Succinimidyl α-methylbutanoate (mPEG-SMB) 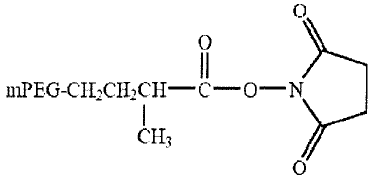
mPEG-CM-HBA-NHS (CM=carboxymethyl; HBA=Hydroxy butyric acid)

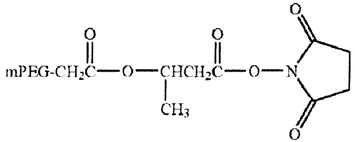

### Structure of a Branched PEG-derivative (Nektar Therapeutics):

### Branched PEG N-Hydroxysuccinimide (mPEG2-NHS)

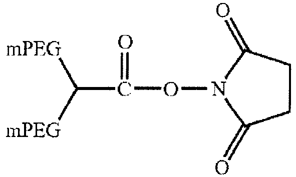

This reagent with branched structure is described in more detail by Kozlowski et al. (BioDrugs 2001;5:419-29).

Other non-limiting examples of PEG derivatives are commercially available from NOF Corporation (Tokyo, Japan; see www.nof.co.jp/english: Catalogue 2005)

### General Structure of Linear PEG-derivatives (NOF Corp.):

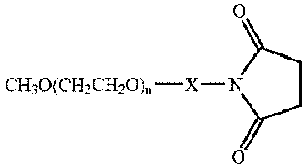

X=carboxymethyl 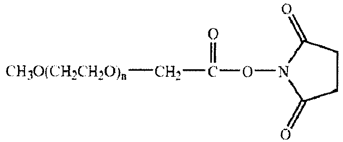
X=carboxypentyl 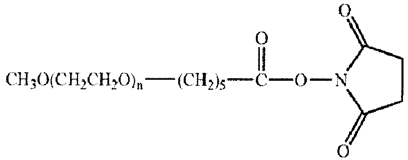
x=succinate 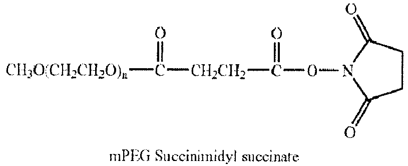
x=glutarate

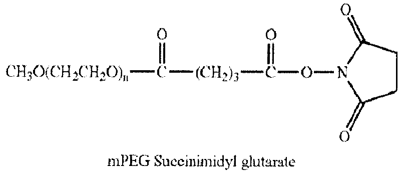

### Structures of Branched PEG-derivatives (NOF Corp.): 2,3-Bis(methylpolyoxyethylene-oxy)-1-(1,5-dioxo-5-succinimidyloxy, pentyloxy)propane

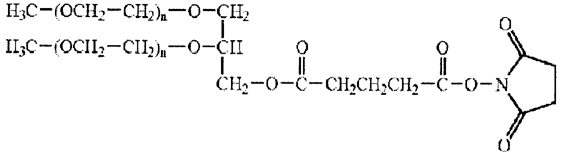

### 2,3-Bis(methylpolyoxyethylene-oxy)-1-(succinimidyl carboxypentyloxy)propane

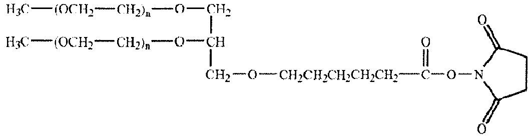

These propane derivatives show a glycerol backbone with a 1,2 substitution pattern. In the present disclosure branched PEG derivatives based on glycerol structures with 1,3 substitution or other branched structures described in US2003/0143596A1 are also contemplated.

PEG derivatives with degradable (for example, hydrolysable linkers) as described by Tsubery et al. (J Biol Chem 2004;279:38118-24) and Shechter et al. (WO04089280A3) are also contemplated.

### C. Hydroxyalkyl starch (HAS) and hydroxylethyl starch (HES)

In various embodiments of the present disclosure, a therapeutic protein molecule is chemically modified using hydroxyalkyl starch (HAS) or hydroxylethyl starch (HES) or derivatives thereof.

HES is a derivative of naturally occurring amylopectin and is degraded by alpha-amylase in the body. HES is a substituted derivative of the carbo- hydrate polymer amylopectin, which is present in corn starch at a concentration of up to 95 % by weight. HES exhibits advantageous biological properties and is used as a blood volume replacement agent and in hemodilution therapy in the clinics (Sommermeyer et al., 1987, Krankenhauspharmazie, 8 (8), 271-278; and Weidler et al., 1991, Arzneim.-Forschung/Drug Res., 41, 494-498).

Amylopectin consists of glucose moieties, wherein in the main chain alpha-1,4-glycosidic bonds are present and at the branching sites alpha-1, 6-glycosidic bonds are found. The physical-chemical properties of this molecule are mainly determined by the type of glycosidic bonds. Due to the nicked alpha-1,4-glycosidic bond, helical structures with about six glucose-monomers per turn are produced. The physicochemical as well as the biochemical properties of the polymer can be modified via substitution. The introduction of a hydroxyethyl group can be achieved via alkaline hydroxyethylation. By adapting the reaction conditions it is possible to exploit the different reactivity of the respective hydroxy group in the unsubstituted glucose monomer with respect to a hydroxyethylation. Owing to this fact, the skilled person is able to influence the substitution pattern to a limited extent.

HAS refers to a starch derivative which has been substituted by at least one hydroxyalkyl group. Therefore, the term hydroxyalkyl starch is not limited to compounds where the terminal carbohydrate moiety comprises hydroxyalkyl groups R1, R2, and/or R3, but also refers to compounds in which at least one hydroxy group present anywhere, either in the terminal carbohydrate moiety and/or in the remaining part of the starch molecule, HAS', is substituted by a hydroxyalkyl group R1, R2, or R3.

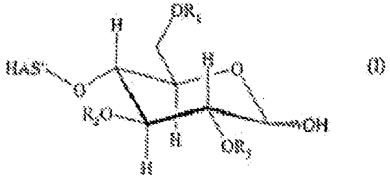

The alkyl group may be a linear or branched alkyl group which may be suitably substituted. Preferably, the hydroxyalkyl group contains 1 to 10 carbon atoms, more preferably from 1 to 6 carbon atoms, more preferably from 1 to 4 carbon atoms, and even more preferably 2-4 carbon atoms. "Hydroxyalkyl starch" therefore preferably comprises hydroxyethyl starch, hydroxypropyl starch and hydroxybutyl starch, wherein hydroxyethyl starch and hydroxypropyl starch are particularly preferred.

Hydroxyalkyl starch comprising two or more different hydroxyalkyl groups is also comprised in the present disclosure. The at least one hydroxyalkyl group comprised in HAS may contain two or more hydroxy groups. According to one embodiment, the at least one hydroxyalkyl group comprised HAS contains one hydroxy group.

The term HAS also includes derivatives wherein the alkyl group is mono-or polysubstituted. In one embodiment, the alkyl group is substituted with a halogen, especially fluorine, or with an aryl group, provided that the HAS remains soluble in water. Furthermore, the terminal hydroxy group a of hydroxyalkyl group may be esterified or etherified. HAS derivatives are described in WO/2004/024776, which is incorporated by reference in its entirety.

### D. Methods of attachment

A therapeutic protein may be covalently linked to the polysaccharide compounds by any of various techniques known to those of skill in the art.

The coupling of the water soluble polymer can be carried out by direct coupling to the protein or via linker molecules. One example of a chemical linker is MBPH (4-[4-N-Maleimidophenyl]butyric acid hydrazide) containing a carbohydrate-selective hydrazide and a sulfhydryl-reactive maleimide group (Chamow et al., J Biol Chem 1992;267:15916-22). Other exemplary and preferred linkers are described below.

In various aspects of the present disclosure, sialic acid moieties are bound to a therapeutic protein, e.g., albumin, A1PI, FVIIa or other members of the serpin or blood coagulation factor protein families for example by the method described in US Patent No. 4,356,170, which is herein incorporated by reference.

Other techniques for coupling PSA to polypeptides are also known and contemplated by the present disclosure. For example, US Publication No. 2007/0282096 describes conjugating an amine or hydrazide derivative of, e.g., PSA, to proteins. In addition, US Publication No. 2007/0191597 describes PSA derivatives containing an aldehyde group for reaction with substrates (e.g., proteins) at the reducing end. These references are incorporated by reference in their entireties.

In addition, various methods are disclosed at column 7, line 15, through column 8, line 5 of U.S. Patent No. 5,846,951 (incorporated by reference in its entirety). Exemplary techniques include linkage through a peptide bond between a carboxyl group on one of either the blood coagulation protein or polysaccharide and an amine group of the blood coagulation protein or polysaccharide, or an ester linkage between a carboxyl group of the blood coagulation protein or polysaccharide and a hydroxyl group of the therapeutic protein or polysaccharide. Another linkage by which the therapeutic protein is covalently bonded to the polysaccharide compound is via a Schiff base, between a free amino group on the blood coagulation protein being reacted with an aldehyde group formed at the non-reducing end of the polysaccharide by periodate oxidation (Jennings HJ and Lugowski C, J Immunol. 1981;127:1011-8; Fernandes AI and Gregoriadis G, Biochim Biophys Acta. 1997;1341:26-34). The generated Schiff base is in one aspect stabilized by specific reduction with NaCNBH3 to form a secondary amine. An alternative approach is the generation of terminal free amino groups in the PSA by reductive amination with NH4Cl after prior oxidation. Bifunctional reagents can be used for linking two amino or two hydroxyl groups. For example, PSA containing an amino group is coupled to amino groups of the protein with reagents like BS3 (Bis(sulfosuccinimidyl)suberate / Pierce, Rockford, IL). In addition heterobifunctional cross linking reagents like Sulfo-EMCS (N-ε-Maleimidocaproyloxy) sulfosuccinimide ester / Pierce) is used for instance to link amine and thiol groups.

In another approach, a PSA hydrazide is prepared and coupled to the carbohydrate moiety of the protein after prior oxidation and generation of aldehyde functions.

As described above, a free amine group of the therapeutic protein reacts with the 1-carboxyl group of the sialic acid residue to form a peptidyl bond or an ester linkage is formed between the 1-carboxylic acid group and a hydroxyl or other suitable active group on a blood coagulation protein. Alternatively, a carboxyl group forms a peptide linkage with deacetylated 5-amino group, or an aldehyde group of a molecule of a therapeutic protein forms a Schiff base with the N-deacetylated 5-amino group of a sialic acid residue.

The above description can be applied to PEG insofar as the reactive groups are the same for PEG and PSA.

Alternatively, the water soluble polymer is associated in a non-covalent manner with a therapeutic protein. For example, the water soluble polymer and the pharmaceutically active compound are in one aspect linked via hydrophobic interactions. Other non-covalent associations include electrostatic interactions, with oppositely charged ions attracting each other.

In various embodiments, the therapeutic protein is linked to or associated with the water soluble polymer in stoichiometric amounts (e.g., 1:1, 1:2, 1:3, 1:4, 1:5, 1:6, 1:7, 1:7, 1:8, 1:9, or 1:10, etc.). In various embodiments, 1-6, 7-12 or 13-20 water soluble polymers are linked to the therapeutic protein. In still other embodiments, 1, 2, 3, 4, 5, 6, 7, 8, 9, 10, 11, 12, 13, 14, 15, 16, 17, 18, 19, 20 or more water soluble polymers are linked to the therapeutic protein. In one embodiment, a single water soluble polymer is linked to the therapeutic protein. In another embodiment, a single water soluble polymer is linked to the therapeutic protein via a cysteine residue.

Moreover, the therapeutic protein, prior to conjugation to a water soluble polymer via one or more carbohydrate moieties, may be glycosylated in vivo or in vitro. These glycosylated sites can serve as targets for conjugation of the proteins with water soluble polymers (US Patent Application No. 20090028822, US Patent Application No. 2009/0093399, US Patent Application No. 2009/0081188, US Patent Application No. 2007/0254836, US Patent Application No. 2006/0111279, and DeFrees S. et al., Glycobiology, 2006, 16, 9, 833-43).

### E. Aminooxy linkage

In one embodiment, the reaction of hydroxylamine or hydroxylamine derivatives with aldehydes (e.g., on a carbohydrate moiety following oxidation by sodium periodate) to form an oxime group is applied to the preparation of conjugates of blood coagulation protein. For example, a glycoprotein (e.g., a therapeutic protein according to the present disclosure that has been glycosylated or is capable of being glycosylated) is first oxidized with a oxidizing agent such as sodium periodate (NaIO4) (Rothfus JA and Smith EL., J Biol Chem 1963, 238, 1402-10; and Van Lenten L and Ashwell G., J Biol Chem 1971, 246, 1889-94). The periodate oxidation of glycoproteins is based on the classical Malaprade reaction described in 1928, the oxidation of vicinal diols with periodate to form an active aldehyde group (Malaprade L., Analytical application, Bull Soc Chim France, 1928, 43, 683-96). Additional examples for such an oxidizing agent are lead tetraacetate (Pb(OAc)4), manganese acetate (MnO(Ac)3), cobalt acetate (Co(OAc)2), thallium acetate (TlOAc), cerium sulfate (Ce(SO4)2) (US 4,367,309) or potassium perruthenate (KRuO4) (Marko et al., J Am Chem Soc 1997,119, 12661-2). By "oxidizing agent" a mild oxidizing compound which is capable of oxidizing vicinal diols in carbohydrates, thereby generating active aldehyde groups under physiological reaction conditions is meant.

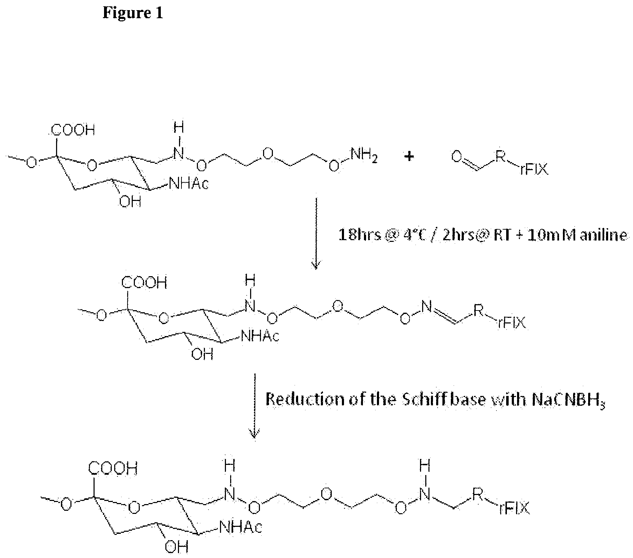

The second step is the coupling of the polymer containing an aminooxy group to the oxidized carbohydrate moiety to form an oxime linkage. In various embodiments of the present disclosure, this step can be carried out in the presence of catalytic amounts of the nucleophilic catalyst aniline or aniline derivatives (Dirksen A and Dawson PE, Bioconjugate Chem. 2008; Zeng Y et al., Nature Methods 2009;6:207-9). The aniline catalysis dramatically accelerates the oxime ligation allowing the use of very low concentrations of the reagents. In another embodiment of the present disclosure the oxime linkage is stabilized by reduction with NaCNBH3 to form an alkoxyamine linkage (Figure 1). Additional catalysts are described below.

In various embodiments, the reaction steps to conjugate a water soluble polymer to a therapeutic protein are carried out separately and sequentially (i.e., starting materials (e.g., therapeutic protein, water soluble polymer, etc), reagents (e.g., oxidizing agents, aniline, etc) and reaction products (e.g., oxidized carbohydrate on a therapeutic protein, activated aminooxy water soluble polymer, etc) are separated between individual reaction steps). In another embodiment, the starting materials and reagents (e.g., therapeutic protein, water-soluble polymer, thiol reductant, oxidizing agent, etc.) necessary to complete a conjugation reaction according to the present disclosure is carried out in a single vessel (i.e., "a simultaneous reaction"). In one embodiment the native therapeutic protein is mixed with the aminooxy- polymer reagent. Subsequently the oxidizing reagent is added and the conjugation reaction is performed.

Additional information on aminooxy technology can be found in the following references, each of which is incorporated in their entireties: EP 1681303A1 (HASylated erythropoietin); WO 2005/014024 (conjugates of a polymer and a protein linked by an oxime linking group); WO96/40662 (aminooxy-containing linker compounds and their application in conjugates); WO 2008/025856 (Modified proteins); Peri F et al., Tetrahedron 1998, 54, 12269-78; Kubler-Kielb J and. Pozsgay V., J Org Chem 2005, 70, 6887-90; Lees A et al., Vaccine 2006, 24(6), 716-29; and Heredia KL et al., Macromoecules 2007, 40(14), 4772-9.

In various embodiments, the water soluble polymer which is linked according to the aminooxy technology described herein to an oxidized carbohydrate moiety of a therapeutic protein (e.g., A1PI, FVIIa, or other members of the serpin or blood coagulation factor protein families) include, but are not limited to polyethylene glycol (PEG), branched PEG, PolyPEG®, polysialic acid (PSA), carbohydrate, polysaccharides, pullulane, chitosan, hyaluronic acid, chondroitin sulfate, starch, hydroxylethyl starch (HES), hydroxyalkyl starch (HAS), dermatan sulfate, dextran, carboxymethyl-dextran, polyalkylene oxide (PAO), polyalkylene glycol (PAG), polypropylene glycol (PPG) polyoxazoline, polyacryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC).

### NUCLEOPHILIC CATALYSTS

In various embodiments, the conjugation of water soluble polymers to therapeutic proteins can be catalyzed by aniline or aniline derivatives. Aniline strongly catalyzes aqueous reactions of aldehydes and ketones with amines to form stable imines such as hydrazones and oximes. The following diagram compares an uncatalyzed versus the aniline-catalyzed oxime ligation reaction (Kohler JJ, ChemBioChem 2009;10:2147-50):

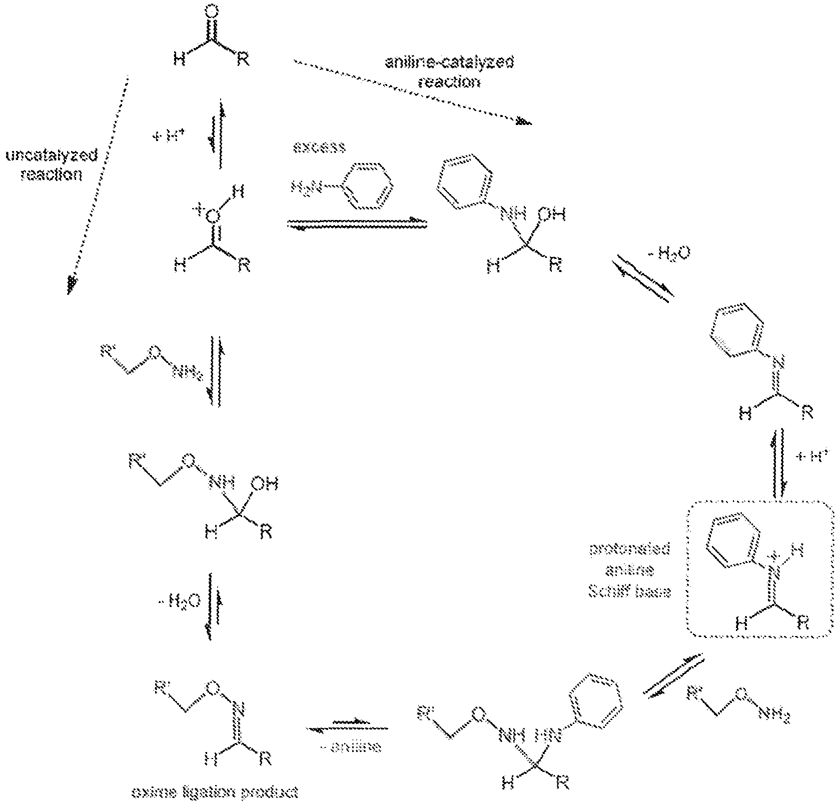

Although aniline catalysis can accelerate the oxime ligation allowing short reaction times and the use of low concentrations of the aminooxy reagent, aniline has toxic properties that must be considered when, for example, the conjugated therapeutic protein is form the basis of a pharmaceutical. For example, aniline has been shown to induce methemoglobinemia (Harrison, J.H.., and Jollow, D.J., Molecular Pharmacology, 32(3) 423-431, 1987). Long-term dietary treatment of rats has been shown to induce tumors in the spleen (Goodman, DG., et al., J Natl Cancer Inst., 73(1):265-73, 1984). In vitro studies have also shown that aniline has the potential to induce chromosome mutations and has the potentially genotoxic activity (Bombhard E.M. and Herbold B, Critical Reviews in Toxicology 35,783-835, 2005).

In various embodiments, aniline derivatives as alternative oxime ligation catalysts are provided. Such aniline derivatives include, but are not limited to, o-amino benzoic acid, m-amino benzoic acid, p-amino benzoic acid, sulfanilic acid, o-aminobenzamide, o-toluidine, m-toluidine, p-toluidine, o-anisidine, m-anisidine, and p-anisidine.

In various embodiments, m-toluidine (aka meta-toluidine, m-methylaniline, 3-methylaniline, or 3-amino-1-methylbenzene) is used to catalyze the conjugation reactions described herein. M-toluidine and aniline have similar physical properties and essentially the same pKa value (m-toluidine:pKa 4.73, aniline:pKa 4.63).

The nucleophilic catalysts of the present disclosure are useful for oxime ligation (e.g, using aminooxy linkage) or hydrazone formation (e.g., using hydrazide chemistry). In various embodiments of the present disclosure, the nucleophilic catalyst is provided in the conjugation reaction at a concentration of 0.1, 0.2, 0.3, 0.5, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 11, 12, 13, 14, 15, 16, 17, 18, 19, 20, 25, 30, 35, 40, 45, or 50 mM. In various embodiments, the nucleophilic catalyst is provided between 1 to 10 mM. In various embodiments of the present disclosure, the pH range of conjugation reaction is 4.5, 5.0, 5.5, 6.0, 6.5, 7.0 and 7.5. In one embodiment, the pH is between 5.5 to 6.5.

### REDUCING AGENTS

In various embodiments of the invention, a mild reduction step is used to reduce an accessible cysteine residue of a therapeutic protein, thereby allowing conjugation of a water-soluble polymer with a sulfhydryl-specific group to the therapeutic protein. As disclosed herein, a reducing agent or "thiol reductant" includes, but is not limited to, Tris[2-carboxyethyl] phosphine hydrochloride (TCEP), dithiothreitol (DTT), dithioerythritol (DTE), sodium borohydride (NaBH4), sodium cyanoborohydride (NaCNBH3), β-mercaptoethanol (BME) and cysteine hydrochloride.

In various embodiments, the maleimide group (MAL) is used for conjugation to the thiol (SH) group of cysteine. One basic prerequisite for this type of modification is a reduced cysteine which is accessible. However, since the cysteine side chain is usually present in an oxidized state in the form of a disulfide bond, reduction with a suitable reductant is carried out before conjugation.

According to the present disclosure, the reductant is used in the lowest possible concentration so as to prevent a possible loss of activity or unfolding of the native form of the protein (Kim, et al., Bioconjugate Chem., 19:786-791 (2008)). In another embodiment, ethylene diamine tetraacetic acid (EDTA) is added to the reduction feed. This helps keep the re-oxidation rate of the reduced SH groups low (Yang, et al., Protein eng., 16:761-770 (2003)).

Although DTT and BME are the most popular reducing agents, as disclosed herein TCEP provides the advantages of being an effective reductive agent having an excellent stability in solution. TCEP can reduce oxidized SH groups without reducing disulfide bridges. The structure of proteins are not affected and this reagent and can therefore be used in a "simultaneous" approach (simultaneous reduction and conjugation reaction in a one-pot reaction) (Hermanson GT, Bioconjugate Techniques. 2nd edition, Elsevier, New York 2008).

In one embodiment of the instant disclosure, an immobilized TCEP reducing gel (Thermo Fisher Scientific, Rockford, IL) is contemplated for use in a "sequential" approach.

In various embodiments, the ratio of reductive agent to SH group (e.g., present on a therapeutic proteins ranges from equimolar up to 100 fold (i.e, 1:100, or 100-fold molar excess). In various embodiments, the amount of reductive agent is 1-fold, 2-fold , 3-fold , 4-fold, 5-fold, 6-fold, 7-fold, 8-fold, 9-fold, 10-fold, 11-fold, 12-fold, 13-fold, 14-fold, 15-fold , 16-fold , 17-fold , 18-fold , 19-fold or 20-fold molar excess relative to the therapeutic protein concentration.

### PURIFICATION OF CONJUGATED PROTEINS

In various embodiments, purification of a protein that has been incubated with an oxidizing agent and/or a therapeutic protein that has been conjugated with a water soluble polymer according to the present disclosure, is desired. Numerous purification techniques are known in the art and include, without limitation, chromatographic methods such as ion-exchange chromatography, hydrophobic interaction chromatography, size exclusion chromatography and affinity chromatography or combinations thereof, filtration methods, and precipitation methods (Guide to Protein Purification, Meth. Enzymology Vol 463 (edited by Burgess RR and Deutscher MP), 2nd edition, Academic Press 2009).

### EXEMPLARY EMBODIMENTS

[KEEP THE FOLLOWING PARAGRAPHS WHICH WERE INCLUDED IN THE PROVISIONAL APPLICATION] The present disclosure provides the following exemplary embodiments:
A1. A method of preparing a therapeutic protein conjugate comprising the step of
   contacting a therapeutic protein, or biologically-active fragment thereof, with a thiol reductant and a water soluble polymer under conditions that (a) produce a reduced cysteine sulfhydryl group on the therapeutic protein, and (b) allow conjugation of the water-soluble polymer to the reduced cysteine sulfhydryl group;
   said therapeutic protein having an amino acid sequence with no more than one accessible cysteine sulhydryl group.
A2. The method of paragraph A1 wherein the amino acid sequence of the therapeutic protein contains no more than one cysteine residue.
A3. The method according to any one of paragraphs A1-A2 comprising a quantity of therapeutic protein between 0.100 and 10.0 gram weight.
A4. The method according to any one of paragraphs A1-A3 wherein the accessible cysteine sulhydryl group is present in a native amino acid sequence of the therapeutic protein.
A5. The method according to any one of paragraphs A1-A3 wherein the amino acid sequence of therapeutic protein is modified to include the accessible cysteine sulfhydryl group.
A6. The method according to any one of paragraphs A1 and A3-A5 wherein the conditions that produce a reduced cysteine sulfhydryl group on the therapeutic protein do not reduce a disulfide bond between other cysteine amino acids in the protein.
A7. The method according to any one of paragraphs A1-A6 wherein the conditions prevent formation of an adduct between the thiol reductant and a water-soluble polymer.
A8. The method according to any one of paragraphs A1-A7 wherein the therapeutic protein is a serpin.
A9. The method according to any one of paragraphs A1-A7 wherein the therapeutic protein is a blood coagulation protein.

### The present disclosure also provides the following exemplary embodiments:

B1. A method of preparing a therapeutic protein conjugate comprising the steps of:
   contacting a therapeutic protein or biologically-active fragment thereof with a thiol reductant under conditions that allow the reduction of a sulfhydryl group on the therapeutic protein, and
   contacting a water-soluble polymer with the therapeutic protein under conditions that allow conjugation of the water-soluble polymer to the reduced sulfhydryl group;
   said therapeutic protein comprising at least one cysteine residue, and
   said therapeutic protein comprising only one cysteine residue which comprises an accessible sulfhydryl group that is completely or partially oxidized, said only one cysteine residue is not involved in a di-sulfide bond with another cysteine residue in the therapeutic protein's amino acid sequence.
B2. The method according to paragraph B1 wherein the thiol reductant concentration is between 1 and 100-fold molar excess relative to the therapeutic protein concentration. In one embodiment the thio reductant concentration is between 1 and 10-fold molar excess relative to the therapeutic protein concentration.
B3. The method according to any one of the previous paragraphs B1-B2 wherein at least 70% of the therapeutic protein conjugate comprises a single water-soluble polymer. In one embodiment, 10-100% of the therapeutic protein conjugate comprises a single water-soluble polymer.
B4. The method according to any one of the previous paragraphs B1-B3 further comprising the step of purifying the therapeutic protein conjugate.
B5. The method according to paragraph B4 wherein the therapeutic protein conjugate is purified using a technique selected from the group consisting of ion-exchange chromatography, hydrophobic interaction chromatography, size exclusion chromatography and affinity chromatography or combinations thereof.
B6. The method according to any one of the previous paragraphs B1-B5 wherein the therapeutic protein, water-soluble polymer and thiol reductant are incubated together in a single vessel, wherein the reduction of the oxidized SH group and the conjugation reaction is carried out simultaneously.
B7. The method according to any one of paragraphs B1-B5 wherein the thiol reductant is removed following incubation with the therapeutic protein and prior to incubating the therapeutic protein with the water-soluble polymer, wherein the reduction of the oxidized SH group and the conjugation reaction is carried out sequentially.
B8. The method according to any one of the previous paragraphs B1-B7 wherein the only one cysteine residue is present in the native amino acid sequence of the therapeutic protein.
B9. The method according to any one of paragraphs B1-B7 wherein the therapeutic protein's amino acid sequence is modified to comprise the only one cysteine residue.
B10. The method according to any one of the previous paragraphs B1-B9 wherein the therapeutic protein is a glycoprotein.
B11. The method according to paragraph B10 wherein the therapeutic protein is glycosylated in vivo.
B12. The method according to paragraph B10 wherein the therapeutic protein is glycosylated in vitro.
B13. The method according to any one of the previous paragraphs B1-B12 wherein the therapeutic protein conjugate has an increased half-life relative to native therapeutic protein.
B14. The method according to paragraph B13 wherein the therapeutic protein conjugate has at least a 1.5-fold increase in half-life relative to native therapeutic protein. In one embodiment, the therapeutic protein conjugate has at least a 1 to 10-fold increase in half-life relative to native therapeutic protein.
B15. The method according to any one of the previous paragraphs B1-B14 wherein the therapeutic protein conjugate retains at least 20% biological activity relative to native therapeutic protein.
B16. The method according to paragraph B15 wherein the therapeutic protein conjugate retains at least 60% biological activity relative to native therapeutic protein. In one embodiment, the therapeutic protein conjugate retains between 10 to 100% biological activity relative to native therapeutic protein.
B17. The method according to any one of the previous paragraphs B1-B16 wherein the thiol reductant is selected from the group consisting of: Tris[2-carboxyethyl] phosphine hydrochloride (TCEP), dithiothreitol (DTT), dithioerythritol (DTE), sodium borohydride (NaBH4), sodium cyanoborohydride (NaCNBH3), β-mercaptoethanol (BME), cysteine hydrochloride and cysteine.
B18. The method according to paragraph B17 wherein the thiol reductant is TCEP.
B19. The method according to any one of the previous paragraphs B1-B18 wherein the water-soluble polymer is selected from the group consisting of linear, branched or multi-arm water soluble polymer.
B20. The method according to any one of the previous paragraphs B1-B19 wherein the water-soluble polymer has a molecular weight between 3,000 and 150,000 Daltons (Da).
B21. The method according to paragraph B20 wherein the water-soluble polymer is linear and has a molecular weight between 10,000 and 50,000 Da. In one embodiment, the water-soluble polymer is linear and has a molecular weight of 20,000.
B22. The method according to any one of the previous paragraphs B1-B21 wherein the water-soluble polymer is selected from the group consisting of polyethylene glycol (PEG), branched PEG, PolyPEG® (Warwick Effect Polymers; Coventry, UK), polysialic acid (PSA), starch, hydroxylethyl starch (HES), hydroxyalkyl starch (HAS), carbohydrate, polysaccharides, pullulane, chitosan, hyaluronic acid, chondroitin sulfate, dermatan sulfate, dextran, carboxymethyl-dextran, polyalkylene oxide (PAO), polyalkylene glycol (PAG), polypropylene glycol (PPG), polyoxazoline, polyacryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC), and functional derivatives thereof.
B23. The method according to paragraph B22 wherein the water soluble polymer is derivatized to contain a sulfhydryl-specific group selected from the group consisting of: maleimide (MAL), vinylsulfones, orthopyridyl-disulfides (OPSS) and iodacetamides.
B24. The method according to paragraph B23 wherein the water soluble polymer is PEG and the sulfhydryl-specific group is MAL.
B25. The method according to paragraph B23 wherein the water soluble polymer is PSA and the sulfhydryl-specific group is MAL.
B26. The method according to any one of the previous paragraphs B1-B25 wherein the therapeutic protein is selected from the group consisting of: alpha-1 proteinase inhibitor (A1PI), antithrombin III, alpha-1-antichymotrypsin, human serum albumin, alcoholdehydrogenase, biliverdin reductase, buturylcholinesterase, complement C5a, cortisol-binding protein, creatine kinase, coagulation factor V (FV), coagulation factor VII (FVII), ferritin, heparin cofactor, interleukin 2, protein C inhibitor, tissue factor and vitronectin.

In one embodiment, the therapeutic protein is selected from the group consisting of: ovalbumin, plasminogen-activator inhibitor, neuroserpin, C1-Inhibitor, nexin, alpha-2-antiplasmin, heparin cofactor II, alpha1-antichymotrypsin, alpha1-microglobulin, coagulation factor VIII (FVIII), and coagulation factor XIII (XIII).

In another embodiment, the therapeutic protein is a protein of the serpin superfamily selected from the group consisting of: A1PI (alpha-1 proteinase inhibitor), or A1AT (alpha-1-antitrypsin), ATR (alpha-1-antitrypsin-related protein), AACT or ACT (alpha-1-antichymotrypsin), PI4 (proteinase inhibitor 4), PCI or PROCI (protein C inhibitor), CBG, (corticosteroid-binding globulin), TBG (thyroxine-binding globulin), AGT (angiotensinogen), centerin, PZI (protein Z-dependent protease inhibitor), PI2 (proteinase inhibitor 2), PAI2 or PLANH2 (plasminogen activator inhibitor-2), SCCA1 (squamous cell carcinoma antigen 1), SCCA2 (squamous cell carcinoma antigen 2), PI5 (proteinase inhibitor 5), PI6 (proteinase inhibitor 6), megsin, PI8 (proteinase inhibitor 8), PI9 (proteinase inhibitor 9), PI10 (proteinase inhibitor 10), epipin, yukopin, PI13 (proteinase inhibitor 13), PI8L1 (proteinase inhibitor 8-like 1), AT3 or ATIII (antithrombin-III), HC-II or HCF2 (heparin cofactor II), PAI1 or PLANH1 (plasminogen activator inhibitor-1), PN1 (proteinase nexin I), PEDF, (pigment epithelium-derived factor), PLI (plasmin inhibitor), C1IN or C1 INH (plasma proteinase C1 inhibitor), CBP1 (collagen-binding protein 1), CBP2 (collagen-binding protein 2), PI12 (proteinase inhibitor 12), and PI14 (proteinase inhibitor 14).

In another embodiment, the therapeutic protein is a blood coagulation factor protein selected from the group consisting of: Factor IX (FIX), Factor VIII (FVIII), Factor VIIa (FVIIa), Von Willebrand Factor (VWF), Factor FV (FV), Factor X (FX), Factor XI (FXI), Factor XII (FXII), thrombin (FII), protein C, protein S, tPA, PAI-1, tissue factor (TF) and ADAMTS 13 protease.
B27. The method according to paragraph B26 wherein the therapeutic protein is A1PI.
B28. A therapeutic protein conjugate produced by the method according to any one of the previous paragraphs B1-B27.
B29. A method of preparing an A1PI conjugate comprising the steps of:
   contacting the A1PI with TCEP under conditions that allow the reduction of a sulfhydryl group on the A1PI, and
   contacting a linear PEG derivatized to contain a MAL group with the A1PI under conditions that allow conjugation of the water-soluble polymer to the reduced sulfhydryl group;
   said A1PI comprising only one cysteine residue which comprises an accessible sulfhydryl group that is completely or partially oxidized, said only one cysteine residue is not involved in a di-sulfide bond with another cysteine residue in the A1PI's amino acid sequence;
   said TCEP concentration is between 3 and 4-fold molar excess relative to the A1PI concentration;
   wherein at least 70% of the A1PI conjugate comprises a single water-soluble polymer;
   said A1PI conjugate having an increased half-life relative to native A1PI; and
   said A1PI conjugate retaining at least 60% biological activity relative to native A1PI.
B30. A method of preparing a serpin conjugate comprising contacting a water-soluble polymer or functional derivative thereof with a serpin or biologically-active fragment thereof under conditions that allow conjugation;
   said water-soluble polymer or functional derivative thereof selected from the group consisting of polysialic acid (PSA), starch, hydroxylethyl starch (HES), hydroxyalkyl starch (HAS), carbohydrate, polysaccharides, pullulane, chitosan, hyaluronic acid, chondroitin sulfate, dermatan sulfate, dextran, carboxymethyl-dextran, PolyPEG® (Warwick Effect Polymers; Coventry, UK), polyalkylene oxide (PAO), polyalkylene glycol (PAG), polypropylene glycol (PPG), polyoxazoline, polyacryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC), N-hydroxysuccinimide ester-PEG (NHS-PEG), PEG carbonate, PEG aldehydes, aminooxy-PEG, PEG hydrazide (PEG-Hz), PEG hydrazine, PEG thiol (PEG-SH), amino PEG (PEG -NH2), carboxyl PEG (PEG-COOH), Hydroxyl PEG (PEG-OH), PEG epoxide, oxidized PSA, aminooxy-PSA, PSA hydrazide (PSA-Hz), PSA hydrazine, PEG vinylsulfone, PEG orthpyridyl-disulfide (OPSS), PEG ioacetamide, PEG benzotriazole, PSA thiol (PSA-SH), MAL-PSA and amino PSA (PSA-NH2).
   said serpin conjugate retaining at least 60% biological activity relative to native glycosylated serpin.
B31. The method according to paragraph B30 wherein at least 70% of the serpin conjugate comprises at least one water-soluble polymer. In one embodiment, 10-100 % of the serpin conjugate comprises at least one water-soluble polymer.
B32. The method according to any one of paragraphs B30-B31 further comprising the step of purifying the serpin conjugate.
B33. The method according to paragraph B32 wherein the serpin conjugate is purified using a technique selected from the group consisting of ion-exchange chromatography, hydrophobic interaction chromatography, size exclusion chromatography and affinity chromatography or combinations thereof.
B34. The method according to any one of paragraphs B30-B33 wherein the serpin is a glycoprotein.
B35. The method according to claim 34 wherein the serpin is glycosylated in vivo.
B36. The method according to paragraph B34 wherein the serpin is glycosylated in vitro.
B37. The method according to any one of paragraphs B30-B36 wherein the serpin conjugate has an increased half-life relative to native therapeutic protein.
B38. The method according to paragraph B37 wherein the serpin conjugate has at least a 1.5-fold increase in half-life relative to native serpin. In one embodiment, the serpin conjugate has between 1 and 10-fold increase in half-life relative to native serpin.
B39. The method according to any one of paragraphs B30-B38 wherein the serpin conjugate retains at least 20% biological activity relative to native therapeutic protein.
B40. The method according to paragraph B39 wherein the serpin conjugate retains at least 60% biological activity relative to native serpin. In one embodiment, the serpin conjugate retains between 10 and 100% biological activity relative to native serpin.
B41. The method according to any one of paragraphs B30-B40 wherein the water-soluble polymer is selected from the group consisting of linear, branched or multi-arm water soluble polymer.
B42. The method according to any one paragraphs B30-B41 wherein the water-soluble polymer has a molecular weight between 3,000 and 150,000 Daltons (Da).
B43. The method according to paragraph B42 wherein the water-soluble polymer is linear and has a molecular weight between 10,000 and 50,000 Da.
B44. The method according to any one of paragraphs B30-B43 wherein the water soluble polymer functional derivative is PEG-NHS.
B45. The method according to any one of paragraphs B30-B43 wherein the water soluble polymer functional derivative is aminooxy-PEG and the serpin is glycosylated.
B46. The method according to any one of paragraphs B30-B43 wherein the water soluble polymer functional derivative is aminooxy-PSA and the serpin is glycosylated.
B47. The method according to any one of paragraphs B45-B46 wherein a carbohydrate moiety of the glycosylated serpin is oxidized by incubation with a buffer comprising an oxidizing agent selected from the group consisting of sodium periodate (NaIO4), lead tetraacetate (Pb(OAc)4) and potassium perruthenate (KRuO4) prior to contacting with the water-soluble polymer functional derivative; wherein an oxime linkage is formed between the oxidized carbohydrate moiety and an active aminooxy group on the water-soluble polymer functional derivative.
B48. The method according to paragraph B47 wherein the oxidizing agent is sodium periodate (NaIO4).
B49. The method according to paragraph B46 wherein the aminooxy-PSA is prepared by reacting an activated aminooxy linker with oxidized PSA;
   wherein the aminooxy linker is selected from the group consisting of:
   a) a 3-oxa-pentane-1,5-dioxyamine linker of the formula: 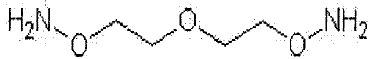
   b) a 3,6,9-trioxa-undecane-1,11-dioxyamine linker of the formula: 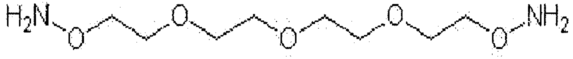 and
   c) a 3,6,9,12,15-penatoxa-heptadecane-1,17-dioxyamine linker of the formula: 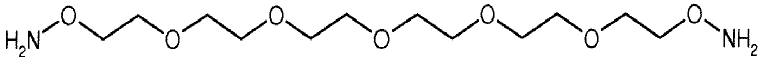 wherein the PSA is oxidized by incubation with a oxidizing agent to form a terminal aldehyde group at the non-reducing end of the PSA.
B50. The method according to any one of paragraphs B47-B49 wherein contacting the oxidized carbohydrate moiety with the activated water soluble polymer functional derivative occurs in a buffer comprising a nucleophilic catalyst selected from the group consisting of aniline, o-amino benzoic acid, m-amino benzoic acid, p-amino benzoic acid, sulfanilic acid, o-aminobenzamide, o-toluidine, m-toluidine, p-toluidine, o-anisidine, m-anisidine, p-anisidine and derivatives thereof.
B51. The method according to any one of paragraphs B47-B50 further comprising the step of reducing the oxime linkage by incubating the therapeutic protein in a buffer comprising a reducing compound selected from the group consisting of sodium cyanoborohydride (NaCNBH3) and ascorbic acid (vitamin C).
B51A. The method according to any one of paragraphs B30-B51 wherein the serpin is A1PI.
B52. A serpin conjugate prepared by the method according to any one of paragraphs B30-B51 and B51A.
B53. A therapeutic protein conjugate comprising:
   (a) a therapeutic protein or biologically-active fragment thereof comprising at least one cysteine residue, said therapeutic protein comprising only one cysteine residue which comprises an accessible sulfhydryl group that is completely or partially oxidized, said only one cysteine residue is not involved in a disulfide bond with another cysteine residue in the therapeutic protein's amino acid sequence; and
   (b) one water-soluble polymer or functional derivative thereof bound to said sulfhydryl group of the therapeutic protein.
B54. The therapeutic protein conjugate according to paragraph B53 wherein the only one cysteine residue is present in the native amino acid sequence of the therapeutic protein.
B55. The therapeutic protein conjugate according to paragraph B53 wherein the therapeutic protein's amino acid sequence is modified to comprise the only one cysteine residue.
B56. The therapeutic protein conjugate according to claim any one of paragraphs B53-B55 wherein the therapeutic protein is a glycoprotein.
B57. The therapeutic protein conjugate according to paragraph B56 wherein the therapeutic protein is glycosylated in vivo.
B58. The therapeutic protein conjugate according to paragraph B56 wherein the therapeutic protein is glycosylated in vivo.
B59. The therapeutic protein conjugate according to any one of paragraphs B53-B58 wherein the therapeutic protein conjugate has at least a 1.5-fold increase in half-life relative to native therapeutic protein. In one embodiment, the therapeutic protein conjugate has at least between 1 and 10-fold increase in half-life relative to native therapeutic protein.
B60. The therapeutic protein conjugate according to any one of paragraphs B53-B59 wherein the therapeutic protein conjugate retains at least 60% biological activity relative to native therapeutic protein. In one embodiment, the therapeutic protein conjugate retains at least 10 and 100% biological activity relative to native therapeutic protein.
B61. The therapeutic protein conjugate according to any one of paragraphs B53-B60 wherein the water-soluble polymer is selected from the group consisting of linear, branched or multi-arm water soluble polymer.
B62. The therapeutic protein conjugate according to any one of paragraphs B53-B61 wherein the water-soluble polymer has a molecular weight between 3,000 and 150,000 Daltons (Da).
B63. The therapeutic protein conjugate according to any one of paragraphs B53-B62 wherein the water-soluble polymer is selected from the group consisting of polyethylene glycol (PEG), branched PEG, PolyPEG® (Warwick Effect Polymers; Coventry, UK), polysialic acid (PSA), starch, hydroxylethyl starch (HES), hydroxyalkyl starch (HAS), carbohydrate, polysaccharides, pullulane, chitosan, hyaluronic acid, chondroitin sulfate, dermatan sulfate, starch, dextran, carboxymethyl-dextran, polyalkylene oxide (PAO), polyalkylene glycol (PAG), polypropylene glycol (PPG), polyoxazoline, polyacryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC), and functional derivatives thereof.
B64. The therapeutic protein conjugate according to any one of paragraphs B53-B63 wherein the water soluble polymer is derivatized to contain a sulfhydryl-specific group selected from the group consisting of: maleimide (MAL), vinylsulfones, orthopyridyl-disulfides (OPSS) and iodacetamides.
B65. The therapeutic protein conjugate according to paragraph B64 wherein the water soluble polymer is PEG and the sulfhydryl-specific group is MAL.
B66. The therapeutic protein conjugate according to paragraph B64 wherein the water soluble polymer is PSA and the sulfhydryl-specific group is MAL.
B67. The therapeutic protein conjugate according to any one of paragraphs B53-B66 wherein the therapeutic protein is selected from the group consisting of: A1PI alpha-1 proteinase inhibitor (A1PI), antithrombin III, alpha-1-antichymotrypsin, human serum albumin, alcoholdehydrogenase, biliverdin reductase, buturylcholinesterase, complement C5a, cortisol-binding protein, creatine kinase, coagulation factor V (FV), coagulation factor VII (FVII), ferritin, heparin cofactor, interleukin 2, protein C inhibitor, tissue factor and vitronectin.

In one embodiment, the therapeutic protein is selected from the group consisting of: ovalbumin, plasminogen-activator inhibitor, neuroserpin, C1-Inhibitor, nexin, alpha-2-antiplasmin, heparin cofactor II, alpha1-antichymotrypsin, alpha1-microglobulin, coagulation factor VIII (FVIII), and coagulation factor XIII (XIII).

In another embodiment, the therapeutic protein is a protein of the serpin superfamily selected from the group consisting of: A1PI (alpha-1 proteinase inhibitor), or A1AT (alpha-1-antitrypsin), ATR (alpha-1-antitrypsin-related protein), AACT or ACT (alpha-1-antichymotrypsin), PI4 (proteinase inhibitor 4), PCI or PROCI (protein C inhibitor), CBG, (corticosteroid-binding globulin), TBG (thyroxine-binding globulin), AGT (angiotensinogen), centerin, PZI (protein Z-dependent protease inhibitor), PI2 (proteinase inhibitor 2), PAI2 or PLANH2 (plasminogen activator inhibitor-2), SCCA1 (squamous cell carcinoma antigen 1), SCCA2 (squamous cell carcinoma antigen 2), PI5 (proteinase inhibitor 5), PI6 (proteinase inhibitor 6), megsin, PI8 (proteinase inhibitor 8), PI9 (proteinase inhibitor 9), PI10 (proteinase inhibitor 10), epipin, yukopin, PI13 (proteinase inhibitor 13), PI8L1 (proteinase inhibitor 8-like 1), AT3 or ATIII (antithrombin-III), HC-II or HCF2 (heparin cofactor II), PAI1 or PLANH1 (plasminogen activator inhibitor-1), PN1 (proteinase nexin I), PEDF, (pigment epithelium-derived factor), PLI (plasmin inhibitor), C1IN or C1 INH (plasma proteinase C1 inhibitor), CBP1 (collagen-binding protein 1), CBP2 (collagen-binding protein 2), PI12 (proteinase inhibitor 12), and PI14 (proteinase inhibitor 14).

In another embodiment, the therapeutic protein is a blood coagulation factor protein selected from the group consisting of: Factor IX (FIX), Factor VIII (FVIII), Factor VIIa (FVIIa), Von Willebrand Factor (VWF), Factor FV (FV), Factor X (FX), Factor XI (FXI), Factor XII (FXII), thrombin (FII), protein C, protein S, tPA, PAI-1, tissue factor (TF) and ADAMTS 13 protease.
B68. The therapeutic protein conjugate according to paragraph B67 wherein the therapeutic protein is A1PI.
B69. A serrin conjugate comprising:
   (a) a serpin or biologically-active fragment thereof; and
   (b) at least one water-soluble polymer or functional derivative thereof bound to said serpin or biologically0active fragment thereof, said water-soluble polymer or functional derivative thereof selected from the group consisting of polysialic acid (PSA), starch, hydroxylethyl starch (HES), hydroxyalkyl starch (HAS), carbohydrate, polysaccharides, pullulane, chitosan, hyaluronic acid, chondroitin sulfate, dermatan sulfate, dextran, PolyPEG® (Warwick Effect Polymers; Coventry, UK), carboxymethyl-dextran, polyalkylene oxide (PAO), polyalkylene glycol (PAG), polypropylene glycol (PPG), polyoxazoline, polyacryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC), N-hydroxysuccinimide ester-PEG (NHS-PEG), PEG carbonate, PEG aldehydes, aminooxy-PEG, PEG hydrazide (PEG-Hz), PEG hydrazine, PEG thiol (PEG-SH), amino PEG (PEG -NH2), carboxyl PEG (PEG-COOH), Hydroxyl PEG (PEG-OH), PEG epoxide, oxidized PSA, aminooxy-PSA, PSA hydrazide (PSA-Hz), PSA hydrazine, PEG vinylsulfone, PEG orthpyridyl-disulfide (OPSS), PEG ioacetamide, PEG benzotriazole, PSA thiol (PSA-SH), MAL-PSA and amino PSA (PSA-NH2);
   said serpin conjugate retaining at least 60% biological activity relative to native glycosylated serpin.
B70. The serpin conjugate according to paragraph B69 wherein the serpin is a glycoprotein.
B71. The serpin conjugate according to paragraph B70 wherein the serpin is glycosylated in vivo.
B72. The serpin conjugate according to paragraph B70 wherein the serpin is glycosylated in vitro.
B73. The serpin conjugate according to any one of paragraphs B69-B72 wherein the serpin conjugate has at least a 1.5-fold increase in half-life relative to native serpin. In one embodiment, the serpin conjugate has at least a between 1 and 10-fold increase in half-life relative to native serpin.
B74. The serpin conjugate according to any one of paragraphs B69-B73 wherein the water-soluble polymer is selected from the group consisting of linear, branched or multi-arm water soluble polymer.
B75. The serpin conjugate according to any one of paragraphs B69-B74 wherein the water-soluble polymer has a molecular weight between 3,000 and 150,000 Daltons (Da).
B76. The serpin conjugate according to paragraph B75 wherein the water-soluble polymer is linear and has a molecular weight between 10,000 and 50,000 Da. In one embodiment, the water-soluble polymer is linear and has a molecular weight of 20,000 Da.
B77. The serpin conjugate according to any one of paragraphs B69-B76 wherein the water soluble polymer functional derivative is PEG-NHS.
B78. The serpin conjugate according to any one of paragraphs B69-B76 wherein the water soluble polymer functional derivative is aminooxy-PEG and the serpin is glycosylated.
B79. The serpin conjugate according to any one of paragraphs B69-B76 wherein the water soluble polymer functional derivative is aminooxy-PSA and the serpin is glycosylated.
B80. The serpin conjugate according to any one of paragraphs B69-B79 wherein the serpin is selected from the group consisting of:A1PI, antihrombin III, alpha-1-antichymotrypsin, ovalbumin, plasminogen-activator inhibitor, neuroserpin, C1-Inhibitor, nexin, alpha-2-antiplasmin and heparin cofactor II.
B81. A method of treating a disease comprising administering a therapeutic protein conjugate according to any one of claims 53-68 in an amount effective to treat said disease.
B82. A method of treating a disease comprising administering a serpin conjugate according to any one of paragraphs B69-B80 in an amount effective to treat said disease.
B83. A kit comprising a pharmaceutical composition comprising i) a therapeutic protein conjugate according to any one of paragraphs B53-B68; and ii) a pharmaceutically acceptable excipient; packaged in a container with a label that describes use of the pharmaceutical composition in a method of treating a disease.
B84. A kit comprising a pharmaceutical composition comprising i) a serpin conjugate according to any one of paragraphs B69-B80; and ii) a pharmaceutically acceptable excipient; packaged in a container with a label that describes use of the pharmaceutical composition in a method of treating a disease.
B85. The kit according to any one of paragraphs B83 to B84 wherein the pharmaceutical composition is packaged in a unit dose form.

### The present disclosure also provides the following exemplary embodiments:

C1. A method of preparing a therapeutic protein conjugate comprising the steps of:
   contacting a therapeutic protein comprising a single, accessible and oxidizable sulfhydryl group with a thiol reductant under conditions that allow the reduction of the sulfhydryl group, and
   contacting a water-soluble polymer with the therapeutic protein under conditions that allow conjugation of the water-soluble polymer to the reduced sulfhydryl group.
CIA. The method according to paragraph C1 wherein the therapeutic protein is selected from the group consisting of alpha-1 proteinase inhibitor (A1PI), antithrombin III, alpha-1-antichymotrypsin, ovalbumin, plasminogen-activator inhibitor, neuroserpin, C1-Inhibitor, nexin, alpha-2-antiplasmin, heparin cofactor II, alpha1-antichymotrypsin, alpha1-microglobulin, albumin, alcoholdehydrogenase, biliverdin reductase, buturylcholinesterase, complement C5a, cortisol-binding protein, creatine kinase, Factor V (FV), Factor VII (FVII), ferritin, heparin cofactor, interleukin 2, protein C inhibitor, tissue factor and vitronectin or a biologically active fragment, derivative or variant thereof.
C1B. The method according to any one of paragraphs C1-C1A wherein the thiol reductant is selected from the group consisting of Tris[2-carboxyethyl] phosphine hydrochloride (TCEP), dithiothreitol (DTT), DTE, sodium borohydride (NaBH4), and sodium cyanoborohydride (NaCNBH3).
C1C. The method according to any one of paragraphs C1-C1B wherein the water soluble polymer is selected from the group consisting of polyethylene glycol (PEG), branched PEG, PolyPEG® (Warwick Effect Polymers; Coventry, UK), polysialic acid (PSA), starch, hydroxylethyl starch (HES), hydroxyalkyl starch (HAS), carbohydrate, polysaccharides, pullulane, chitosan, hyaluronic acid, chondroitin sulfate, dermatan sulfate, dextran, carboxymethyl-dextran, polyalkylene oxide (PAO), polyalkylene glycol (PAG), polypropylene glycol (PPG), polyoxazoline, polycryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC), and functional derivatives thereof.
C2. The method of paragraph C1B wherein the thiol reductant is TCEP.
C2A. The method according to paragraph C2 wherein the therapeutic protein, water-soluble polymer and thiol reductant are incubated together in a single vessel.
C3. The method according to any one of paragraphs C1-C2 wherein the thiol reductant concentration is between 1-100 molar excess relative to the therapeutic protein concentration.
C4. The method according to any one of paragraphs C1C3 wherein the thiol reductant concentration is a 3-fold molar excess relative to the therapeutic protein concentration.
C5. The method according to any one of paragraphs C1-C4 wherein the water-soluble polymer is selected from the group consisting of a linear, branched or multi-arm water soluble polymer.
C6. The method according to paragraph C5 wherein the water-soluble polymer has a molecular weight between 3,000 and 150,000 Da.
C7. The method according to paragraph C6 wherein the water-soluble polymer is linear and has a molecular weight of 20,000 Da.
C8. The method according to any one of paragraphs C1C7 wherein the water-soluble polymer is PEG.
C9. The method according to any one of paragraphs C1-C7 wherein the water-soluble polymer is PSA.
C10. The method according to any one of paragraphs C1-C9 wherein the water-soluble polymer is derivatized with a sulfhydryl-specific agent selected from the group consisting of maleimide (MAL), vinylsulfones, orthopyridyl-disulfides (OPSS) and iodacetamides.
C11. The method according to paragraph C8 wherein the sulfhydryl-specific agent is MAL.
C12. The method according to paragraph C11 wherein the water-soluble polymer derivative is MAL-PEG.
C13. The method according to paragraph C11 wherein the water-soluble polymer derivative is MAL-PSA.
C14. The method according to any one of paragraphs C1-C13 wherein the therapeutic protein conjugate retains at least 20% biological activity relative to native therapeutic protein.
C15. The method according to paragraph C14 wherein the therapeutic protein conjugate retains at least 60% biological activity relative to native therapeutic protein.
C16. The method according to any one of paragraphs C1-C15 wherein the therapeutic protein conjugate has an increased half-life relative to native therapeutic protein.
C17. The method according to paragraph C16 wherein the therapeutic protein conjugate has at least a 2-fold increase in half-life relative to native therapeutic protein.
C1 8.The method according to any one paragraphs C1-C17 wherein the therapeutic protein conjugate comprises a single water-soluble polymer.
C19. The method according to paragraph C18 wherein at least 20% of the therapeutic protein conjugate comprises a single water-soluble polymer.
C20. The method according to any one paragraphs C1-C19 wherein the single, accessible and oxidizable sulfhydryl group is a sulfhydryl group on a cysteine residue.
C21. The method according to paragraph C20 wherein the cysteine residue is present in the native amino acid sequence of the therapeutic protein.
C22. The method according to paragraph C21 wherein the therapeutic protein is purified from human plasma.
C23. The method according to paragraph C22 wherein the therapeutic protein is naturally-glycosylated A1PI.
C24. The method according to paragraph C21 wherein the therapeutic protein is produced recombinantly in a host cell.
C25. The method according to paragraph C24 wherein the host cell is selected from the group consisting of a yeast cell, a mammalian cell, an insect cell, and a bacterial cell.
C26. The method according to paragraph C25 wherein a naturally-glycosylated therapeutic protein is produced by the mammalian cell.
C27. The method according to paragraph C26 wherein a naturally-glycosylated A1PI is produced by the mammalian cell.
C28. The method according to paragraph C25 wherein the therapeutic protein is produced by the bacterial cell.
C29. The method according to paragraph C28 wherein the therapeutic protein is glycosylated in vitro following purification from the bacterial cell.
C30. The method of paragraph C29 wherein A1PI is glycosylated in vitro following purification from the bacterial cell.
C31. The method according to paragraph C20 wherein the native amino acid sequence of the therapeutic protein is modified to comprise single, accessible and oxidizable sulfhydryl group on the cysteine residue.
C32. The method according to paragraph C31 wherein one or more cysteine residues have been inserted, deleted or substituted in the native amino acid sequence of the therapeutic protein.
C33. The method according to paragraph C32 wherein the therapeutic protein is produced recombinantly in a host cell.
C34. The method according to paragraph C33 wherein the host cell is selected from the group consisting of a yeast cell, a mammalian cell, an insect cell, and a bacterial cell.
C35. The method according to paragraph C34 wherein a naturally-glycosylated therapeutic protein is produced by the mammalian cell.
C36. The method according to paragraph C34 wherein the therapeutic protein is produced by the bacterial cell.
C37. The method according to paragraph C36 wherein the therapeutic protein is glycosylated in vitro following purification from the bacterial cell.
C38. The method according to any one of paragraphs C1-C37 further comprising the step of purifying the therapeutic protein conjugate.
C39. The method according to paragraph C38 wherein the therapeutic protein conjugate is purified using a technique selected from the group consisting of ion-exchange chromatography, hydrophobic interaction chromatography, size exclusion chromatography and affinity chromatography or combinations thereof.
C40. A therapeutic protein conjugate produced by the method according to any one of paragraphs C1-C39.

### The present disclosure also provides the following exemplary embodiments:

D1. A method of preparing a glycosylated serpin conjugate comprising contacting a water-soluble polymer with a glycosylated serpin under conditions that allow conjugation;
   said glycosylated serpin conjugate retaining at least 20% biological activity relative to native glycosylated serpin; and
   said glycosylated serpin conjugate having an increased half-life relative to native glycosylated serpin.
D1A. The method according to paragraph D1 wherein the glycosylated serpin is selected from the group consisting of: A1PI (alpha-1 proteinase inhibitor), or A1AT (alpha-1-antitrypsin), ATR (alpha-1-antitrypsin-related protein), AACT or ACT (alpha-1-antichymotrypsin), PI4 (proteinase inhibitor 4), PCI or PROCI (protein C inhibitor), CBG, (corticosteroid-binding globulin), TBG (thyroxine-binding globulin), AGT (angiotensinogen), centerin, PZI (protein Z-dependent protease inhibitor), PI2 (proteinase inhibitor 2), PAI2 or PLANH2 (plasminogen activator inhibitor-2), SCCA1 (squamous cell carcinoma antigen 1), SCCA2 (squamous cell carcinoma antigen 2), PI5 (proteinase inhibitor 5), PI6 (proteinase inhibitor 6), megsin, PI8 (proteinase inhibitor 8), PI9 (proteinase inhibitor 9), PI10 (proteinase inhibitor 10), epipin, yukopin, PI13 (proteinase inhibitor 13), PI8L1 (proteinase inhibitor 8-like 1), AT3 or ATIII (antithrombin-III), HC-II or HCF2 (heparin cofactor II), PAI1 or PLANH1 (plasminogen activator inhibitor-1), PN1 (proteinase nexin I), PEDF, (pigment epithelium-derived factor), PLI (plasmin inhibitor), C1IN or C1 INH (plasma proteinase C1 inhibitor), CBP1 (collagen-binding protein 1), CBP2 (collagen-binding protein 2), PI12 (proteinase inhibitor 12), and PI14 (proteinase inhibitor 14).
D1B. The method according to any one of paragraphs D1-D1A wherein the water-soluble polymer is selected from the group consisting of polyethylene glycol (PEG), branched PEG, PolyPEG® (Warwick Effect Polymers; Coventry, UK), polysialic acid (PSA), starch, hydroxylethyl starch (HES), hydroxyalkyl starch (HAS), carbohydrate, polysaccharides, pullulane, chitosan, hyaluronic acid, chondroitin sulfate, dermatan sulfate, dextran, carboxymethyl-dextran, polyalkylene oxide (PAO), polyalkylene glycol (PAG), polypropylene glycol (PPG), polyoxazoline, polyacryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC), and functional derivatives thereof.
D1C. The method according to paragraph D1B wherein the water soluble polymer derivative is selected from the group consisting of N-hydroxysuccinimide ester-PEG (NHS-PEG), PEG carbonate, PEG aldehydes, aminooxy-PEG, PEG hydrazide (PEG-Hz), PEG hydrazine, PEG maleimide (MAL-PEG), PEG thiol (PEG-SH), Amino PEG (PEG - NH2), Carboxyl PEG (PEG-COOH), Hydroxyl PEG (PEG-OH), PEG epoxide, oxidized PSA, aminooxy-PSA, PSA hydrazide, PSA hydrazine, PEG vinylsulfone, PEG orthpyridyl-disulfide (OPSS), PEG ioacetamide, PEG benzotriazole, PSA-SH, MAL-PSA. and PSA-NH2.
D1D. The method according to any one of paragraphs D1-D2A wherein the water soluble polymer or functional derivative thereof has a molecular weight between 3,000 and 150,000 Da.
D2. The method according to paragraph D1 wherein the glycosylated serpin conjugate retains at least 60% biological activity relative to native glycosylated serpin.
D3. The method according to any one of paragraphs D1-D2 wherein the glycosylated serpin conjugate has at least a 2-fold increase in half-life relative to native glycosylated serpin.
D4. The method according to any one paragraphs D1-D3 wherein the glycosylated serpin conjugate comprises a single water-soluble polymer.
D5. The method according to paragraph D4 wherein at least 20% of the glycosylated serpin conjugate comprises a single water-soluble polymer.
D6. The method according to any one of paragraphs D1-D5 wherein the glycosylated serpin comprises a single, accessible and oxidizable sulfhydryl group.
D7. The method according to paragraph D6 wherein the single, accessible and oxidizable sulfhydryl group is a sulfhydryl group on a cysteine residue.
D8. The method according to paragraph D7 further comprising contacting the glycosylated serpin with a single, accessible and oxidizable sulfhydryl group with a thiol reductant under conditions that allow the reduction of the sulfhydryl group.
D9. The method according to paragraph D8 wherein the thiol reductant is selected from the group consisting of Tris[2-carboxyethyl] phosphine hydrochloride (TCEP), dithiothreitol (DTT), DTE, sodium borohydride (NaBH4), and sodium cyanoborohydride (NaCNBH3).
D10. The method according to paragraph D9 wherein the thiol reductant is TCEP.
D11. The method according to any one of paragraphs D8-D10 wherein the therapeutic protein, water-soluble polymer and thiol reductant are incubated together in a single vessel.
D12. The method according to any one of paragraphs D8-D11 wherein the thiol reductant concentration is between 1-100-fold molar excess relative to the glycosylated serpin concentration.
D13. The method according to any one of paragraph D8-D12 wherein the thiol reductant concentration is a 3-fold molar excess relative to the glycosylated serpin concentration.
D14. The method according to any one of paragraphs D8-D13 wherein the cysteine residue is present in the native amino acid sequence of the glycosylated serpin.
D15. The method according to paragraph D14 wherein the glycosylated serpin is purified from human plasma.
D16. The method according to paragraph D15 wherein the glycosylated serpin is A1PI.
D17. The method according to paragraph D14 wherein the serpin is produced recombinantly in a host cell.
D18. The method according to paragraph D17 wherein the host cell is selected from the group consisting of a yeast cell, a mammalian cell, an insect cell, and a bacterial cell.
D19. The method according to paragraph D18 wherein a naturally-glycosylated serpin is produced by the mammalian cell.
D20. The method according to paragraph D19 wherein the naturally-glycosylated serpin is A1PI.
D21. The method according to paragraph D18 wherein the serpin is produced by a bacterial cell.
D22. The method according to paragraph D21 wherein the serpin is glycosylated in vitro following purification from the bacterial cell.
D23. The method of paragraph D22 wherein the serpin is A1PI.
D24. The method according to any one of paragraphs D6-D23 wherein the water-soluble polymer is derivatized with a sulfhydryl-specific agent selected from the group consisting of maleimide (MAL), vinylsulfones, orthopyridyl-disulfides (OPSS) and iodacetamides.
D25. The method according to paragraph D24 wherein the sulfhydryl-specific agent is MAL.
D26. The method according to paragraph D25 wherein the water-soluble polymer derivative is MAL-PEG.
D27. The method according to paragraph D25 wherein the water-soluble polymer derivative is MAL-PSA.
D28. The method according to paragraph D24 wherein the water-soluble polymer derivative is selected from the group of linear, branched and multi-arm water soluble polymer derivative.
D29. A method of preparing a glycosylated A1PI conjugate comprising:
   contacting a glycosylated A1PI protein comprising a single, accessible and oxidizable sulfhydryl group with a solution comprising TCEP under conditions that allow the reduction of the sulfhydryl group, and
   contacting a water-soluble polymer or functional derivative thereof to the glycosylated A1PI under conditions that allow conjugation;
   said glycosylated A1PI conjugate retaining at least 20% biological activity relative to native glycosylated A1PI;
   and said glycosylated A1PI conjugate having an increased half-life relative to native glycosylated A1PI; and
   wherein at least 70% of the A1PI is mono-PEGylated.
D30. The method according to paragraph D29 wherein the water-soluble polymer derivative is MAL-PEG.
D31. The method according to paragraph D29 wherein the water-soluble polymer derivative is MAL-PSA.
D32. The method according to any one of paragraphs D29-D31 wherein the therapeutic protein, water-soluble polymer and thiol reductant are incubated together in a single vessel.
D33. The method according to any one of paragraphs D1-D5 wherein the water-soluble polymer is derivatized with a lysine-specific agent selected from the group consisting of N-hydroxysuccinimide ester (NHS).
D34. The method according to paragraph D33 wherein the water-soluble polymer derivative is PEG-NHS.
D35. The method according to paragraph D33 wherein the water-soluble polymer derivative is PSA-NHS.
D36. The method according to any one of paragraphs D1-D5 wherein the water-soluble polymer is derivatized with an aminooxy linker.
D37. The method according to paragraph D36 wherein the water-soluble polymer derivative is aminooxy-PEG.
D38. The method according to paragraph D36 wherein the water-soluble polymer derivative is aminooxy-PSA.
D39. The method according to any one of paragraphs D36-D38 wherein a carbohydrate moiety of the glycosylated serpin is oxidized by incubation with a buffer comprising an oxidizing agent selected from the group consisting of sodium periodate (NaIO4), lead tetraacetate (Pb(OAc)4) and potassium perruthenate (KRuO4) prior to contacting with the water-soluble polymer or functional derivative thereof;
   wherein an oxime linkage is formed between the oxidized carbohydrate moiety and an active aminooxy group on the aminooxy linker-derivatized water-soluble polymer, thereby forming the glycosylated serpin conjugate.
D40. The method according to paragraph D39 wherein the oxidizing agent is sodium periodate (NaIO4).
D41. The method according to any one of paragraphs D36-D40 wherein the aminooxy linker-derivatized water-soluble polymer is aminooxy-PEG.
D42. The method according to any one of paragraphs D36-D40 wherein the aminooxy linker-derivatized water-soluble polymer is aminooxy-PSA.
D43. The method according to paragraph D42 wherein the aminooxy-PSA is prepared by reacting an activated aminooxy linker with oxidized PSA;
   wherein the aminooxy linker is selected from the group consisting of:
   a) a 3-oxa-pentane-1,5-dioxyamine linker of the formula: 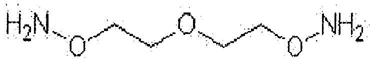
   b) a 3,6,9-trioxa-undecane-1,11-dioxyamine linker of the formula: 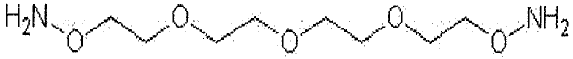 and
   c) a 3,6,9,12,15-penatoxa-heptadecane-1,17-dioxyamine linker of the formula: 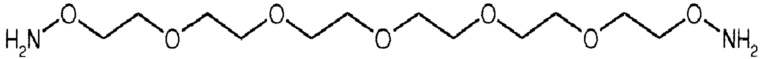 wherein the PSA is oxidized by incubation with a oxidizing agent to form a terminal aldehyde group at the non-reducing end of the PSA.
D44. The method according to any one of paragraphs D39-D43 wherein contacting the oxidized carbohydrate moiety with the activated water soluble polymer occurs in a buffer comprising a nucleophilic catalyst selected from the group consisting of aniline, o-amino benzoic acid, m-amino benzoic acid, p-amino benzoic acid, sulfanilic acid, o-aminobenzamide, o-toluidine, m-toluidine, p-toluidine, o-anisidine, m-anisidine, p-anisidine and derivatives thereof.
D45. The method according to any one of paragraphs D39-D44 further comprising the step of reducing the oxime linkage by incubating the therapeutic protein in a buffer comprising a reducing compound selected from the group consisting of sodium cyanoborohydride (NaCNBH3) and ascorbic acid (vitamin C).
D46. A glycosylated serpin conjugate produced by the method according to any one of paragraphs D1-D45.
D47. A glycosylated serpin conjugate comprising:
   a) a glycosylated serpin protein; and
   b) at least one water-soluble polymer bound to said glycosylated serpin protein of (a), thereby forming a glycosylated serpin conjugate;
      said glycosylated serpin conjugate retaining at least 60% biological activity relative to native glycosylated serpin; and
      said glycosylated serpin conjugate having an increased half-life relative to native glycosylated serpin.
D48. The glycosylated serpin conjugate of paragraph D47 wherein wherein the glycosylated serpin is selected from the group consisting of: A1PI (alpha-1 proteinase inhibitor), or A1AT (alpha-1-antitrypsin), ATR (alpha-1-antitrypsin-related protein), AACT or ACT (alpha-1-antichymotrypsin), PI4 (proteinase inhibitor 4), PCI or PROCI (protein C inhibitor), CBG, (corticosteroid-binding globulin), TBG (thyroxine-binding globulin), AGT (angiotensinogen), centerin, PZI (protein Z-dependent protease inhibitor), PI2 (proteinase inhibitor 2), PAI2 or PLANH2 (plasminogen activator inhibitor-2), SCCA1 (squamous cell carcinoma antigen 1), SCCA2 (squamous cell carcinoma antigen 2), PI5 (proteinase inhibitor 5), PI6 (proteinase inhibitor 6), megsin, PI8 (proteinase inhibitor 8), PI9 (proteinase inhibitor 9), PI10 (proteinase inhibitor 10), epipin, yukopin, PI13 (proteinase inhibitor 13), PI8L1 (proteinase inhibitor 8-like 1), AT3 or ATIII (antithrombin-III), HC-II or HCF2 (heparin cofactor II), PAI1 or PLANH1 (plasminogen activator inhibitor-1), PN1 (proteinase nexin I), PEDF, (pigment epithelium-derived factor), PLI (plasmin inhibitor), C1IN or C1 INH (plasma proteinase C1 inhibitor), CBP1 (collagen-binding protein 1), CBP2 (collagen-binding protein 2), PI12 (proteinase inhibitor 12), and PI14 (proteinase inhibitor 14).
D49. The glycosylated serpin conjugate according to any one of paragraphs D48-D49 wherein the water-soluble polymer is selected from the group consisting of polyethylene glycol (PEG), branched PEG, PolyPEG® (Warwick Effect Polymers; Coventry, UK), polysialic acid (PSA), starch, hydroxylethyl starch (HES), hydroxyalkyl starch (HAS), carbohydrate, polysaccharides, pullulane, chitosan, hyaluronic acid, chondroitin sulfate, dermatan sulfate, dextran, carboxymethyl-dextran, polyalkylene oxide (PAO), polyalkylene glycol (PAG), polypropylene glycol (PPG), polyoxazoline, polyacryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC), and functional derivative thereof.
D50. The glycosylated serpin conjugate of paragraph D49 the water soluble polymer derivative is selected from the group consisting of N-hydroxysuccinimide ester-PEG (NHS-PEG), PEG carbonate, PEG aldehydes, aminooxy-PEG, PEG hydrazide (PEG-Hz), PEG hydrazine, PEG maleimide (MAL-PEG), PEG thiol (PEG-SH), Amino PEG (PEG - NH2), Carboxyl PEG (PEG-COOH), Hydroxyl PEG (PEG-OH), PEG epoxide, oxidized PSA, aminooxy-PSA, PSA hydrazide, PSA hydrazine, PEG vinylsulfone, PEG orthpyridyl-disulfide (OPSS), PEG ioacetamide, PEG benzotriazole, PSA-SH, MAL-PSA. and PSA-NH2.
D51. The glycosylated serpin conjugate according to any one of paragraphs D48-D50 wherein the water soluble polymer or functional derivative thereof has a molecular weight between 3,000 and 150,000 Da.
D52. The glycosylated serpin conjugate according to any one of paragraphs D48-D51 wherein the water soluble polymer or functional derivative thereof is selected from the group of linear, branched and multi-arm water soluble polymer or functional derivative thereof.
D53. The glycosylated serpin conjugate of paragraph D49 wherein the water-soluble polymer is derivatized with a sulfhydryl-specific agent selected from the group consisting of maleimide (MAL), vinylsulfones, orthopyridyl-disulfides (OPSS) and iodacetamides.
D54. The glycosylated serpin conjugate of paragraph D53 wherein the sulfhydryl-specific agent is MAL.
D55. The glycosylated serpin conjugate of paragraph D54 wherein the water-soluble polymer derivative is MAL-PEG.
D56. The glycosylated serpin conjugate of paragraph D54 wherein the water-soluble polymer derivative is MAL-PSA.
D57. The glycosylated serpin conjugate of paragraph D49 wherein the water-soluble polymer is derivatized with a lysine-specific agent selected from the group consisting of N-hydroxysuccinimide ester (NHS).
D58. The glycosylated serpin conjugate of paragraph D57 wherein the lysine-specific agent is NHS.
D59. The glycosylated serpin conjugate of paragraph D57 wherein the water-soluble polymer derivative is PEG-NHS.
D60. The glycosylated serpin conjugate of paragraph D57 wherein the water-soluble polymer derivative is PSA-NHS.
D61. The glycosylated serpin conjugate of paragraph D49 wherein the water-soluble polymer is derivatized with an aminooxy linker.
D62. The glycosylated serpin conjugate of paragraph D61 wherein the water-soluble polymer derivative is aminooxy-PEG.
D63. The glycosylated serpin conjugate of paragraph D61 wherein the water-soluble polymer derivative is aminooxy-PSA.
D64. The glycosylated serpin conjugate according to any one of paragraphs D61-D63 wherein the aminooxy linker is selected from the group consisting of:
   a) a 3-oxa-pentane-1,5-dioxyamine linker of the formula: 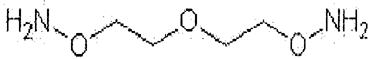
   b) a 3,6,9-trioxa-undecane-1,11-dioxyamine linker of the formula: 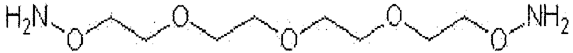 and
   c) a 3,6,9,12,15-penatoxa-heptadecane-1,17-dioxyamine linker of the formula: 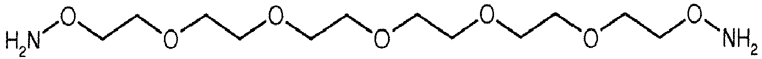
D65. The glycosylated serpin conjugate according to any one of paragraphs D46-D65 wherein the glycosylated serpin is A1PI.
D66. A method of treating a disease associated with a serpin comprising administering a glycosylated serpin conjugate according to any one of paragraphs D46-D65 in an amount effective to treat said disease.
D67. The method according to paragraph D66 wherein the glycosylated serpin is A1PI.
D68. The method according to D67 wherein the water soluble polymer is MAL-PEG.
D69. The method according to any one of paragraphs D67-D68 wherein the disease is emphysema.
D70. A kit comprising a pharmaceutical composition comprising i) a glycosylated serpin conjugate according to any one of paragraphs D46-D65; and ii) a pharmaceutically acceptable excipient; packaged in a container with a label that describes use of the pharmaceutical composition in a method of treating a disease associated with the serpin.
D71. The kit according to paragraph D70 wherein the pharmaceutical composition is packaged in a unit dose form.
D72. A kit comprising a first container comprising a glycosylated serpin conjugate according to one of paragraphs D46-D65, and a second container comprising a physiologically acceptable reconstitution solution for said composition in the first container, wherein said kit is packaged with a label that describes use of the pharmaceutical composition in a method of treating a disease associated with the serpin.
D73. The kit according to paragraph D72 wherein the pharmaceutical composition is packaged in a unit dose form.

### The present disclosure also provides the following exemplary embodiments:

E1. A method of preparing a glycosylated therapeutic protein conjugate comprising contacting a water soluble polymer to a glycosylated therapeutic protein under conditions that allow conjugation, said glycosylated therapeutic protein conjugate retaining at least 20% biological activity relative to native glycosylated therapeutic protein, and said glycosylated therapeutic protein conjugate having an increased half-life relative to native glycosylated therapeutic protein.
E2. The method of paragraph E1 wherein the glycosylated therapeutic protein conjugate retains at least 30% biological activity relative to native glycosylated therapeutic protein.
E2A. The method of paragraph E1 wherein the glycosylated therapeutic protein is glycosylated in vivo prior to purification.
E3. The method of paragraph E1 wherein the glycosylated therapeutic protein is glycosylated in vitro following purification.
E4. The method of any one of paragraphs E1-E3 wherein the water soluble polymer is selected from the group consisting of polyethylene glycol (PEG), branched PEG, PolyPEG® (Warwick Effect Polymers; Coventry, UK), polysialic acid (PSA), starch, hydroxylethyl starch (HES), hydroxyalkyl starch (HAS), carbohydrate, polysaccharides, pullulane, chitosan, hyaluronic acid, chondroitin sulfate, dermatan sulfate, dextran, carboxymethyl-dextran, polyalkylene oxide (PAO), polyalkylene glycol (PAG), polypropylene glycol (PPG), polyoxazoline, polyacryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC), and functional derivatives thereof.
E5. The method of paragraph E1 wherein the conjugation occurs in a simultaneous reaction.
E6. The method of any one of paragraphs E1-E5 wherein the glycosylated therapeutic protein is selected from the group consisting of plasma-derived alpha-1 proteinase inhibitor (A1PI), recombinant A1PI, Antithrombin III, Alpha-1-antichymotrypsin, Ovalbumin, Plasminogen-activator inhibitor, Neuroserpin, C1-Inhibitor, nexin, alpha-2-antiplasmin, Heparin cofactor II, alpha1-antichymotrypsin, alpha1-microglobulin, albumin, alcoholdehydrogenase, biliverdin reductase, buturylcholinesterase, complement C5a, cortisol-binding protein, creatine kinase, factor V, factor VII, ferritin, heparin cofactor, interleukin 2, protein C inhibitor, tissue factor and vitronectin or a biologically active fragment, derivative or variant thereof.
E7. The method of any one of paragraphs E1-E6 wherein the glycosylated therapeutic protein conjugate retains at least 40 % biological activity relative to native glycosylated therapeutic protein.
E8. The method of paragraph E7 wherein the glycosylated therapeutic protein conjugate retains at least 50 % biological activity relative to native glycosylated therapeutic protein.
E9. The method of paragraph E8 wherein the glycosylated therapeutic protein conjugate retains at least 60 % biological activity relative to native glycosylated therapeutic protein.
E10. The method of paragraph E8 wherein the glycosylated therapeutic protein conjugate retains at least 70 % biological activity relative to native glycosylated therapeutic protein.
E11. The method of paragraph E8 wherein the glycosylated therapeutic protein conjugate retains at least 80 % biological activity relative to native glycosylated therapeutic protein.
E12. The method of paragraph E8 wherein the glycosylated therapeutic protein conjugate retains at least 90 % biological activity relative to native glycosylated therapeutic protein.
E12A. The method of any one of paragraphs E1-E12 wherein the half-life of the glycosylated therapeutic protein conjugate is at least 2 times higher than the native glycosylated therapeutic protein.
E12B. The method of paragraph E12A wherein the half-life of the glycosylated therapeutic protein conjugate is 8 times higher than the native glycosylated therapeutic protein.
E12C. The method of any one of paragraphs E1-E12B wherein the water soluble polymer is selected from the group of linear, branched or multi-arm water soluble polymer.
E12D. The method of any one of paragraphs E1-E12B wherein the water soluble polymer is PEG.
E12E. The method of paragraph E12D wherein the PEG is selected from the group consisting of N-hydroxysuccinimide ester-PEG (NHS-PEG), PEG carbonate, PEG aldehydes, aminooxy-PEG, PEG hydrazide (PEG-Hz), PEG hydrazine, PEG maleimide (MAL-PEG), PEG thiol (PEG-SH), Amino PEG (PEG -NH2), carboxyl PEG (PEG-COOH), Hydroxyl PEG (PEG-OH) and PEG epoxide.
E13. The method according to paragraph E12E wherein the PEG is between 3,000 and 80,000 Da.
E13A. The method of paragraph E4 wherein the water soluble polymer is PSA.
E14. The method of paragraph E13A wherein the PSA is selected from the group consisting of oxidized PSA, aminooxy-PSA, PSA hydrazide, PSA-SH, MAL-PSA. and PSA-NH2.
E15. The method of paragraph E14 wherein the PSA is between 3,000 and 80,000 Da.
E15A. The method of any one of paragraphs E1-E15 wherein the glycosylated therapeutic protein is purified from human plasma.
E16. The method of any one of paragraphs E1-E15 wherein the glycosylated therapeutic protein is produced recombinantly in a host cell.
E17. The method of paragraph E16 wherein the host cell is an animal cell.
E18. The method of paragraph E17 wherein the animal cell is a mammalian cell.
E19. The method of paragraph E18 wherein the mammalian cell is selected from the group consisting of CHO, COS, HEK 293, BHK, SK-Hep, and HepG2.
E20. The method of paragraph E3 wherein the glycosylated therapeutic protein is produced recombinantly in a bacterial cell.
E21. The method of paragraph E20 wherein the bacterial cell is selected from the group consisting of E. coli.
E22. The method of paragraph E12 wherein the water soluble polymer is NHS-PEG.
E23. The method of paragraph E12 wherein the water soluble polymer is aminooxy-PEG.
E24. The method of paragraph E23 wherein a carbohydrate moiety of the therapeutic protein is oxidized by incubation with a buffer comprising an oxidizing agent selected from the group consisting of sodium periodate (NaIO4), lead tetraacetate (Pb(OAc)4 ) and potassium perruthenate (KRuO4) prior to conjugation; and wherein an oxime linkage is formed between the oxidized carbohydrate moiety and an active aminooxy group on the aminooxy-PEG thereby forming the therapeutic protein conjugate.
E25. The method of paragraph E24 which is a simultaneous reaction.
E26. The method according to any one of paragraphs E24 or E25 wherein the oxidizing agent is sodium periodate (NaIO4).
E27. The method according to paragraph E14 wherein the water soluble polymer is aminooxy-PSA.
E28. The method of paragraph E27 wherein a carbohydrate moiety of the therapeutic protein is oxidized by incubation with a buffer comprising an oxidizing agent selected from the group consisting of sodium periodate (NaIO4), lead tetraacetate (Pb(OAc)4 ) and potassium perruthenate (KRuO4) prior to conjugation; and wherein an oxime linkage is formed between the oxidized carbohydrate moiety and an active aminooxy group on the aminooxy-PSA thereby forming the therapeutic protein conjugate.
E29. The method of paragraph E28 which is a simultaneous reaction.
E30. The method according to any one of paragraphs E28 or E29 wherein the oxidizing agent is sodium periodate (NaIO4).
E30A. The method according to paragraph E27 wherein the aminooxy-PSA is prepared by reacting an activated aminooxy linker with oxidized PSA;
   wherein the aminooxy linker is selected from the group consisting of:
   a) a 3-oxa-pentane-1,5-dioxyamine linker of the formula: 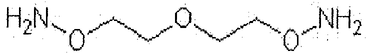
   b) a 3,6,9-trioxa-undecane-1,11-dioxyamine linker of the formula: 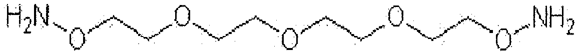 and
   c) a 3,6,9,12,15-penatoxa-heptadecane-1,17-dioxyamine linker of the formula: 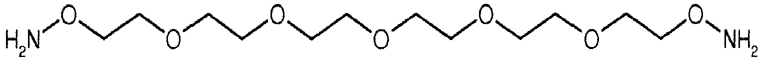 wherein the PSA is oxidized by incubation with a oxidizing agent to form a terminal aldehyde group at the non-reducing end of the PSA.
E30B. The method according to paragraph E30A wherein the aminooxy linker is 3-oxa-pentane-1,5-dioxyamine.
E30C. The method according to paragraph E28 wherein the oxidizing agent is NaIO4.
E31. The method according to any one of paragraph E24-E26 or E28-E30C wherein the contacting of the oxidized carbohydrate moiety with the activated water soluble polymer occurs in a buffer comprising a nucleophilic catalyst selected from the group consisting of aniline and aniline derivatives.
E32. The method according to any one of paragraphs E24-E26 or E28-E31 further comprising the step of reducing the oxime linkage by incubating the therapeutic protein in a buffer comprising a reducing compound selected from the group consisting of sodium cyanoborohydride (NaCNBH3) and ascorbic acid (vitamin C).
E33. The method according to paragraph E32 wherein the reducing compound is sodium cyanoborohydride (NaCNBH3).
E34. The method according to paragraph E12 wherein the water soluble polymer is MAL-PEG.
E35. The method according to vE34 wherein a sulfhydryl (-SH) moiety of the glycosylated therapeutic protein is reduced by incubation with a buffer comprising a reducing agent selected from the group consisting of Tris[2-carboxyethyl] phosphine hydrochloride (TCEP), DTT, DTE, NaBH4, and NaCNBH3.
E36. The method according to any one of paragraph E35 or E36 wherein the reducing agent is TCEP.
E37. The method of paragraph E36 which is a simultaneous reaction.
E38. The method according to any one of paragraphs E36 or E37 wherein the TCEP concentration is between 1-100-fold molar excess relative to the therapeutic protein concentration.
E38. The method according to paragraph E37 wherein the glycosylated therapeutic protein is A1PI.
E39. The method according to paragraph E38 wherein the glycosylated A1PI conjugate is mono-PEGylated.
E40. The method according to paragraph E39 wherein at least 20, 30, 40 50, 60, 70, 80, or 90% of the A1PI is mono-PEGylated.
E41. A method of preparing a glycosylated A1PI conjugate comprising contacting a MAL-PEG to the glycosylated A1PI under conditions that allow conjugation, said glycosylated A1PI conjugate retaining at least 20% biological activity relative to native glycosylated A1PI, and said glycosylated A1PI conjugate having an increased half-life relative to native glycosylated A1PI, wherein the sulfhydryl (-SH) moiety at cysteine 232 of the glycosylated A1PI is reduced by incubation with TCEP , and wherein at least 20% of the A1PI is mono-PEGylated.
E42. The method according to paragraph E41 wherein at least 30, 40, 50, 60, 70, 80 or 90% of the A1PI is mono-PEGylated.
E43. The method according to any one of paragraphs E1-E42 wherein the therapeutic protein conjugate is purified following conjugation.

### The present disclosure also provides the following exemplary embodiments:

F1. A glycosylated serpin conjugate produced by the method according to any one of claims E1-E46.
F2. A glycosylated serpin conjugate comprising:
   (a) a glycosylated serpin; and
   (b) at least one water soluble polymer bound to the glycosylated serpin of (a), said glycosylated serpin conjugate retaining at least 20% biological activity relative to native glycosylated serpin, and said glycosylated serpin conjugate having an increased half-life relative to native glycosylated serpin.
F2A. The glycosylated serpin conjugate of paragraph F1 that is glycosylated in vivo prior to purification.
F3. The glycosylated serpin conjugate of paragraph F1 that is glycosylated in vitro following purification.
F4. The glycosylated serpin conjugate of paragraph F2 wherein the glycosylated serpin conjugate serpin is selected from the group consisting of: A1PI (alpha-1 proteinase inhibitor), or A1AT (alpha-1-antitrypsin), ATR (alpha-1-antitrypsin-related protein), AACT or ACT (alpha-1-antichymotrypsin), PI4 (proteinase inhibitor 4), PCI or PROCI (protein C inhibitor), CBG, (corticosteroid-binding globulin), TBG (thyroxine-binding globulin), AGT (angiotensinogen), centerin, PZI (protein Z-dependent protease inhibitor), PI2 (proteinase inhibitor 2), PAI2 or PLANH2 (plasminogen activator inhibitor-2), SCCA1 (squamous cell carcinoma antigen 1), SCCA2 (squamous cell carcinoma antigen 2), PI5 (proteinase inhibitor 5), PI6 (proteinase inhibitor 6), megsin, PI8 (proteinase inhibitor 8), P19 (proteinase inhibitor 9), PI10 (proteinase inhibitor 10), epipin, yukopin, PI13 (proteinase inhibitor 13), PI8L1 (proteinase inhibitor 8-like 1), AT3 or ATIII (antithrombin-III), HC-II or HCF2 (heparin cofactor II), PAI1 or PLANH1 (plasminogen activator inhibitor-1), PN1 (proteinase nexin I), PEDF, (pigment epithelium-derived factor), PLI (plasmin inhibitor), C1IN or C1 INH (plasma proteinase C1 inhibitor), CBP1 (collagen-binding protein 1), CBP2 (collagen-binding protein 2), PI12 (proteinase inhibitor 12), and PI14 (proteinase inhibitor 14) or a biologically active fragment, derivative or variant thereof.
F5. The glycosylated serpin conjugate of paragraph F2 wherein the water soluble polymer is selected from the group consisting of polyethylene glycol (PEG), branched PEG, PolyPEG® (Warwick Effect Polymers; Coventry, UK),polysialic acid (PSA), starch, hydroxylethyl starch (HES), hydroxyalkyl starch (HAS), hydroxylethyl starch (HES), carbohydrate, polysaccharides, pullulane, chitosan, hyaluronic acid, chondroitin sulfate, dermatan sulfate, dextran, carboxymethyl-dextran, polyalkylene oxide (PAO), polyalkylene glycol (PAG), polypropylene glycol (PPG), polyoxazoline, polyacryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC), and functional derivatives thereof.
F8. The glycosylated serpin conjugate of paragraph F5 wherein the water soluble polymer is approximately 20 kDa.
F9. The glycosylated serpin conjugate of paragraph F2 wherein the serpin conjugate retains at least 40 % biological activity relative to native glycosylated serpin.
F10. The glycosylated serpin conjugate of paragraph F9 wherein the glycosylated serpin conjugate retains at least 50 % biological activity relative to native glycosylated serpin.
F11. The glycosylated serpin conjugate of paragraph F2 wherein the half-life of the serpin conjugate is at least 1-100 times higher than the native glycosylated serpin.
F12. The serpin conjugate of paragraph F11 wherein the half-life of the serpin conjugate is 3 times higher than the native glycosylated serpin.
F13. The glycosylated serpin conjugate of paragraph F5 wherein the water soluble polymer is PEG.
F14. The glycosylated serpin conjugate of paragraph F13 wherein the PEG is selected from the group consisting of N-hydroxysuccinimide ester-PEG (NHS-PEG), aminooxy-PEG, maleimide-PEG (MAL-PEG), PEG carbonate, PEG aldehydes, aminooxy-PEG, PEG hydrazide (PEG-Hz), PEG hydrazine, PEG thiol (PEG-SH), Amino PEG (PEG - NH2), Carboxyl PEG (PEG-COOH), Hydroxyl PEG (PEG-OH) and PEG epoxide.
F15. The glycosylated serpin conjugate of paragraph F5 wherein the water soluble polymer is PSA.
F16. The serpin conjugate of paragraph F15 wherein the PSA is selected from the group consisting of aminooxy-PSA.
F17. The serpin conjugate of paragraph F2 wherein the serpin is purified from human plasma.
F18. The glycosylated serpin conjugate of paragraph F2 wherein the glycosylated serpin is produced recombinantly in a host cell.
F19. The glycosylated serpin conjugate of paragraph 18 wherein the host cell is an animal cell.
F20. The glycosylated serpin conjugate of paragraph F19 wherein the animal cell is a mammalian cell.
F24. The glycosylated serpin conjugate of paragraph F14 wherein the water soluble polymer is NHS-PEG.
F25. The glycosylated serpin conjugate of paragraph F14 wherein the water soluble polymer is aminooxy-PEG.
F26. The glycosylated serpin conjugate of paragraph F25 wherein the aminooxy-PEG is attached to the glycosylated serpin via an oxidized carbohydrate moiety on the glycosylated serpin.
F27. The glycosylated serpin conjugate of paragraph F16 wherein the water soluble polymer is aminooxy-PSA.
F28. The glycosylated serpin conjugate of paragraph F27 wherein the aminooxy-PSA is prepared by reacting an activated aminooxy linker with oxidized PSA;
   wherein the aminooxy linker is selected from the group consisting of:
   a) a 3-oxa-pentane-1,5-dioxyamine linker of the formula: 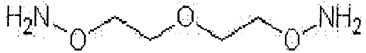
   b) a 3,6,9-trioxa-undecane-1,11-dioxyamine linker of the formula: 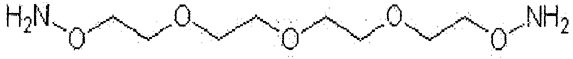 and
   c) a 3,6,9,12,15-penatoxa-heptadecane-1,17-dioxyamine linker of the formula: 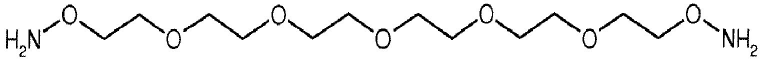 wherein the PSA is oxidized by incubation with a oxidizing agent to form a terminal aldehyde group at the non-reducing end of the PSA.
F29. The glycosylated serpin conjugate of paragraph F14 wherein the water soluble polymer is MAL-PEG.
F30. The glycosylated serpin conjugate of paragraph F29 wherein the MAL-PEG is attached to the serpin via a reduced sulfhydryl (-SH) moiety on the glycosylated serpin.
F31. The glycosylated serpin conjugate of paragraph F27 wherein the glycosylated serpin is A1PI.
F32. The glycosylated serpin conjugate of paragraph F31 wherein the A1PI conjugate is mono-PEGylated.
F33. The glycosylated serpin conjugate of paragraph F32 wherein at least 50% of the A1PI is mono-PEGylated.
F34. The glycosylated serpin conjugate of paragraph F32 wherein at least 60% of the A1PI is mono-PEGylated.
F35. The glycosylated serpin conjugate of paragraph F32 wherein at least 70% of the A1PI is mono-PEGylated.

### The present disclosure also provides the following exemplary embodiments:

G1. A method of treating a disease associated with a serpin comprising administering a glycosylated serpin conjugate according to any of the above claims in an amount effective to treat said disease.
G2. The method according to paragraph G1 wherein the serpin is A1PI.
G3. The method according to paragraph G2 wherein the water soluble polymer is MAL-PEG.
G4. The method according to paragraph G3 wherein the disease is emphysema.

### The present disclosure also provides the following exemplary embodiments:

H1. A kit comprising a pharmaceutical composition comprising i) a glycosylated serpin conjugate according to any of the above claims; and ii) a pharmaceutically acceptable excipient; packaged in a container with a label that describes use of the pharmaceutical composition in a method of treating a disease associated with the serpin.
H2. The kit according to paragraph H1 wherein the pharmaceutical composition is packaged in a unit dose form.
H3. A kit comprising a first container comprising a glycosylated serpin conjugate according to any of the above claims, and a second container comprising a physiologically acceptable reconstitution solution for said composition in the first container, wherein said kit is packaged with a label that describes use of the pharmaceutical composition in a method of treating a disease associated with the serpin.
H4. The kit according to paragraph H3 wherein the pharmaceutical composition is packaged in a unit dose form.

The following examples are not intended to be limiting but only exemplary of specific embodiments of the present disclosure.

### EXAMPLES

### Example 1

### Preparation of the homobifunctional linker NH₂(OCH₂CH₂]₂ONH₂

The homobifunctional linker NH₂[OCH₂CH₂]₂ONH₂

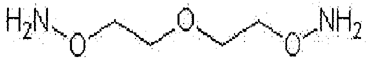

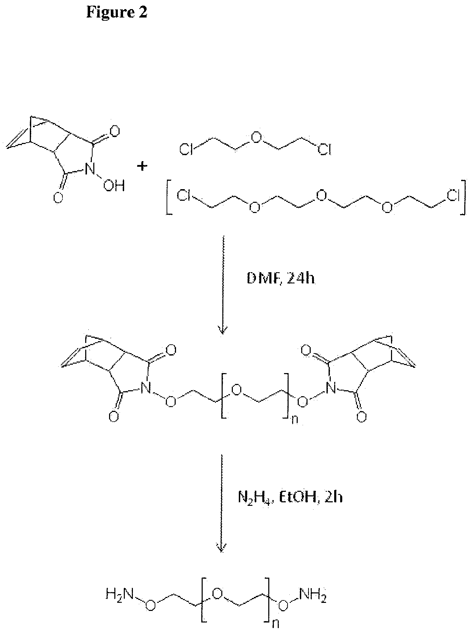

(3-oxa-pentane-1,5-dioxyamine) containing two active aminooxy groups was synthesized according to Boturyn et al. (Tetrahedron 1997;53:5485-92) in a two step organic reaction employing a modified Gabriel-Synthesis of primary amines (Figure 2). In the first step, one molecule of 2,2-chlorodiethylether was reacted with two molecules of Endo-N-hydroxy-5-norbornene-2,3-dicarboximide in dimethylformamide (DMF). The desired homobifunctional product was prepared from the resulting intermediate by hydrazinolysis in ethanol.

### Example 2

### Preparation of the homobifunctional linker NH₂[OCH₂CH₂]₄ONH₂

The homobifunctional linker NH₂[OCH₂CH₂]₄ONH₂

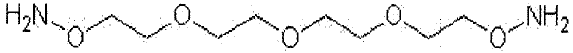

(3,6,9-trioxa-undecane-1,11-dioxyamine) containing two active aminooxy groups was synthesized according to Boturyn et al. (Tetrahedron 1997;53:5485-92) in a two step organic reaction employing a modified Gabriel-Synthesis of primary amines (Figure 2). In the first step one molecule of Bis-(2-(2-chlorethoxy)-ethyl)-ether was reacted with two molecules of Endo-N-hydroxy-5-norbornene-2,3-dicarboximide in DMF. The desired homobifunctional product was prepared from the resulting intermediate by hydrazinolysis in ethanol.

### Example 3

### Preparation of the homobifunctional linker NH₂[OCH₂CH₂]₆ONH₂

The homobifunctional linker NH₂[OCH₂CH₂]₆ONH₂

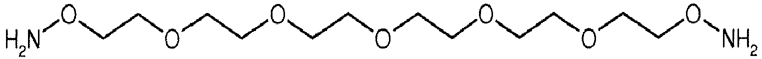

(3,6,9,12,15-penatoxa-heptadecane-1,17-dioxyamine) containing two active aminooxy groups was synthesized according to Boturyn et al. (Tetrahedron 1997;53:5485-92) in a two step organic reaction employing a modified Gabriel-Synthesis of primary amines. In the first step one molecule of hexaethylene glycol dichloride was reacted with two molecules of Endo-N-hydroxy-5-norbornene-2,3-dicarboximide in DMF. The desired homobifunctional product was prepared from the resulting intermediate by hydrazinolysis in ethanol.

### Example 4

### Detailed synthesis of 3-oxa-pentane-1,5 dioxyamine

3-oxa-pentane-1,5 dioxyamine was synthesized according to Botyryn et al. (Tetrahedron 1997; 53:5485-92) in a two step organic synthesis as outlined in Example 1.

### Step 1:

To a solution of Endo-N-hydroxy-5-norbonene-2,3- dicarboxiimide (59.0g; 1.00eq) in 700 ml anhydrous N,N-dimetylformamide anhydrous K2CO3 (45.51g; 1.00eq) and 2,2-dichlorodiethylether (15.84 ml; 0.41eq) were added. The reaction mixture was stirred for 22 h at 50°C. The mixture was evaporated to dryness under reduced pressure. The residue was suspended in 2 L dichloromethane and extracted two times with saturated aqueous NaCl-solution (each 1 L). The Dichloromethane layer was dried over Na2SO4 and then evaporated to dryness under reduced pressure and dried in high vacuum to give 64.5 g of 3-oxapentane-1,5-dioxy-endo-2',3'-dicarboxydiimidenorbornene as a white-yellow solid (intermediate 1).

### Step2:

To a solution of intermediate 1 (64.25 g; 1.00eq) in 800 ml anhydrous Ethanol, 31.0 ml Hydrazine hydrate (4.26eq) were added. The reaction mixture was then refluxed for 2hrs. The mixture was concentrated to the half of the starting volume by evaporating the solvent under reduced pressure. The occurring precipitate was filtered off. The remaining ethanol layer was evaporated to dryness under reduced pressure. The residue containing the crude product 3-oxa-pentane -1,5-dioxyamine was dried in vacuum to yield 46.3g. The crude product was further purified by column chromatography (Silicagel 60; isocratic elution with Dichloromethane/Methanol mixture, 9+1) to yield 11.7 g of the pure final product 3-oxa-pentane-1,5-dioxyamine.

### Example 5

### Preparation of aminooxy-PSA

1000 mg of oxidized PSA (MW = 20 kD) obtained from the Serum Institute of India (Pune, India) was dissolved in 16 ml 50mM phospate buffer pH 6.0. Then 170 mg 3-oxa-pentane-1,5-dioxyamine was given to the reaction mixture. After shaking for 2 hrs at RT 78.5 mg sodium cyanoborohydride was added and the reaction was performed for 18 hours over night. The reaction mixture was then subjected to a ultrafiltration/diafiltration procedure (UF/DF) using a membrane with a 5 kD cut-off made of regenerated cellulose (Millipore).

Alternatively aminooxy PSA can be prepared without an reduction step:
573 mg of oxidized PSA (MW = 20 kD) obtained from the Serum Institute of India (Pune, India) was dissolved in 11,3 ml 50mM phosphate buffer pH 6.0 (Bufffer A). Then 94 mg 3-oxa-pentane-1,5-dioxyamine was given to the reaction mixture. After shaking for 5 h at RT the mixture was then subjected to a weak anion exchange chromatography step employing a Fractogel EMD DEAE 650-M chromatography gel (column dimension: XK16/105). The reaction mixture was diluted with 50 ml Buffer A and loaded onto the DEAE column pre-equilibrated with Buffer A at a flow rate of 1 cm/min. Then the column was washed with 20 CV Buffer B (20 mM Hepes, pH 6.0) to remove free 3-oxa-pentane-1,5-dioxyamine and cyanide at a flow rate of 2 cm/min. The aminooxy-PSA reagent was the eluted with a step gradient consisting of 67 % Buffer B and 43 % Buffer C (20 mM Hepes, 1 M NaCl, pH 7.5). The eluate was concentrated by UF/DF using a 5 kD membrane made of polyether sulfone (50 cm², Millipore). The final diafiltration step was performed against Buffer D (20 mM Hepes, 90 mM NaCl, pH 7.4). The preparation was analytically characterized by measuring total PSA (Resorcinol assay) and total aminooxy groups (TNBS assay) to determine the degree of modification. Furthermore the polydispersity as well as free 3-oxa-pentane-1,5-dioxyamine was determined

### Example 6

### Lyophilization of of aminooxy-PSA reagent

An Aminooxy - PSA reagent was prepared according to Example 5. After diafiltration, the product was frozen at -80°C and lyophilized. After lyophilization the reagent was dissolved in the appropriate volume of water and used for preparation of PSA-protein conjugates via carbohydrate modification.

### Example 7

### PEGylation of A1PI with PEG maleimide (sequential method)

25mg of purified A1PI were dissolved in reaction buffer (20mM Na2HPO4, 5 mM EDTA, pH 7.0) to give a final concentration of 10 mg/ml. To this solution an aliquot of a TCEP (Tris[2-carboxyethyl] phosphine hydrochloride / Thermo Scientific) stock solution (5mg TCEP / ml reaction buffer) was added to get a molar excess of 3M TCEP. The mixture was incubated for 1 hour in the dark at room temperature. Then the TCEP was separated by gelfiltration using a PD-10 column (GE-Healthcare). Subsequently the A1PI was chemically modified using a branched PEG maleimide 20kD (NOF Sunbright MA Series) in a 10 molar excess. The modification reaction was performed for 1 hour at a temperature of T = +2-8°C in the dark followed by a quenching step using L-cysteine (final conc.: 10 mM). After the addition of L-cysteine the reaction mixture was incubated under gentle shaking for an additional hour at the same temperature.

The modified A1PI was diluted with equilibration buffer (25mM Na2HPO4, pH 6.5) to correct the solutions conductivity to < 4.5 mS/cm and loaded onto a pre-packed HiTrap Q FF (GE-Healthcare) with a column volume (CV) of 5ml and a flow rate of ml/min. Then the column was equilibrated with 10 CV equilibration buffer (flow rate: 2 ml/min). Finally the PEG-A1PI was eluted with a linear gradient with elution buffer (25mM Na2HPO4. 1M NaCl, pH 6.5).

### Example 8

### PEGylation of A1PI with PEG maleimide (simultaneous approach)

30mg of purified A1PI were dissolved in reaction buffer (20mM Na2HPO4, 5 mM EDTA, pH 7.0) to give a final concentration of 10 mg/ml. To this solution an aliquot of a TCEP stock solution (5mg TCEP / ml reaction buffer) was added to get a molar excess of 4M TCEP. The mixture was incubated for 10 minutes, then the chemical modification was started by addition of a branched PEG maleimide 20kD (NOF Sunbright MA Series) in a 10 molar excess. The modification reaction was performed for 1 hour at a temperature of T = +2-8°C in the dark followed by a quenching step using L-cysteine (final conc.: 10 mM). After the addition of L-cysteine the reaction mixture was incubated under gentle shaking for an additional hour at the same temperature.

The modified A1 PI was diluted with equilibration buffer (25mM Na2HPO4, pH 6.5) to correct the solutions conductivity to < 4.5 mS/cm and loaded onto a pre-packed HiTrap Q FF (GE-Healthcare) with a column volume (CV) of 5ml using a flow rate of 1 ml/min. Then the column was equilibrated with 10 CV equilibration buffer (flow rate: 2 ml/min). Finally the PEG-A1PI was eluted with a linear gradient with elution buffer (25mM Na2HPO4. 1M NaCl, pH 6.5).

### Example 9

### Pharmacokinetic study of PEGylated A1PI monitored with a PEG-A1PI ELISA

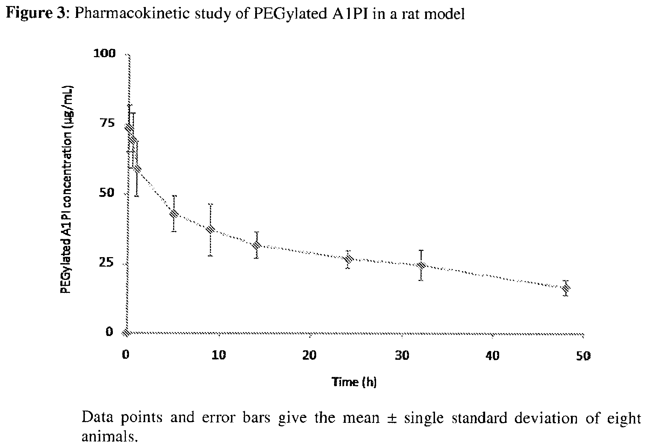

A PEG-A1PI ELISA was used for specifically measuring the concentrations of PEGylated A1PI in plasma samples derived from a rat pharmacokinetic study. The assay basically followed the application US 12/342,405. Briefly, we coated rabbit anti-PEG IgG (Epitomics, #YG-02-04-12P) at a concentration of 5 µg/mL in carbonate buffer, pH 9.5 to Maxisorp F96 plates and detected bound PEGylated A1PI using a anti-human A1PI-peroxidase preparation (The Binding site, PP034). The assay showed a linear dose-concentration relation ranging from 2 to 32 ng/mL A1PI-bound PEG. We established this assay range by serially diluting a preparation of PEGylated A1PI with a known concentration of PEG, measured by using the colorimetric method described by Nag et al. (Anal Biochem 1996; 237: 224-31). A colorimetric assay for estimation of polyethylene glycol and polyethylene glycolated proteins using ammonium ferrothiocyanate), and used the calibration curve obtained for extrapolating the samples' signals. Figure 3 shows the phermacokinetic profile obtained.

PEGylated A1PI was specifically measureable without any interference by endogenous non-PEGylated rat A1PI at all time points after administration with the last samples taken 48 h after administration. The concentrations measured decreased over time as expected with no evidence for a massive dePEGylation of PEGylated A1PI occurring in the rat circulation during the observation period. Thus, the PK profile obtained in the rat model demonstrated the stability of the PEGylated A1PI in the rat circulation because of the specific assay used for monitoring its concentration.

### Example 10

### PEGylation of lysine residues in A1PI with PEG-NHS

A1PI was PEGylated with a PEGylation reagent (SUNBRIGHT GL2-200GS / NOF, Tokyo, Japan) with a molecular weight of 20 kD and containing an active NHS ester (systematic name: 2,3-Bis(methylpolyoxyethylen-oxy)-1-(1,5-dioxo-5-succinimidyloxy, pentyloxy)propan). A solution of purified A1PI in 50 mM phosphate buffer, pH 7.5 was adjusted to a protein concentration of 3.3 mg/ml and the PEGylation reagent (stock solution: 50mg reagent / ml 2 mM HCl) was added to give a final concentration of 10 mg/ml. The PEGylation reaction was carried out under gentle stirring at room tempaerture for 2 hours. Then the reaction was stopped by the addition with gtlycine (final conc. 10 mM) for 1 hour. Subsequently the pH of the reaction was adjusted to 6.5 by addition of 2N HCl and the mixture was loaded onto an anion-exchange chromatography resin (Q-Sepharose FF / GE-Healthcare) pre-equilibrated with 25 mM phosphate buffer, pH 6.5. Then the column was washed with 20 CV equilibration buffer to remove excess reagent and the PEGylated A1PI was eluted with elution buffer (25 mM phosphate buffer, 1 M NaCl) using a flow rate of 1 ml/min.

Finally the eluate was concentrated by ultrafiltration/diafiltration using Vivaspin devices (Sartorius, Göttingen, Gemany) with a membrane consisting of polyethersulfone and a molecular weight cut-off of 10 kD. The final diafiltration step was performed against 50mM phosphate buffer pH 7.0.

### Example 11

### PEGylation of carbohydrate residues in A1PI (sequential method)

To 2.0 mg A1PI dissolved in 1.5 ml 20 mM phosphate buffer, pH 6.0, an aqueous sodium periodate solution (10 mM) was added to give a final concentration of 100 µM. The mixture was shaken in the dark for 1h at 4°C and quenched for 15min at RT by the addition of an 1 M glycerol solution (final concentration: 10 mM). Then low molecular weight contaminants were separated by gelfiltration on PD-10 columns (GE Healthcare) pre-equilibrated with the same buffer system. Subsequently a linear aminooxy-PEG reagent (NOF SUNBRIGHT ME 200C / NOF, Tokyo, Japan) was added to the A1PI containing fraction and the mixture was shaken at pH 6.0 for 18 hours at 4°C. Finally the conjugate was further purified by IEX under conditions as described above.

### Example 12

### PEGylation of carbohydrate residues in A1PI (simultaneous approach)

10 mg A1PI is dissolved in 12 ml histidine - buffer, pH 6.0 (20 mM L-histidine, 150 mM NaCl, 5 mM CaCl₂). Then an aqueous sodium periodate solution (5 mM) is added to give a final concentration of 120 µM. Subsequently a linear PEG - aminooxy reagent with a MW of 20 kD (reagent (NOF SUNBRIGHT ME 200C / NOF, Tokyo, Japan) is added to give a 5 fold molar excess of PEG reagent. The mixture is incubated for 18 hours in the dark at 4°C under gentle stirring and quenched for 15 min at room temperature by the addition of 25 µl of 1 M aqueous cysteine solution. Finally the conjugate is further purified by IEX under conditions as described above.

### Example 13

### PEGylation of carbohydrate residues in A1PI in the presence of nucleophilic catalyst

A1PI is PEGylated via carbohydrate residues as described above. The chemical reaction with the aminooxy reagent is performed in the presence of the nucleophilic catalyst aniline (Zheng et al, Nature Methods 2009;6:207-9) using a concentration of 10 mM. As an alternative to this catalyst m-toluidine (concentration: 10 mM) is used. The chemical reaction is carried out for 2 hours at room temperature instead of 18 hours at 4°C. As an alternative m-toluidine or other nucleophilic catalysts as described in US 2012/0035344 A1 can be used.

### Example 14

### Preparation of PSA maleimide

0.68 g oxidized PSA was dissolved in 15.1 ml 50 mM phosphate buffer pH 6.0 to give a final concentration of 43 mg/ml. Then a 50 mM solution of the bifunctional EMCH linker (Pierce / 16.7 mg/ml in 50 mM phosphate buffer) containing a maleimide and a hydrazide group was added. The pH was corrected to pH 6.0 and the solution was incubated in the dark for 30 minutes at room temperature under gentle stirring. Subsequently 2.6 ml of a 1M NaBH₃CN solution (= 50 M excess) was added and another incubation was performed for 180 minutes in the dark at R.T. under gentle stirring. Then the solution was diluted 1:1 with 50mM phosphate buffer pH 6 to reduce the conductivity (∼7mS/cm). Then the mixture was applied onto a prepacked IEX column with a bed volume of 8ml (monolith type DEAE CIM / BIA Separations) for purification of the PSA maleimide linker at a flow rate of 4 ml/min. Then the column was washed with 32 column volumes 50 mM phosphate buffer pH 6.0 using a flow rate of 40 ml/min. Then the linker was eluted with a gradient of 59% 50mM phosphate buffer pH 6.0 and 41% 50mM phosphate buffer/1M NaCl pH 7.5. Finally the eluate was subjected to UF/DF using a Polyethersulfone membrane (type BIOMAX 5 / Millipore). The final diafiltration step was carried out against 50 mM phosphate buffer, pH 7.5 containing 90 mM NaCl.

### Example 15

### Polysialylation of A1PI with PSA maleimide (sequential method)

A1PI is polysialylated by use of a polysialylation reagent containing an active maleimide group. An example of this type of reagent is described above (reaction of oxidized PSA with the bifunctional EMCH linker (Pierce) and subsequent purification by ion-exchange chromatography).

A1PI is reduced with the TCEP reagent (Tris[2-carboxyethyl] phosphine hydrochloride / Thermo Scientific) in reaction buffer (20mM Na2HPO4, 5 mM EDTA, pH 7.0) using a protein concentration of 10 mg/ml and a 3 M reagent excess. The mixture is incubated for 1 hour in the dark at room temperature. Then the TCEP is separated by gelfiltration using a PD-10 column (GE-Healthcare). Subsequently the A1PI is chemically modified with PSA maleimide in reaction buffer using a 10 molar reagent excess. The modification reaction is performed for 1 hour at a temperature of 4°C in the dark followed by a quenching step using L-cysteine (final conc.: 10 mM). After the addition of L-cysteine the reaction mixture is incubated under gentle stirring for an additional hour at the same temperature. Finally the polysialylated A1PI is purified by IEX on Q-Sepharose FF.

### Example 16

### Polysialylation of A1PI with PSA maleimide (simultaneous approach)

A1PI is polysialylated by use of a polysialylation reagent containing an active maleimide group. An example of this type of reagent is described above (reaction of oxidized PSA with the bifunctional EMCH linker (Pierce) and subsequent purification by ion-exchange chromatography).

A1PI is dissolved in reaction buffer (20 mM Na2HPO4, 5 mM EDTA, pH 7.0) to give a final concentration of 10 mg/ml. To this solution an aliquot of a TCEP (Tris[2-carboxyethyl] phosphine hydrochloride / Thermo Scientific) stock solution (5mg TCEP / ml reaction buffer) is added to get a 4 fold molar excess. The mixture is incubated for 10 minutes, then the chemical modification is started by addition of the PSA maleimide reagent (Example 14) in 10 molar excess.

The modification reaction is performed for 1 hour at 4°C in the dark. After the addition of L-cysteine the reaction mixture is incubated under gentle stirring for an additional hour at the same temperature. Finally the polysialylated A1PI is purified by IEX on Q-Sepharose FF.

### Example 17

### Modification of SH groups in human serum albumin (HSA) with PEG maleimide

30mg of purified HSA is dissolved in reaction buffer (20mM Na2HPO4, 5 mM EDTA, pH 7.0) to give a final concentration of 10 mg/ml. To this solution an aliquot of a TCEP stock solution (5mg TCEP / ml reaction buffer) is added to result in a molar excess of 4. The mixture is incubated for 10 minutes. The chemical modification is stalled by addition of a 10-fold molar excess of a branched PEG reagent (molecular weight 20 kD) containing a terminal maleimide group. An example of this type of reagent is the Sunbright ® MA series from NOF (NOF Corp., Tokyo, Japan). The modification reaction is performed for 1 hour at a temperature of T = +2-8°C in the dark followed by a quenching step using L-cysteine (final conc.: 10 mM). After the addition of L-cysteine, the reaction mixture is incubated under gentle shaking for an additional hour at the same temperature. The pH value is then adjusted to 6.8 by dropwise addition of 0.1 M HCl.

Subsequently, the conjugate is purified by anion-exchange chromatography on DEAE - Sepharose FF. The reaction mixture is applied onto a chromatographic column (volume: 20 ml). The column is then washed with 10 column volumes (CV) starting buffer (25 mM sodium acetate, pH 6.2). The PEG-HSA conjugate is eluted with 25 mM sodium acetate buffer, pH 4.5 and the OD at 280 nm is measured. The conjugate containing fractions are pooled and subjected to UF/DF using a 10 kD membrane of regenerated cellulose.

### Example 18

### Modification of SH groups in recombinant factor VIII (rFVIII)

A recombinant FVIII (rFVIII) mutant containing a free and accessible sulfhydryl groups is prepared according to US 7,632,921 B2 by recombinant DNA technology and is used for chemical modification via free SH groups. This mutant is chemically modified using a 10-fold molar excess of a branched PEG reagent (molecular weight 20 kD) containing a terminal maleimide group. An example of this type of reagent is the Sunbright ® MA series from NOF (NOF Corp., Tokyo, Japan). The reaction is carried out for 1 hour at room temperature in the presence of a 5 fold excess of TCEP. Then the conjugate is purified by Hydrophobic Interaction Chromatography (HIC). The ionic strength is increased by adding a buffer containing 8M ammonium acetate (8M ammonium acetate, 50mM Hepes, 5mM CaCl2, 350mM NaCl, 0.01% Tween 80, pH 6.9) to get a final concentration of 2.5M ammonium acetate. Then the reaction mixture is loaded onto a chromatographic column packed with Phenyl - Sepharose FF, which is equilibrated with equilibration buffer (2.5M ammonium acetate, 50mM Hepes, 5mM CaCl2, 350mM NaCl, 0.01% Tween 80, pH 6.9). The product is eluted with elution buffer (50mM Hepes, 5mM CaCl2, 0.01% Tween 80, pH 7.4), and the eluate is concentrated by UF/DF using a 30 kD membrane made of regenerated cellulose.

### Example 19

### Polysialylation of other therapeutic proteins

Polysialylation reactions performed in the presence of alternative nucleophilic catalysts like m-toluidine or o-aminobenzoic acid as described herein may be extended to other therapeutic proteins. For example, in various aspects of the present disclosure, the above polysialylation or PEGylation reactions as described above with PSA aminooxy or PEG aminooxy reagents is repeated with therapeutic proteins such as those proteins described herein.

### Example 20

### PEGylation of a therapeutic protein using branched PEG

PEGylation of a therapeutic protein of the present disclosure may be extended to a branched or linear PEGylation reagent, which is made of an aldehyde and a suitable linker containing an active aminooxy group.

### Example 21

### Polysialylation of other therapeutic proteins

Polysialylation reactions performed in the presence of alternative nucleophilic catalysts like m-toluidine or o-aminobenzoic acid as described herein may be extended to other therapeutic proteins. For example, in various aspects of the present disclosure, the above polysialylation or PEGylation reactions as described above with PSA aminooxy or PEG aminooxy reagents is repeated with therapeutic proteins such as those proteins described herein. The polysialylation reaction is carried out in in individual reaction steps or, in the alternative, in a simultaneous reaction as described herein.

### Example 22

### PEGylation of a therapeutic protein using branched PEG

PEGylation, according to the examples provided herein, of a therapeutic protein of the present disclosure may be extended to a branched or linear PEGylation reagent, which is made of an aldehyde and a suitable linker containing an active aminooxy group.

### Example 23

### PEGylation of a therapeutic protein using branched PEG

PEGylation of a therapeutic protein of the present disclosure may be extended to a branched or linear PEGylation reagent as described above, which is made of an aldehyde and a suitable linker containing an active aminooxy group. The PEGylation reaction is carried out in in individual reaction steps or, in the alternative, in a simultaneous reaction as described herein.

### Example 24

### Polysialylation of albumin [sequential approach]

PSA maleimide was prepared according to Example 14 and is used for the polysialylation of human serum albumin (HSA) via free SH-groups using the sequential approach. HSA is reduced with the TCEP reagent (Tris[2-carboxyethyl] phosphine hydrochloride / Thermo Scientific) in reaction buffer (20mM Na₂HPO₄, 5 mM EDTA, pH 7.0) using a protein concentration of 10 mg/ml and a 3 M reagent excess. The mixture is incubated for 1 hour in the dark at room temperature. Then TCEP is separated by gelfiltration using a PD-10 column (GE-Healthcare). Subsequently, the HSA is chemically modified with PSA maleimide in reaction buffer using a 10 molar reagent excess. The modification reaction is performed for 1 hour at a temperature of 4°C in the dark followed by a quenching step using L-cysteine (final conc.: 10 mM). After the addition of L-cysteine the reaction mixture is incubated under gentle stirring for an additional hour at the same temperature. Finally, the polysialylated HSA is purified by IEX on Q-Sepharose FF.

### Example 25

### Polysialylation of albumin (simultaneous approach)

PSA maleimide was prepared according to Example 14 and is used for the polysialylation of human serum albumin (HSA) via free SH-groups using the simultaneous approach HSA is dissolved in reaction buffer (20 mM Na₂HPO₄, 5 mM EDTA, pH 7.0) to give a final concentration of 10 mg/ml. To this solution an aliquot of a TCEP (Tris[2-carboxyethyl] phosphine hydrochloride / Thermo Scientific) stock solution (5mg TCEP / ml reaction buffer) is added to get a 4 fold molar excess. The mixture is incubated for 10 minutes, then the chemical modification is started by addition of the PSA maleimide reagent (Example 14) in 10 fold molar excess. The modification reaction is performed for 1 hour at 4°C in the dark. After the addition of L-cysteine the reaction mixture is incubated under gentle stirring for an additional hour at the same temperature. Finally, the polysialylated HSA is purified by IEX on Q-Sepharose FF.

### Example 26

### PEGylation of A1PI

Several batches with 2.6 mg of A1PI (pure A1PI starting material) in 1 ml of reaction buffer (20 mM Na₂HPO₄, 5 mM EDTA, pH 7.0) were processed by use of different molar excesses of the reductant TCEP. The goal of these investigation was the optimization of the TCEP concentration for the reaction PEG-maleimide with A1PI.

The PEGylation reaction was carried out in a 10-fold molar PEG excess simultaneously with reduction on the one hand and sequentially after removal of the reductants on the other. The optimum molar TCEP excess was tested for both variants.

Finally, the mixture was quenched with cysteine in a 10-fold molar excess based on the quantity of PEG reagent used at room temperature for one hour.

### Determination of the optimum reductant excess.

All factors with the exception of the molar excess of TCEP were kept constant in the modification batches. Since it was the objective of this technique to bind the 20 kD-MAL-PEG selectively to the only cysteine of A1PI, all that had to be taken into account for HPLC analysis was the ratio of mono-PEG-AIPI to the native A1PI. Poly-PEG-A1PI was not observed under the applicable conditions, which confirms the assumption of specific coupling.

The comparison of two conjugations demonstrated that conjugation proceeded much better in the case of sequential reduction / PEGylation than in the simultaneous approach. Even with a 3-molar excess of TCEP, a maximum ratio of about 79 % of mono PEG A1PI was achieved, while the highest mono-PEG-A1PI ratio of about 74 % was obtained with a 4-molar excess on TCEP in a simultaneous process. Reduction with mercaptoethanol in the concentrations 0.4, 2 and 4 mM (= 8, 40, 80-fold molar excess) was also tested in the sequential variant, also including the concentration (2 mM) with the highest PEGylation turnover in the comparison. It turned out that mercaptoethanol could be a useful alternative for the sequential approach, but not for the simultaneous conjugation process, because it was not compatible with the MAL-PEG reaction.

**Table 1**

| Molar TCEP excess | Mono-PEG-A1PI (left) | Native A1PI (left) | Mono-PEG-A1PI (right) | Native A1PI (right) |
|---|---|---|---|---|
| 0 | 2.87% | 97.13% | 2.87% | 97.13% |
| 1 | 34.83% | 65.17% | 69.65% | 30.35% |
| 2 | 55.38% | 44.62% | 76.53% | 23.47% |
| 3 | 67.97% | 32.03% | 78.91% | 21.09% |
| 4 | 73.95% | 26.05% | 76.33% | 23.67% |
| 5 | 71.85% | 28.15% | 74.09% | 25.91% |
| 10 | 70.94% | 29.06% | 67.49% | 32.51% |
| 100 | 21.06% | 78.94% | 52.22% | 47.78% |
| **2 mM mercaptoethanol* | - | - | 77.52% | 22.48% |

### Testing the optimum molar PEG excess

The influence of the PEG excess on the PEGylation reaction was tested both for the simultaneous and the sequential process. For this purpose, the modification batches were treated under the same reaction conditions as for the optimization of the reductant (TCEP). The simultaneous process was adjusted to a 4-fold and the sequential process a 3-fold molar TCEP excess, i.e. to the ideal reductant excesses determined earlier. All factors except the PEG excess were kept constant. Analysis of the PEGylation turnover of the respective batches was carried out by HPLC.

**Table 2**

| Molar PEG excess | Mono-PEG-A1PI (%) left | Native A1PI (%) left | Mono-PEG-A1PI (%) right | Native A1PI (%) right |
|---|---|---|---|---|
| 1 | 14.35 | 85.65 | 68.76 | 31.24 |
| 3 | 37.83 | 62.12 | 85.47 | 14.53 |
| 5 | 63.21 | 36.79 | 85.23 | 14.77 |
| 10 | 68.94 | 31.06 | 84.22 | 15.78 |
| 15 | 68.79 | 31.21 | 82.46 | 17.54 |
| 25 | 67.00 | 33.00 | 76.72 | 23.28 |
| 50 | 35.14 | 64.86 | 41.22 | 58.78 |

The comparison of both PEGylation variants (Table 2) showed that even a 3-fold molar PEG excess resulted in the maximum PEGylation turnover of up to 85% in a sequential process. On the other hand, the simultaneous process carried out under the same reaction conditions (3-fold PEG excess) does not even achieve half of this PEGylation turnover. Moreover, Table 2 shows that a PEG excess which is too high will probably impede the PEGylation reaction owing to the high ratio of native A1PI.

A better PEGylation rate was also achieved in the sequential process by collecting the reduced A1PI with a Falkon tube filled with the respective PEG excess during the removal of TECP by means of a PD-10 column, because the reduced A1PI was able to react directly with the PEG, resulting in a higher PEGylation turnover.

In comparison, the PEG reagent was added only after the PD-10 column procedure had been conducted in the TCEP optimization so that even after this short residence time a small portion of the reduced A1PI seemed to re-oxidise and the PEG turnover was smaller.

### Example 27

### Inhibition of elastase with PEGylated A1PI

Mono-PEGylated A1PI was prepared via modification of SH-groups by reaction with MAL-PEG 20 kD as described in Example 8 and subjected to an in vitro activity test. This test determines the capability of A1PI to inhibit porcine pancreas elastase as a measure of its functional activity. In brief, the elastase inhibitor activity of A1PI or the A1PI derivative is determined in a two-step reaction. In the first step the A1PI sample is incubated with an excess of porcine elastase. This causes complex formation and inactivation of elastase. In the second step, the remaining elastase activity is measured by addition of the elastase-specific chromogenic substrate Suc(Ala)₃-pNA. The release of pNA can be measured photometrically at 405nm and is a direct measure for the residual elastase activity. The residual elastase activity is within a predefined range indirect proportional to the A1PI concentration. The assay calibration is achieved by using an A1PI reference preparation, calibrated against the 1^{st} WHO standard for α1-antitrypsin (WHO 05/162; 12.4 mg functionally active A1PI/mL). The results were expressed in mg active A1PI/ml. In addition the total protein content was measured by the Bradford assay and the ratio activity / total protein was calculated.

As a result an activity of 74% was determined for the mono-PEGylated A1PI modification variant as compared to non-modified A1PI.

### Preferred Embodiments

1. A method of preparing a therapeutic protein conjugate comprising the step of contacting a therapeutic protein, or biologically-active fragment thereof, with a thiol reductant and a water soluble polymer, or functional derivative thereof, under conditions that (a) produce a reduced cysteine sulfhydryl group on the therapeutic protein, and (b) allow conjugation of the water-soluble polymer to the reduced cysteine sulfhydryl group;
   said therapeutic protein having an amino acid sequence with no more than one accessible cysteine sulhydryl group.
2. The method according to item 1 wherein the therapeutic protein is selected from the group consisting of:
   a protein of the serpin superfamily selected from the group consisting of: A1PI (alpha-1 proteinase inhibitor), or A1AT (alpha-1-antitrypsin), ATR (alpha-1-antitrypsin-related protein), AACT or ACT (alpha-1-antichymotrypsin), PI4 (proteinase inhibitor 4), PCI or PROCI (protein C inhibitor), CBG, (corticosteroid-binding globulin), TBG (thyroxine-binding globulin), AGT (angiotensinogen), centerin, PZI (protein Z-dependent protease inhibitor), PI2 (proteinase inhibitor 2), PAI2 or PLANH2 (plasminogen activator inhibitor-2), SCCA1 (squamous cell carcinoma antigen 1), SCCA2 (squamous cell carcinoma antigen 2), PI5 (proteinase inhibitor 5), PI6 (proteinase inhibitor 6), megsin, PI8 (proteinase inhibitor 8), PI9 (proteinase inhibitor 9), PI10 (proteinase inhibitor 10), epipin, yukopin, PI13 (proteinase inhibitor 13), PI8L1 (proteinase inhibitor 8-like 1), AT3 or ATIII (antithrombin-III), HC-II or HCF2 (heparin cofactor II), PAI1 or PLANH1 (plasminogen activator inhibitor-1), PN1 (proteinase nexin I), PEDF, (pigment epithelium-derived factor), PLI (plasmin inhibitor), C1IN or C1 INH (plasma proteinase C1 inhibitor), CBP1 (collagen-binding protein 1), CBP2 (collagen-binding protein 2), PI12 (proteinase inhibitor 12), and PI14 (proteinase inhibitor 14);
   a protein selected from the group consisting of: antithrombin III, alpha-1-antichymotrypsin, human serum albumin, alcoholdehydrogenase, biliverdin reductase, buturylcholinesterase, complement C5a, cortisol-binding protein, creatine kinase, ferritin, heparin cofactor, interleukin 2, protein C inhibitor, tissue factor; vitronectin; ovalbumin, plasminogen-activator inhibitor, neuroserpin, C1-Inhibitor, nexin, alpha-2-antiplasmin, heparin cofactor II, alpha 1-antichymotrypsin, alpha1-microglobulin; and
   a blood coagulation factor protein selected from the group consisting of: Factor IX (FIX), Factor VIII (FVIII), Factor VIIa (FVIIa), Von Willebrand Factor (VWF), Factor FV (FV), Factor X (FX), Factor XI (FXI), Factor XII (FXII), Factor XIII (FXIII) thrombin (FII), protein C, protein S, tPA, PAI-1, tissue factor (TF) and ADAMTS 13 protease.
3. The method according to item 3 wherein the therapeutic protein is A1PI.
4. The method according to item 3 wherein the therapeutic protein is human serum albumin.
5. The method according to item 1 wherein the therapeutic protein is a glycoprotein.
6. The method according to item 5 wherein the therapeutic protein is glycosylated in vivo.
7. The method according to item 5 wherein the therapeutic protein is glycosylated in vitro.
8. The method according to item 1 comprising a quantity of therapeutic protein between 0.100 and 10.0 gram weight.
9. The method according to item 1 wherein the water-soluble polymer is selected from the group consisting of linear, branched or multi-arm water soluble polymer.
10. The method according to item 9 wherein the water-soluble polymer has a molecular weight between 3,000 and 150,000 Daltons (Da).
11. The method according to item 10 wherein the water-soluble polymer is linear and has a molecular weight between 10,000 and 50,000 Da.
12. The method according to item 11 wherein the water-soluble polymer is linear and has a molecular weight of 20,000.
13. The method according to item 9 wherein the water-soluble polymer is selected from the group consisting of polyethylene glycol (PEG), branched PEG, PolyPEG® (Warwick Effect Polymers; Coventry, UK), polysialic acid (PSA), starch, hydroxylethyl starch (HES), hydroxyalkyl starch (HAS), carbohydrate, polysaccharides, pullulane, chitosan, hyaluronic acid, chondroitin sulfate, dermatan sulfate, dextran, carboxymethyl-dextran, polyalkylene oxide (PAO), polyalkylene glycol (PAG), polypropylene glycol (PPG), polyoxazoline, polyacryloylmorpholine, polyvinyl alcohol (PVA), polycarboxylate, polyvinylpyrrolidone, polyphosphazene, polyoxazoline, polyethylene-co-maleic acid anhydride, polystyrene-co-maleic acid anhydride, poly(1-hydroxymethylethylene hydroxymethylformal) (PHF), 2-methacryloyloxy-2'-ethyltrimethylammoniumphosphate (MPC), and functional derivatives thereof.
14. The method according to item 9 wherein the water soluble polymer is derivatized to contain a sulfhydryl-specific group selected from the group consisting of: maleimide (MAL), vinylsulfones, orthopyridyl-disulfides (OPSS) and iodacetamides.
15. The method according item 13 wherein the water soluble polymer is PEG and the sulfhydryl-specific group is MAL.
16. The method according to item 13 wherein the water soluble polymer is PSA and the sulfhydryl-specific group is MAL.
17. The method according to item 1 wherein the thiol reductant is selected from the group consisting of: Tris[2-carboxyethyl] phosphine hydrochloride (TCEP), dithiothreitol (DTT), dithioerythritol (DTE), sodium borohydride (NaBH₄), sodium cyanoborohydride (NaCNBH₃), β-mercaptoethanol (BME), cysteine hydrochloride and cysteine.
18. The method according to item 17 wherein the thiol reductant is TCEP.
19. The method according to item 17 wherein the thiol reductant concentration is between 1 and 100-fold molar excess relative to the therapeutic protein concentration.
20. The method according to item 19 wherein the thio reductant concentration is between 1 and 10-fold molar excess relative to the therapeutic protein concentration.
21. The method according to item 1 wherein the amino acid sequence of the therapeutic protein contains no more than one cysteine residue.
22. The method item 1 wherein the accessible cysteine sulfhydryl group is present in a native amino acid sequence of the therapeutic protein.
23. The method according to item 1 wherein the amino acid sequence of therapeutic protein is modified to include the accessible cysteine sulfhydryl group.
24. The method according to item 1 wherein the conditions that produce a reduced cysteine sulfhydryl group on the therapeutic protein do not reduce a disulfide bond between other cysteine amino acids in the protein.
25. The method according to item 1 wherein therapeutic protein comprises only one cysteine residue which comprises an accessible sulfhydryl group that is completely or partially oxidized, said only one cysteine residue is not involved in a disulfide bond with another cysteine residue in the therapeutic protein's amino acid sequence.
26. The method according to item 1 further comprising the step of purifying the therapeutic protein conjugate.
27. The method according to item 26 wherein the therapeutic protein conjugate is purified using a technique selected from the group consisting of ion-exchange chromatography, hydrophobic interaction chromatography, size exclusion chromatography and affinity chromatography or combinations thereof.
28. The method according to item 1 wherein the therapeutic protein, water-soluble polymer and thiol reductant are incubated together in a single vessel, wherein the reduction of the oxidized SH group and the conjugation reaction is carried out simultaneously.
29. The method according to item 1 wherein the thiol reductant is removed following incubation with the therapeutic protein and prior to incubating the therapeutic protein with the water-soluble polymer, wherein the reduction of the oxidized SH group and the conjugation reaction is carried out sequentially.
30. The method according to item 1 wherein the therapeutic protein conjugate retains at least 20% biological activity relative to native therapeutic protein.
31. The method according to item 1 wherein at least 70% of the therapeutic protein conjugate comprises a single water-soluble polymer.
32. The method according to item 1 wherein the therapeutic protein conjugate has an increased half-life relative to native therapeutic protein.
33. The method according to item 32 wherein the therapeutic protein conjugate has at least a 1.5-fold increase in half-life relative to native therapeutic protein
34. A method of preparing an A1PI conjugate comprising the steps of:
   contacting the A1PI with TCEP under conditions that allow the reduction of a sulfhydryl group on the A1PI, and
   contacting a linear PEG derivatized to contain a MAL group with the A1PI under conditions that allow conjugation of the water-soluble polymer to the reduced sulfhydryl group;
   said A1PI comprising only one cysteine residue which comprises an accessible sulfhydryl group that is completely or partially oxidized, said only one cysteine residue is not involved in a di-sulfide bond with another cysteine residue in the A1PI's amino acid sequence;
   said TCEP concentration is between 3 and 4-fold molar excess relative to the A1PI concentration;
   wherein at least 70% of the A1PI conjugate comprises a single water-soluble polymer;
   said A1PI conjugate having an increased half-life relative to native A1PI; and
   said A1PI conjugate retaining at least 60% biological activity relative to native A1PI.

## Claims

1. A method of preparing a therapeutic protein conjugate comprising the step of contacting a therapeutic protein, or biologically-active fragment thereof, with a thiol reductant and a polysialic acid (PSA), or functional derivative thereof, under conditions that (a) produce a reduced cysteine sulfhydryl group on the therapeutic protein, and (b) allow conjugation of the polysialic acid (PSA) to the reduced cysteine sulfhydryl group;
said therapeutic protein having an amino acid sequence with no more than one accessible cysteine sulfhydryl group;
wherein the therapeutic protein, water-soluble polymer and thiol reductant are incubated together in a single vessel, wherein the reduction of the oxidized sulfhydryl group and the conjugation reaction is carried out simultaneously, wherein the therapeutic protein is selected from the group consisting of:
a protein of the serpin superfamily selected from the group consisting of: A1PI (alpha-1 proteinase inhibitor), A1AT (alpha-1-antitrypsin), ATR (alpha-1-antitrypsin-related protein), AACT or ACT (alpha-1-antichymotrypsin), PI4 (proteinase inhibitor 4), PCI (protein C inhibitor), CBG (corticosteroid-binding globulin), TBG (thyroxine-binding globulin), AGT (angiotensinogen), centerin, PZI (protein Z-dependent protease inhibitor), PI2 (proteinase inhibitor 2), SCCA1 (squamous cell carcinoma antigen 1), SCCA2 (squamous cell carcinoma antigen 2), PI5 (proteinase inhibitor 5), PI6 (proteinase inhibitor 6), megsin, PI8 (proteinase inhibitor 8), PI9 (proteinase inhibitor 9), PI10 (proteinase inhibitor 10), epipin, yukopin, PI13 (proteinase inhibitor 13), PI8L1 (proteinase inhibitor 8-like 1), AT3 or ATIII (antithrombin-III), PN1 (proteinase nexin I), PEDF (pigment epithelium-derived factor), PLI (plasmin inhibitor), C1IN or C1 INH (plasma proteinase C1 inhibitor), CBP1 (collagen-binding protein 1), CBP2 (collagen-binding protein 2), PI12 (proteinase inhibitor 12), and PI14 (proteinase inhibitor 14),
a protein selected from the group consisting of: human serum albumin, alcohol dehydrogenase, biliverdin reductase, buturylcholinesterase, complement C5a, cortisol-binding protein, creatine kinase, ferritin, heparin cofactor, interleukin 2, vitronectin, ovalbumin, plasminogen-activator inhibitor, neuroserpin, nexin, alpha-2-antiplasmin, alphal-microglobulin, and
a blood coagulation factor protein selected from the group consisting of: Factor IX (FIX), Factor VIII (FVIII), Factor VIIa (FVIIa), Von Willebrand Factor (VWF), Factor FV (FV), Factor X (FX), Factor XI (FXI), Factor XII (FXII), Factor XIII (FXIII) thrombin (FII), protein C, protein S, tPA, TF (tissue factor) and ADAMTS 13 protease.

2. The method according to claim 1, wherein the therapeutic protein is A1P1.

3. The method according to claim 1, wherein the therapeutic protein is human serum albumin.

4. The method according to claim 1 wherein the therapeutic protein is plasminogen-activator inhibitor, which is PAI-1 or PLANHI (plasminogen activator inhibitor-I) or PAI2 or PLANH2 (plasminogen activator inhibitor-2).

5. The method according to claim 1 wherein the therapeutic protein is heparin cofactor, which is HC-II or HCF2 (heparin cofactor II).

6. The method according to claim 1 wherein the therapeutic protein is a glycoprotein, optionally an in vivo glycosylated glycoprotein or an in vitro glycosylated glycoprotein.

7. The method according to claim 1 comprising a quantity of the therapeutic protein between 0.100 and 10.0 gram weight.

8. The method according to claim 1 wherein the polysialic acid (PSA) is selected from the group consisting of linear, branched or multi-arm water soluble polymer.

9. The method according to claim 8 wherein the polysialic acid (PSA) has a molecular weight between 3,000 and 150,000 Daltons (Da), wherein optionally the water-soluble polymer is linear and has a molecular weight between 10,000 and 50,000 Da such as 20,000 Da.

10. The method according to claim 8 wherein the polysialic acid (PSA) is derivatized to contain a sulfhydryl-specific group selected from the group consisting of: maleimide (MAL), vinylsulfones, orthopyridyl-disulfides (OPSS) and iodacetamides wherein optionally the sulfhydryl-specific group is MAL.

11. The method according to claim 1 wherein the thiol reductant is selected from the group consisting of: Tris[2-carboxyethyl] phosphine hydrochloride (TCEP), dithiothreitol (DTT), dithioerythritol (DTE), sodium borohydride (NaBH₄), sodium cyanoborohydride (NaCNBH₃), β-mercaptoethanol (BME), cysteine hydrochloride and cysteine.

12. The method according to claim 11 wherein the thiol reductant concentration is between 1 and 100-fold molar excess relative to the therapeutic protein concentration such as between 1 and 10-fold molar excess.

13. The method according to claim 1 wherein the amino acid sequence of the therapeutic protein contains no more than one cysteine residue.

14. The method according to claim 1 wherein the accessible cysteine sulfhydryl group is present in a native amino acid sequence of the therapeutic protein or wherein the amino acid sequence of the therapeutic protein is modified to include the accessible cysteine sulfhydryl group.

15. The method according to claim 1 wherein the conditions that produce a reduced cysteine sulfhydryl group on the therapeutic protein do not reduce a disulfide bond between other cysteine amino acids in the therapeutic protein.

16. The method according to claim 1 wherein the therapeutic protein comprises only one cysteine residue which comprises an accessible sulfhydryl group that is completely or partially oxidized, said only one cysteine residue is not involved in a disulfide bond with another cysteine residue in the therapeutic protein's amino acid sequence.

17. The method according to claim 1 wherein the therapeutic protein conjugate retains at least 20% biological activity relative to native therapeutic protein.

18. The method according to claim 1 wherein at least 70% of the therapeutic protein conjugate comprises a single polysialic acid (PSA).

19. The method according to claim 1 wherein the therapeutic protein conjugate has an increased half-life relative to native therapeutic protein, optionally wherein the therapeutic protein conjugate has at least a 1.5-fold increase in half-life relative to native therapeutic protein.

20. The method according to any preceding claim further comprising the step of purifying the therapeutic protein conjugate, optionally wherein the therapeutic protein conjugate is purified using a technique selected from the group consisting of ion-exchange chromatography, hydrophobic interaction chromatography, size exclusion chromatography and affinity chromatography or combinations thereof.
